(19) Europäisches Patentamt
European Patent Office
Office européen des brevets

(11) **EP 4 596 687 A1**

(12) **EUROPEAN PATENT APPLICATION**
published in accordance with Art. 153(4) EPC

(43) Date of publication:
**06.08.2025 Bulletin 2025/32**

(21) Application number: **23872616.0**

(22) Date of filing: **29.09.2023**

(51) International Patent Classification (IPC):
***C12N 7/01*** *(2006.01)* ***A61K 35/763*** *(2015.01)*
***A61K 39/395*** *(2006.01)* ***A61P 35/00*** *(2006.01)*
***A61P 35/02*** *(2006.01)* ***C07K 16/28*** *(2006.01)*
***C07K 19/00*** *(2006.01)* ***C12N 1/15*** *(2006.01)*
***C12N 1/19*** *(2006.01)* ***C12N 1/21*** *(2006.01)*
***C12N 5/10*** *(2006.01)* ***C12N 15/13*** *(2006.01)*
***C12N 15/38*** *(2006.01)* ***C12P 21/08*** *(2006.01)*

(52) Cooperative Patent Classification (CPC):
**A61K 35/763; A61K 39/395; A61P 35/00; A61P 35/02; C07K 14/03; C07K 16/00; C07K 16/28; C07K 19/00; C12N 5/10; C12N 7/00; C12N 15/74; C12N 15/80; C12N 15/81**

(86) International application number:
**PCT/JP2023/035689**

(87) International publication number:
**WO 2024/071397 (04.04.2024 Gazette 2024/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **30.09.2022 JP 2022159009**

(71) Applicants:
- **The University of Tokyo**
  **Bunkyo-ku, Tokyo 113-8654 (JP)**
- **Uchida, Hiroaki**
  **Tokyo 105-0001 (JP)**

(72) Inventors:
- **TAHARA, Hideaki**
  **Tokyo 113-8654 (JP)**
- **UCHIDA, Hiroaki**
  **Tokyo 105-0001 (JP)**

(74) Representative: **advotec.**
**Patent- und Rechtsanwaltspartnerschaft mbB**
**Widenmayerstraße 4**
**80538 München (DE)**

Remarks:
The complete document including Reference Table(s) and the Sequence Listing(s) can be downloaded from the EPO website

# (54) IL13RA2-TARGETING HERPES SIMPLEX VIRUS AND ANTI-IL13RA2 ANTIBODY OR ANTIGEN-BINDING FRAGMENT THEREOF

(57) The present invention provides an IL13RA2-targeting herpes simplex virus in which an anti-IL13RA2 antibody or an antigen-binding fragment thereof is presented on the viral surface. Also provided is an anti-IL13RA2 antibody or an antigen-binding fragment thereof capable of specifically recognizing tumor cells.

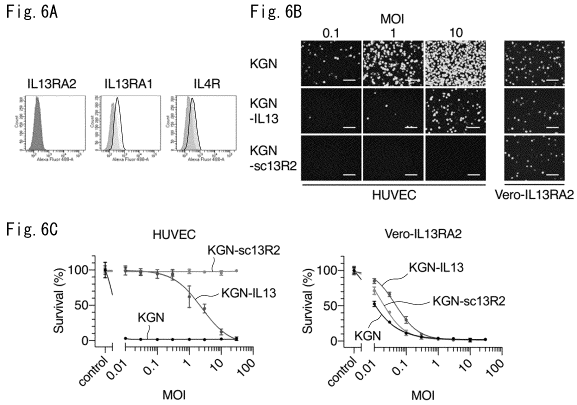


EP 4 596 687 A1

## Description

FIELD

The present invention relates to herpes simplex virus that can target and specifically lyse tumor cells expressing IL13RA2 by providing anti-IL13RA2 antibody on the virus surface. The present invention also relates to anti-IL13RA2 antibody or its antigen-binding fragment, that can specifically recognizes the target tumor cells.

BACKGROUND

It is a highly desired goal to develop novel treatment methods for cancer that efficiently kill only cancer cells. The interleukin 13 receptor subunit alpha 2 (IL13RA2) has been reported to be expressed in many types of solid tumors such as malignant glioma and malignant melanoma, and hematological tumors including cutaneous T cell lymphoma, and since its expression in normal cells is limited, it is considered promising as a target molecule for cancer treatment (NPLs 1 to 3). Since the dissociation constant for IL13RA2 and its ligand interleukin 13 (IL-13) is extremely low at less than 100 nM (NPL 4), it has been attempted to utilize IL-13 as a drug delivery module for development of cancer treatment methods targeting IL13RA2 (cancer targeting module). For example, a method of treatment for malignant glioma has been developed using recombinant protein (IL13-PE) having *Pseudomonas aeruginosa* exotoxin (PE) bonded to IL-13 (NPL 5). Unlike IL13RA2, however, the interleukin 13 receptor subunit alpha 1 (IL13RA1) which performs a physiological function as IL-13 receptor is also expressed in numerous normal cells including vascular endothelial cells (NPL 6), and while its dissociation constant is 1.69 nM, which is higher than IL13RA2, it is able to bind to IL-13 (NPL 4). It has also been reported that when IL13RA1 forms a complex with interleukin 4 receptor (IL4R), its binding capacity with IL-13 increases by a factor of 5 (NPL 7). It has therefore been pointed out that any method of treatment using IL-13 as a cancer targeting module to specifically kill cancer cells would entail a risk of side-effects due to damage to normal cells that express IL13RA1 (NPL 8). This has suggested a need for a different treatment scheme that involves specific recognition of cancer cells alone.

Oncolytic virus therapies are treatment methods for cancer using viruses that selectively proliferate in cancer cells. Herpes simplex virus (HSV) has the longest history of application as an oncolytic virus. Several research groups, including the research group of the present inventors, have reported on receptor retargeted oncolytic HSV (RR-oHSV), which allows infection via target molecules selectively expressed by cancer cells, rather than via HSV receptors, by genetic modification of HSV (NPLs 9 to 10). Specifically, these methods allow HSV to selectively invade cancer cells by modifying glycoprotein D (gD), which is essential for HSV to invade cells, to inactivate binding with herpesvirus entry mediator (HVEM), the original gD receptor, 3-OS-HS, and nectin-1, by fusing a cancer targeting module with gD. Using such methods, Zhou et al. have used developed IL13RA2-targeting RR-oHSV using IL-13 as a cancer targeting module (NPL 11). However, the infectivity or *in vivo* antitumor effect of IL-13-inserted RR-oHSV for IL13RA1-expressing cells has not been studied, and it appears that further research is necessary to estimate the potential for clinical development.

The present inventors have previously developed RR-oHSV that invades and propagates only in cells expressing target molecules, by inactivating its binding with HVEM and 3-OS-HS by a deletion of amino acids from position 2 to position 24 of gD, and with nectin-1 by a mutation of the amino acid at position 38 from tyrosine to cysteine, and then by inserting a single-chain antibody (scFv) into the deleted region from position 2 to position 24, as a cancer targeting module (PTL 1, NPL 12). RR-oHSV can be modified to target various molecules of interest by replacing the single-chain antibody-inserted in modified gD, and to date, we have successfully developed RR-oHSV for epidermal growth factor receptor (EGFR), carcinoembryonic antigen (CEA), epithelial cell adhesion molecule (EpCAM) and epiregulin (EREG) (NPLs 12 to 14).

[CITATION LIST]

[PATENT LITERATURE]

[PTL 1] International Patent Publication No. WO2020/090871

[NON PATENT LITERATURE]

[NPL 1] Liu, et al., Mol Cancer Ther. 2003 Aug; 2(8):783-7

[NPL 2] Beard, et al., Clin Cancer Res.2013 Sep 15; 19(18):4941-50.

[NPL 3] Geskin, et al., Blood 2015 Apr 30; 125(18):2798-805.

[NPL 4] Lupardus, et al., Structure. 2010 Mar 10; 18(3):332-42.

[NPL 5] Kunwar, et al., J Clin Oncol 2007 Mar 1; 25(7):837-44.
[NPL 6] Akaiwa, et al., Cytokine 2001 Jan 21; 13(2):75-84.
[NPL 7] Andrews, et al., J Biol Chem 2002 Nov 29; 277(48):46073-8.
[NPL 8] Balyasnikova, et al., J Biol Chem 2012 Aug 31; 287(36):30215-27.
[NPL 9] Uchida, et al., Curr Cancer Drug Targets 2018; 18(2):162-170.
[NPL 10] Menotti and Avitabile, Int J Mol Sci 2020 Nov 5; 21(21):8310.
[NPL 11] Zhou and Roizman, Proc Natl Acad Sci USA 2006 Apr 4; 103(14):5508-13.
[NPL 12] Uchida, et al., Mol Ther. 2013 Mar; 21(3):561-9.
[NPL 13] Shibata, et al., Gene Ther. 2016 23(6):479-88.
[NPL 14] Ikeda, et al., J Virol 2021 Apr 12; 95(9):e01766-20.

SUMMARY

[TECHNICAL PROBLEM]

One of the objectives of the present invention is to provide a herpes simplex virus which is highly safe and can specifically lyse tumor cells. Another objective of the invention is to provide a novel antibody and its antigen-binding fragment, which can specifically recognize tumor cells.

[SOLUTION TO PROBLEM]

It is necessary to acquire a module for targeting the antigen (for example, an antibody or its antigen-binding fragment) which enable the oncolytic HSV to become receptor retargeted oncolytic HSV (RR-oHSV). Such a module needs to be capable of transforming the HSV to use a tumor-associated antigen as the virus receptor, which can be specifically recognized and can complete the molecular mechanisms for the virus entry into target cells after the linkage. In previous research, the present inventors have in fact reported that not all the scFv that recognize target antigens are able to cause inactivation of gD, followed by stepwise activation of other envelope glycoproteins, gH/gL heterodimer and gB, to thereby establish HSV infiltration into the target cells (NPL 14). Even when the multiple scFv's that recognize target antigens are available, it is extremely difficult to predict which incorporated scFv's can lead to the establishment of HSV infiltration.

The present inventors therefore attempted to obtain an antibody and antigen-binding fragment that can specifically recognize IL13RA2 and have succeeded in obtaining anti-IL13RA2 antibody and its antigen-binding fragment. The present inventors found that the obtained anti-IL13RA2 antibody and its antigen-binding fragment can specifically bind to IL13RA2. Moreover, surprisingly, the anti-IL13RA2 antibody was found to be efficiently internalized in tumor cells.

Upon further research, the present inventors created a recombinant herpes simplex virus displaying an antigen-binding fragment of the aforementioned anti-IL13RA2 antibody on its surface and found that it infects and lyses IL13RA2-positive cells with higher efficiency and exhibits higher IL13RA2 selectivity, compared to recombinant herpes simplex virus displaying IL-13, the original ligand, on its surface.

Specifically, the present invention encompasses the following aspects.

[1] IL13RA2-targeting herpes simplex virus displaying anti-IL13RA2 antibody or its antigen-binding fragment on the virus surface.

[2] The herpes simplex virus according to [1] above, wherein the heavy chain CDRs (HCDR1, HCDR2, HCDR3) and light chain CDRs (LCDR1, LCDR2, LCDR3) of the anti-IL13RA2 antibody or its antigen-binding fragment have the amino acid sequences:

(i) HCDR1: RHGIH (SEQ ID NO: 1)
(ii) HCDR2: VIWAGGSTNYNSALMS (SEQ ID NO: 2)
(iii) HCDR3: DHFDSFDY (SEQ ID NO: 3)
(iv) LCDR1: TASSSVSSSYLH (SEQ ID NO: 4)
(v) LCDR2: STSNLAS (SEQ ID NO: 5) and
(vi) LCDR3: HQYHRSPLT (SEQ ID NO: 6),

or these amino acid sequences having a mutation of one or more amino acids.

[3] The herpes simplex virus according to [1] or [2] above, wherein the anti-IL13RA2 antibody or its antigen-binding fragment comprises:

a sequence having at least 80% sequence identity with the amino acid sequence of the $V_L$ region listed as SEQ ID

NO: 8, and
a sequence having at least 80% sequence identity with the amino acid sequence of the $V_H$ region listed as SEQ ID NO: 10.

[4] The herpes simplex virus according to any one of [1] to [3] above, wherein the antigen-binding fragment is Fab, Fab', F(ab')2, Fv, scFv, dsFv, bis-scFv, a diabody, a triabody or a tetrabody.
[5] The herpes simplex virus according to any one of [1] to [4] above, wherein the antigen-binding fragment is scFv.
[6] The herpes simplex virus according to any one of [1] to [5] above, wherein the antigen-binding fragment contains a sequence having at least 90% sequence identity with the sequence of SEQ ID NO: 7 (scFv sequence).
[7] The herpes simplex virus according to any one of [1] to [6] above, wherein the anti-IL13RA2 antibody or its antigen-binding fragment is a chimerized or humanized anti-IL13RA2 antibody or its antigen-binding fragment.
[8] The herpes simplex virus according to any one of [1] to [7] above, wherein the anti-IL13RA2 antibody or its antigen-binding fragment is incorporated into an envelope protein.
[9] The herpes simplex virus according to [8] above, wherein the anti-IL13RA2 antibody or its antigen-binding fragment is incorporated into envelope protein gD, envelope protein gB, envelope protein gC or envelope protein gH.
[10] The herpes simplex virus according to [8] above, wherein the anti-IL13RA2 antibody or its antigen-binding fragment is incorporated into envelope protein gD.
[11] The herpes simplex virus according to [8] above, wherein the anti-IL13RA2 antibody or its antigen-binding fragment is incorporated into the deleted portion of the amino acids from position 2 to position 24 of SEQ ID NO: 12 of envelope protein gD, or between position 24 and position 25, into the deleted portion of the amino acids from position 6 to position 38, or into the deleted portion of the amino acids from position 61 to position 218, of SEQ ID NO: 12.
[12] The herpes simplex virus according to any one of [1] to [11] above, wherein the envelope protein gD has an amino acid mutation that weakens infectivity into cells expressing Nectin-1, HVEM and/or 3-OS-HS.
[13] The herpes simplex virus according to [12] above, wherein the amino acid mutation that weakens infectivity into cells expressing Nectin-1 is one or more selected from the group consisting of a deletion of all of the amino acids from position 6 to position 38 of SEQ ID NO: 12, a deletion of all of the amino acids from position 61 to position 218 of SEQ ID NO: 12, a mutation of the amino acid at position 3 and/or position 38 of SEQ ID NO: 12 and a mutation of the amino acid at position 222 and position 223 of SEQ ID NO: 12.
[14] The herpes simplex virus according to [13] above, wherein the amino acid mutation at position 3 of SEQ ID NO: 12 is a deletion or a substitution to cysteine, the amino acid mutation at position 38 is a substitution to cysteine, the amino acid mutation at position 222 is a substitution to asparagine and the amino acid mutation at position 223 is a substitution to isoleucine.
[15] The herpes simplex virus according to any one of [12] to [14] above, wherein of the amino acid mutations of envelope protein gD, the amino acid mutation that weakens infectivity into cells expressing HVEM and/or 3-OS-HS is a deletion of all or some of the amino acids from position 2 to position 38 of SEQ ID NO: 12, a deletion of all of the amino acids from position 61 to position 218 of SEQ ID NO: 12, an amino acid mutation at position 27 of SEQ ID NO: 12, an amino acid mutation at position 29 of SEQ ID NO: 12, and/or an amino acid mutation at position 30 of SEQ ID NO: 12.
[16] The herpes simplex virus according to [15] above, wherein a portion of the deletion of amino acids from position 2 to position 38 of SEQ ID NO: 12 is a deletion of amino acids from position 2 to position 24, a deletion of amino acids from position 7 to position 11, a deletion of amino acids from position 7 to position 32, or a deletion of amino acids from position 6 to position 38, the amino acid mutation at position 27 is a substitution to alanine, proline or arginine, the amino acid mutation at position 29 is a substitution to alanine, and the amino acid mutation at position 30 is a substitution to alanine.
[17] The herpes simplex virus according to any one of [1] to [16] above, wherein the envelope protein gB, envelope protein gK, UL20 protein and/or UL24 protein of the herpes simplex virus have an amino acid mutation that promotes entry into target cells and/or an amino acid mutation that promotes multinucleated giant cell formation.
[18] The herpes simplex virus according to [16] above, wherein the amino acid mutation that promotes entry into target cells and/or the amino acid mutation that promotes multinucleated giant cell formation is a mutation according to the following (a), (b), (c) and/or (d).

(a) an amino acid mutation at position 285, an amino acid mutation at position 549, an amino acid mutation at position 796, an amino acid mutation at position 800, an amino acid mutation at position 813, an amino acid mutation at position 817, an amino acid mutation at position 854, an amino acid mutation at position 855, an amino acid mutation at position 858, an insertion of an amino acid between position 816 and position 817, a nonsense mutation of the amino acid at position 869 and/or a nonsense mutation of the amino acid at position 877, of SEQ ID NO: 13,
(b) an amino acid mutation at position 33, an amino acid mutation at position 40, an amino acid mutation at position 86, an amino acid mutation at position 99, an amino acid mutation at position 111, an amino acid mutation at

position 121, an amino acid mutation at position 243, an amino acid mutation at position 304 and/or an amino acid mutation at position 310, of SEQ ID NO: 14,
(c) an amino acid mutation at position 49, amino acid mutations at positions 49, 50 and 51, an amino acid mutation at position 209, amino acid mutations at positions 209, 212 and 213 or a nonsense mutation of the amino acid at position 217, of SEQ ID NO: 15, and/or a deletion of the entire amino acid sequence listed as SEQ ID NO: 15, and
(d) amino acid mutations at positions 62, 63 and 64 of SEQ ID NO: 16.

[19] The herpes simplex virus according to [18] above, wherein

the amino acid mutation at position 285 of SEQ ID NO: 13 is a substitution to asparagine, the amino acid mutation at position 549 of SEQ ID NO: 13 is a substitution to threonine, the amino acid mutation at position 796 of SEQ ID NO: 13 is a substitution to cysteine, the amino acid mutation at position 800 of SEQ ID NO: 13 is a substitution to tryptophan, the amino acid mutation at position 813 of SEQ ID NO: 13 is a substitution to isoleucine, the amino acid mutation at position 817 of SEQ ID NO: 13 is a substitution to histidine or proline, the amino acid mutation at position 854 of SEQ ID NO: 13 is a substitution to phenylalanine, the amino acid mutation at position 855 of SEQ ID NO: 13 is a substitution to valine, and the amino acid mutation at position 858 of SEQ ID NO: 13 is a substitution to cysteine or histidine,
the amino acid mutation at position 33 of SEQ ID NO: 14 is a substitution to serine, the amino acid mutation at position 40 of SEQ ID NO: 14 is a substitution to valine or threonine, the amino acid mutation at position 86 of SEQ ID NO: 14 is a substitution to proline, the amino acid mutation at position 99 of SEQ ID NO: 14 is a substitution to asparagine, the amino acid mutation at position 111 of SEQ ID NO: 14 is a substitution to valine, the amino acid mutation at position 121 of SEQ ID NO: 14 is a substitution to isoleucine, the amino acid mutation at position 243 of SEQ ID NO: 14 is a substitution to tyrosine, the amino acid mutation at position 304 of SEQ ID NO: 14 is a substitution to proline, and the amino acid mutation at position 310 of SEQ ID NO: 14 is a substitution to leucine,
the amino acid mutation at position 49 of SEQ ID NO: 15 is a substitution to alanine, the amino acid mutation at position 50 of SEQ ID NO: 15 is a substitution to alanine, the amino acid mutation at position 51 of SEQ ID NO: 15 is a substitution to alanine, the amino acid mutation at position 209 of SEQ ID NO: 15 is a substitution to alanine, the amino acid mutation at position 212 of SEQ ID NO: 15 is a substitution to alanine, and the amino acid mutation at position 213 of SEQ ID NO: 15 is a substitution to alanine, and
the amino acid mutation at position 62 of SEQ ID NO: 16 is a substitution to glycine, the amino acid mutation at position 63 is a substitution to valine and the amino acid mutation at position 64 is a substitution to serine.

[20] The herpes simplex virus according to any one of [1] to [19] above, which has a restricted growth modification.
[21] The herpes simplex virus according to [20] above, wherein the restricted growth modification is a mutation that inactivates expression of functional ICP34.5.
[22] Nucleic acid coding for a herpes simplex virus according to any one of [1] to [21] above.
[23] Cells producing a herpes simplex virus according to any one of [1] to [21] above.
[24] A method for producing a herpes simplex virus according to any one of [1] to [21] above.
[25] A pharmaceutical composition comprising a herpes simplex virus according to any one of [1] to [21] above.
[26] The pharmaceutical composition according to [25] above, which is a therapeutic agent for a tumor that expresses IL13RA2.
[27] The pharmaceutical composition according to [26] above, wherein the tumor is melanoma, osteosarcoma, leukemia, lymphoma, prostate cancer, lung cancer (including malignant mesothelioma), glioma, head or neck cancer, renal carcinoma, Kaposi's sarcoma, colorectal cancer, pancreatic carcinoma, adrenal cancer, breast cancer or ovarian cancer.
[28] An anti-IL13RA2 antibody or its antigen-binding fragment, wherein the heavy chain CDRs (HCDR1, HCDR2, HCDR3) and light chain CDRs (LCDR1, LCDR2, LCDR3) have the amino acid sequences:

(i) HCDR1: RHGIH (SEQ ID NO: 1)
(ii) HCDR2: VIWAGGSTNYNSALMS (SEQ ID NO: 2)
(iii) HCDR3: DHFDSFDY (SEQ ID NO: 3)
(iv) LCDR1: TASSSVSSSYLH (SEQ ID NO: 4)
(v) LCDR2: STSNLAS (SEQ ID NO: 5) and
(vi) LCDR3: HQYHRSPLT (SEQ ID NO: 6),

or these amino acid sequences having a mutation of one or more amino acids.
[29] The anti-IL13RA2 antibody or its antigen-binding fragment according to [28] above, wherein the anti-IL13RA2 antibody or its antigen-binding fragment comprises:

a sequence having at least 80% sequence identity with the amino acid sequence of the $V_L$ region listed as SEQ ID NO: 8, and
a sequence having at least 80% sequence identity with the amino acid sequence of the $V_H$ region listed as SEQ ID NO: 10.

[30] The anti-IL13RA2 antibody or its antigen-binding fragment according to [28] or [29] above, wherein the antigen-binding fragment is Fab, Fab', F(ab')2, Fv, scFv, dsFv, bis-scFv, a diabody, a triabody or a tetrabody.
[31] The anti-IL13RA2 antibody or its antigen-binding fragment according to any one of [28] to [30] above, wherein the antigen-binding fragment contains a sequence having at least 90% sequence identity with the sequence of SEQ ID NO: 7 (scFv sequence).
[32] The anti-IL13RA2 antibody or its antigen-binding fragment according to any one of [28] to [31] above, which is chimerized or humanized.
[33] Nucleic acid coding for an anti-IL13RA2 antibody or its antigen-binding fragment according to any one of [28] to [32] above.
[34] Cells producing an anti-IL13RA2 antibody or its antigen-binding fragment, and containing nucleic acid according to [33] above.
[35] Cell producing an anti-IL13RA2 antibody or its antigen-binding fragment according to any one of [28] to [32] above.
[36] Cells deposited under Accession No. NITE BP-03673.
[37] A method for producing an anti-IL13RA2 antibody or its antigen-binding fragment according to any one of [27] to [31] above using cells according to any one of [34] to [36] above.
[38] A pharmaceutical composition comprising an anti-IL13RA2 antibody or its antigen-binding fragment according to any one of [28] to [32] above.
[39] The pharmaceutical composition according to [38] above, which is for treatment of a tumor that expresses IL13RA2.
[40] The pharmaceutical composition according to [39] above, wherein the tumor is melanoma, osteosarcoma, leukemia, lymphoma, prostate cancer, lung cancer (including malignant mesothelioma), glioma, head or neck cancer, renal carcinoma, Kaposi's sarcoma, colorectal cancer, pancreatic carcinoma, adrenal cancer, breast cancer or ovarian cancer.
[41] The pharmaceutical composition according to any one of [38] to [40] above, wherein the anti-IL13RA2 antibody or its antigen-binding fragment is included in a pharmaceutical composition in the form of an antibody-drug conjugate.

[ADVANTAGEOUS EFFECTS OF INVENTION]

According to the invention, it is possible to selectively and efficiently lyse and kill IL13RA2-positive cancer cells. The invention can further provide novel treatment means that can avoid potential side-effects, is safe for the human body and can be administered at low dosage.

BRIEF DESCRIPTION OF DRAWINGS

Fig. 1 is a schematic diagram showing the structure of the IL-13-inserted HSV genome and targeted gD mutant. Shown are the structures of the genome of IL-13-inserted HSV and the IL-13-inserted gD mutant. $U_L$: unique long segment; $U_S$: unique short segment; CMVp: human cytomegalovirus major immediate early (HCMV-IE) promoter; EGFP: EGFP expression cassette inserted between UL3 and UL4; NT: intracellular entry-promoting mutation (D285 N/A549T) in gB; gD-IL13: IL-13-inserted gD; black squares: terminal and internal inverted repeats; SP: signal peptide; TM: transmembrane domain; CT: cytosolic end; N: amino terminal; C: carboxy terminal.
Fig. 2 shows target specificity at the stage of intracellular entry of IL-13-inserted HSV. Intracellular entry of IL-13-inserted HSV into the J1.1-2 cell subline having forced expression of gD receptor or IL13RA2 was examined. The EGFP fluorescent signal was observed 8 hours after infection of the cells shown in the bottom panels with the viruses shown in the left panels. Bars: 300 $\mu$m.
Fig. 3 shows specificity of intracellular entry of IL-13-inserted HSV using CHO-K1 cells and their sublines. Intracellular entry of IL-13-inserted HSV into the CHO-K1 cell sublines having forced expression of gD receptor or IL13RA2 was examined. The EGFP fluorescent signal was observed 10 hours after infection of the cells shown in the top panels with the viruses shown in the left panels, under conditions of 30 gc/cell. Bars: 300 $\mu$m.
Fig. 4 shows target specificity with stepwise intracellular entry of IL-13-inserted RR-oHSV and anti-IL13RA2 single-chain antibody-inserted RR-oHSV. Intracellular entry of IL-13- or anti-IL13RA2 single-chain antibody-inserted RR-oHSV into J1.1-2 cell sublines having forced expression of gD receptor, IL13RA2 or IL13RA1 and IL4R was examined. The EGFP fluorescent signal was observed 8 hours after infection of the cells shown in the bottom panels with the viruses shown in the left panels. Bars: 300 μm.

Fig. 5 shows specificity with intracellular entry of IL-13-inserted HSV and anti-IL13RA2 single-chain antibody-inserted HSV into CHO-K1 cells and their sublines. Intracellular entry of IL-13- or anti-IL13RA2 single-chain antibody-inserted HSV into CHO-K1 cell sublines having forced expression of gD receptor, IL13RA2 and IL13RA1 and IL4R was examined. The EGFP fluorescent signal was observed 8 hours after infection of the cells shown in the top panels with the viruses shown in the left panels, under conditions of MOI 1. Bars: 300 μm.

Fig. 6 shows infectivity of IL-13-inserted HSV and anti-IL13RA2 single-chain antibody-inserted HSV into human umbilical cord venous vascular endothelial cells. (a) Expression of IL13RA2, IL13RA1 and IL4R in HUVEC. The mouse-derived antibodies for the molecules shown at the top panels and Alexa Fluor 488-labeled anti-mouse IgG antibody were used for staining of HUVEC, and flow analysis was carried out. The gray histogram is an isotype-matching negative control antibody. (b) Intracellular entry into HUVEC. The viruses shown at the left panels were infected into HUVEC or Vero-IL13RA2 with the MOI shown at the top panels, and the fluorescent signal was observed after 12 hours. Vero-IL13RA2 was infected with the same virus solution used to infect HUVEC at MOI 0.1. Bars: 300 μm. (c) Cytotoxicity for HUVEC. The cells shown at the top graph were infected with KGN, KGN-IL13 and KGN-sc13R2, and MTT assay was conducted after 4 days. The cell count relative to non-infectious conditions (control) was recorded as the viable cell count (%). The error bars represent standard deviation (n = 6).

Fig. 7 shows expression of IL13RA2 in U87 cells and H1299 cells. Mouse anti-IL13RA2 antibody SHM38 and Alexa Fluor 488-labeled goat anti-mouse IgG antibody were used for staining, and flow analysis was carried out. The gray histogram is an isotype-matching negative control antibody.

Fig. 8 shows target specificity for intracellular entry and intercellular propagation of anti-IL13RA2 single-chain antibody-inserted RR-oHSV. (a) Expression of IL13RA2 in human cancer cell lines. The 4E6 antibody and Alexa Fluor 488-labeled anti-mouse IgG antibody were used for staining of the human cancer cells shown in the top panels, and flow analysis was carried out. The gray histogram is an isotype-matching negative control antibody. (b) Intracellular entry into human cancer cell lines. The EGFP fluorescent signal was observed 6 hours after infection of the human cancer cell lines shown in the top panels with the viruses shown in the left panels at MOI 1. Bars: 300 μm. (c) Intercellular propagation in human cancer cell lines. Vero-IL13RA2 cells infected with the viruses shown at the left panels were treated by acidic treatment to inactivate the virus remaining outside the cells, and were overlaid on the monolayer cultured cell lines shown at the top panels at 100 cells/well. Shown are the EGFP fluorescent signals after culturing for 36 hours in methyl cellulose-containing medium. Arrow heads: Monoinfected cells; Bars: 300 μm.

Fig. 9 shows effects of BhKt mutation on intercellular propagation and cytotoxicity of anti-IL13RA2 single-chain antibody-inserted RR-oHSV in IL13RA2-positive human cancer cells. (a) Plaque size in IL13RA2-positive cancer cell lines. The monolayer cultured IL13RA2-positive cancer cells shown in the top graphs were infected with the viruses shown in the bottom graphs, and the plaque area was determined from the EGFP fluorescent signal after culturing for 3 days in methyl cellulose-containing medium. Shown are the mean (gray lines) ±SD (black lines) for plaque area (n = 15). A two-tailed Mann-Whitney U test was used for statistical analysis. *: $P < 0.05$. (b) Cytotoxicity for IL13RA2-positive cancer cell line. The cells shown in the top graph were infected with KGN-sc13R2 (black lines) or KGN-sc13R2-BhKt (gray lines), and MTT assay was carried out after 4 days. The cell count relative to non-infectious conditions (control) was recorded as the viable cell count (%). The error bars represent standard deviation (n = 6).

Fig. 10 shows the antitumor effect of KGN-sc13R2-BhKt for immunodeficient mice with cancer. (a) Antitumor effect of intratumoral administration for subcutaneous tumor model of U87(+) cells. When the tumor volume reached about 290 $mm^3$, PBS (black circles; n = 6) or KGN-sc13R2-BhKt at $10^2$ pfu (gray circles; n = 6) was administered into the tumor (arrows), and the tumor volume was subsequently monitored. (b) Antitumor effect of intratumoral administration for subcutaneous tumor model of H1299(+) cells. When the tumor volume reached about 150 $mm^3$, PBS (black circles; n = 6) or KGN-sc13R2-BhKt at $10^2$ (gray circles; n = 6) or $10^4$ (triangles; n = 6) pfu was administered into the tumor (arrows), and the tumor volume was subsequently monitored. (c) Antitumor effect of intravenous administration for subcutaneous tumor model of U87(+) cells. When the tumor volume reached about 290 $mm^3$, PBS (black circles; n = 6) or KGN-sc13R2-BhKt at $10^6$ (gray circles; n = 6) pfu was intravenously administered (arrows), and the tumor volume was subsequently monitored. Shown are the mean and standard error for the tumor volume. Two-way repeated measurement distributed analysis was used for statistical analysis.

Fig. 11 shows the antitumor effect of a virus with deletion of ICP34.5 of KGN-sc13R2-BhKt (KGΔN-sc13R2-BhKt) on a U87-IL13RA2+ subcutaneous tumor model. U87-IL13RA2+ was subcutaneously grafted into SCID Beige mice, and upon reaching a tumor volume of about 290 $mm^3$, PBS or each virus at $10^2$ pfu was administered into the tumor. The arrows indicate the day of administration (9 days after grafting). The error bars represent standard error (n = 6). (a) Change in mean tumor volume ($mm^3$) of mice administered PBS, KGN-sc13R2-BhKt or KGΔN-sc13R2-BhKt. Change in tumor volume ($mm^3$) of mice administered (b) PBS, (c) KGN-sc13R2-BhKt or (d) KGΔN-sc13R2-BhKt.

DESCRIPTION OF EMBODIMENTS

The invention will now be described in greater detail by concrete embodiments. However, the invention is not restricted to the described embodiments and may be carried out in any form within a range that is not outside of the gist of the invention.

The published data sources cited throughout the present disclosure, such as patent publications, patent application publications, non-patent literature and sequence accession numbers, are incorporated herein in their entirety by reference, for any purpose.

For the purpose of the present disclosure, the word "to" when used between numerical values indicates a range that is above and including the lower value and is below and including the upper value. The terms "about" or "approximately" as used herein encompass a range of $\pm$10% of the indicated numerical value.

Throughout the present specification, reference to an amino acid mutation, unless otherwise specified, means a substitution or deletion of the amino acid, or an insertion of one or more (such as about 1 to 10, preferably 1 to 5 and more preferably 1 to 3) amino acids between the amino acid and an adjacent amino acid. For example, a "mutation of R21" in reference to a given sequence means a deletion of the amino acid at position 21 of the amino acid sequence, a substitution thereof with another amino acid, or an insertion of one or more amino acids between the amino acid at position 21 and either adjacent amino acid. For this example, the amino acid number mentioned herein may shift by about 1 to 10 for different HSVs. For an arbitrary HSV, therefore, if the amino acid number shifts by $\pm$n (where n is an integer of 1 to 10), R21 may be read as R(21 $\pm$n).

IL13RA2-targeting herpes simplex virus

As used herein, the term "IL13RA2-targeting herpes simplex virus" means herpes simplex virus manipulated so as to specifically bind and infect IL13RA2-positive cancer cells. The gene for the IL13RA2-targeting herpes simplex virus is modified by genetic engineering.

The herpes simplex virus to be used for the invention is an oncolytic virus. The term "oncolytic virus", as used for the purpose of the invention, refers to a virus modified so as to preferentially infect cancer cells and kill the cancer cells.

The IL13RA2-targeting herpes simplex virus of the invention displays the anti-IL13RA2 antibody or its antigen-binding fragment, which binds IL13RA2, on the herpes simplex virus surface. The anti-IL13RA2 antibody or its antigen-binding fragment can be incorporated into an envelope protein, as a glycoprotein, exposed on the virus surface, in order to promote targeting of the desired cells. Methods for incorporating the anti-IL13RA2 antibody or its antigen-binding fragment are known, and such methods include a method of using a cross linker such as glutaraldehyde, a method of using a tag (an epitope such as biotin, glutathione or Myc, or 6xhistidine) with its ligand, a method of using a multiple-specific antibody (for example, a bispecific antibody) that recognizes two antigens, i.e. the virus envelope protein and the anti-IL13RA2 antibody or its antigen-binding fragment, and a method of expression as a fusion protein of the anti-IL13RA2 antibody or its antigen-binding fragment with an envelope protein, the preferred method being expression as a fusion protein with an envelope protein. The anti-IL13RA2 antibody or its antigen-binding fragment is produced in a recombinant virus so that it is displayed on the outer side of the viral envelope.

As used herein, the term "IL-13" includes cytokines secreted primarily by T helper 2 cells. IL-13, together with IL-4 which shares a receptor (IL-13R$\alpha$1/IL-4R$\alpha$), performs an important role in eliciting humoral immunity by antibody production. IL-13 is a 13 kDa polypeptide monomer protein, the structure of IL-13 being described in detail in Moy, Diblasio et al. 2001, J Mol Biol 310 p.219-30, for example.

IL13RA2 is known as a high affinity receptor of IL-13, and it functions as a decoy for IL-13 while also inhibiting signal transduction of IL-4 via STAT6, and it has recently been reported to be involved in numerous signaling paths (such as WNT/$\beta$-Catenin, MAPK/ERK, AKT/PKB, Src/FAK and PIP3K), while also possibly performing a protective role against inflammatory disease such as dermatitis (Sivaprasad et al., J Immunol. 2010; 185(11): 6802). For example, the precursor of human IL13RA2 (another form of IL13RA2) may be a polypeptide having the amino acid sequence listed as SEQ ID NO: 17 (NCBI Accession No.: NP_000631.1). The precursor of human IL13RA2 listed as SEQ ID NO: 17 includes the signal peptide (positions 1 to 26), and mature human IL13RA2 corresponds to position 27 to position 380 of the amino acid sequence listed as SEQ ID NO: 17.

In the anti-IL13RA2 antibody or its antigen-binding fragment displayed on the IL13RA2-targeting herpes simplex virus of the invention, or another form of the anti-IL13RA2 antibody or its antigen-binding fragment of the invention, the heavy chain CDRs (HCDR1, HCDR2, HCDR3) and light chain CDRs (LCDR1, LCDR2, LCDR3) have the respective amino acid sequences:

(i) HCDR1: (SEQ ID NO: 1: RHGIH)
(ii) HCDR2: (SEQ ID NO: 2: VIWAGGSTNYNSALMS)
(iii) HCDR3: (SEQ ID NO: 3: DHFDSFDY)

(iv) LCDR1: (SEQ ID NO: 4: TASSSVSSSYLH)
(v) LCDR2: (SEQ ID NO: 5: STSNLAS) and
(vi) LCDR3: (SEQ ID NO: 6: HQYHRSPLT),

or these amino acid sequences having a mutation of one or more amino acids. A "CDR" is a region among the variable regions of an immunoglobulin molecule that forms an antigen binding site, and it is a portion that undergoes the greatest degree of change in amino acid sequence for each immunoglobulin molecule, being also referred to as a "hypervariable region". The CDR includes three heavy chains (HCDR) and light chains (LCDR) each (respectively CDR1, CDR2 and CDR3). For the present application, the CDRs of an immunoglobulin molecule are identified according to the Kabat numbering system (Kabat et al., 1987, Sequences of Proteins of Immunological Interest, US Department of Health and Human Services, NIH, USA).

In one form of the anti-IL13RA2 antibody or its antigen-binding fragment displayed on the IL13RA2-targeting herpes simplex virus of the invention, the heavy chain CDRs (HCDR1, HCDR2, HCDR3) have the respective amino acid sequences:

(i) HCDR1: (SEQ ID NO: 1: RHGIH)
(ii) HCDR2: (SEQ ID NO: 2: VIWAGGSTNYNSALMS)
(iii) HCDR3: (SEQ ID NO: 3: DHFDSFDY),

or these amino acid sequences with one or more (one, two or three) amino acid mutations.

In another form of the anti-IL13RA2 antibody or its antigen-binding fragment displayed on the IL13RA2-targeting herpes simplex virus of the invention, the light chain CDRs (LCDR1, LCDR2, LCDR3) have the respective amino acid sequences:

(i) LCDR1: (SEQ ID NO: 4: TASSSVSSSYLH)
(ii) LCDR2: (SEQ ID NO: 5: STSNLAS)
(iii) LCDR3: (SEQ ID NO: 6: HQYHRSPLT),

or these amino acid sequences with one or more (one, two or three) amino acid mutations.

One form of the anti-IL13RA2 antibody or its antigen-binding fragment displayed on the IL13RA2-targeting herpes simplex virus of the invention may be an anti-IL13RA2 antibody or its antigen-binding fragment comprising a sequence having at least 80% (such as at least 85%, at least 90%, at least 95%, at least 98%, at least 99%, or 100%) sequence identity with the amino acid sequence of the $V_L$ region listed as SEQ ID NO: 8, and a sequence having at least 80% (such as at least 85%, at least 90%, at least 95%, at least 98%, at least 99% or 100%) sequence identity with the amino acid sequence of the $V_H$ region listed as SEQ ID NO: 10.

As used herein, the meanings of "substitution" in which an amino acid mutation to a CDR is replaced with another amino acid, "deletion" in which the amino acid is removed, and "insertion" in which another amino acid is added, are most preferably "conservative amino acid substitutions". A conservative amino acid substitution is determined based on similarity with the properties of polarity, charge, solubility, hydrophobicity, hydrophilicity and/or amphiphilicity of the residue in question. For example, non-polar (hydrophobic) amino acids include alanine (Ala, A), leucine (Leu, L), isoleucine (Ile, I), valine (Val, V), proline (Pro, P), phenylalanine (Phe, F), tryptophan (Trp, W) and methionine (Met, M); polar neutral amino acids include glycine (Gly, G), serine (Ser, S), threonine (Thr, T), cysteine (Cys, C), tyrosine (Tyr, Y), asparagine (Asn, N) and glutamine (Gln, Q); positively charged (basic) amino acids include arginine (Arg, R), lysine (Lys, K) and histidine (His, H); and negatively charged (acidic) amino acids include aspartic acid (Asp, D) and glutamic acid (Glu, E). The terms "substitution", "insertion" and "deletion" all have a preferred range of 1, 2 or 3 amino acids. Mutation may be introduced by using a recombinant DNA method for systematic or random substitution of amino acids of a polypeptide molecule and carrying out assay of recombinant mutants obtained by activation.

According to an exemplary aspect, the anti-IL13RA2 antibody which is displayed on the IL13RA2-targeting herpes simplex virus of the invention may include a mouse constant region (IgG1, IgG2a, IgG2b, IgG3, IgM, IgD, IgE, IgA). According to a partial aspect, the antibody may be a chimeric antibody, humanized antibody or human antibody having a human constant region (IgG1, IgG2, IgG3, IgG4, IgM, IgD, IgE, IgA1, IgA2). The mouse antibody, chimeric antibody, humanized antibody or human antibody may have only the heavy chain, for example, displayed on the virus surface. According to yet another partial aspect, the antibody may be an antibody with double specificity, triple specificity or multi specificity (such as a bispecific antibody, trispecific antibody or multispecific antibody), in order to increase the affinity or avidity.

According to one embodiment, the antigen-binding fragment to be used for the invention may be any antigen-binding fragment of an antibody described throughout the present specification. Antigen-binding fragments include Fab, Fab', F(ab')2, Fv, scFv, dsFv, bis-scFv, diabodies, triabodies and tetrabodies, with no limitation to these so long as the

antigen-binding fragment is derived from anti-IL13RA2 antibody. Diabodies (dimers), triabodies (trimers) and tetrabodies (tetramers) are known technologies. Exemplary references include Kortt et al., Biomol. Eng. 2001, 18:95-108, (2001) and Todorovska et al., J. Immunol Methods. 248:47-66, (2001). According to one embodiment, the antigen-binding fragment used for the invention is preferably scFv.

The fragment scFv comprises an antibody light chain variable (V) region ($V_L$ region) linked to an antibody heavy chain V region ($V_H$ region) via a synthetic peptide (linker), and can be formed using common recombinant DNA technology (see Janeway et al., Immunobiology, 2nd Edition, Garland Publishing, New York (1996), for example). Examples of linkers include, but are not limited to, the flexible linker $(Gly_4Ser)_3$. For scFv, the order of linkage of the $V_L$ region and $V_H$ region connected by the linker is not particularly restricted. A disulfide-stabilized variable region fragment (dsFv) may likewise be prepared by recombinant DNA technology (Reiter et al., Protein Engineering, 7, 697-704(1994), for example).

According to one embodiment, the antigen-binding fragment to be used for the invention may be an antigen-binding fragment containing a sequence having at least 90%, at least 91%, at least 92%, at least 93%, at least 94%, at least 95%, at least 96%, at least 97%, at least 98%, at least 99%, or 100% sequence identity to the sequence listed as SEQ ID NO: 7. Specifically, the amino acid sequence listed as SEQ ID NO: 7 is a sequence including the following amino acid sequence.

[Chemical Formula 1]

D I V L T Q T P A I M S A S L G E R V T M T C T A S S S V S S S Y L H W Y Q Q K

<$V_L$ domain: SEQ ID NO: 8>

P G S S P K L W I Y S T S N L A S G V P L R F S G S G S G T S Y S L T I S S M E

A E D A A T Y Y C H Q Y H R S P L T F G A G T K L E L K R A A S G G G G S G G G

<Linker Sequence 1:

G S G G G G S E V K L E E S G P G L V A P S Q S L S I A C T V S G F S L T R H G

SEQ ID NO: 9> <$V_H$ domain: SEQ ID NO: 10>

I H W V R Q P P G K N L E W L G V I W A G G S T N Y N S A L M S R L S I I K D N

S K S Q V F L K M N S L Q T V D T A M Y Y C A R D H F D S F D Y W G Q G T T L T

V S S A G G G G S G S (SEQ ID NO: 7)

<Linker Sequence 2: SEQ ID NO: 11>

An antigen-binding fragment having the amino acid sequence listed as SEQ ID NO: 7 includes the $V_L$ region including LCDR1 to LCDR3 (SEQ ID NO: 4 to 6) (SEQ ID NO: 8), the $V_H$ region including HCDR1 to HCDR3 (SEQ ID NO: 1 to 3) (SEQ ID NO: 10), and linker sequences 1 and 2 (SEQ ID NO: 9 and 11).

According to a partial aspect of the disclosure, the antigen-binding fragment displayed on the IL13RA2-targeting herpes simplex virus of the invention may be an antigen-binding fragment wherein the heavy chain CDRs (HCDR1, HCDR2, HCDR3) and light chain CDRs (LCDR1, LCDR2, LCDR3) have the amino acid sequences:

(i) HCDR1: (SEQ ID NO: 1: RHGIH)

(ii) HCDR2: (SEQ ID NO: 2: VIWAGGSTNYNSALMS)
(iii) HCDR3: (SEQ ID NO: 3: DHFDSFDY)
(iv) LCDR1: (SEQ ID NO: 4: TASSSVSSSYLH)
(v) LCDR2: (SEQ ID NO: 5: STSNLAS) and
(vi) LCDR3: (SEQ ID NO: 6: HQYHRSPLT),

or these amino acid sequences having a mutation of one or more amino acids,
and having at least 80%, at least 85%, at least 90%, at least 95%, at least 98%, at least 99%, or 100% sequence identity with the sequence listed as SEQ ID NO: 7.

The term "sequence identity", for the purpose of the invention, is defined as the percentage of amino acid residues among a candidate sequence that are identical to the amino acid residues of the specific reference polypeptide sequence, after aligning the sequences and introducing gaps as necessary to obtain maximum sequence identity, without conservative substitutions being considered in the sequence identity. The alignment for measurement of the sequence identity can be achieved by a method within the scope of the ability of a person skilled in the art using publicly available computer software such as BLAST, BLAST-2, ALIGN or Megalign (DNASTAR) software, for example. A person skilled in the art can set suitable parameters for sequence alignment, including any algorithm required to achieve maximum alignment with respect to the full lengths of the sequences being compared. For the present purpose, however, the sequence identity value is obtained using the sequence comparison computer program BLAST for pairwise alignment.

Under the conditions employed by BLAST for amino acid sequence comparison, the sequence identity (%) between a given amino acid sequence A and a given amino acid sequence B is calculated as follows:

$$\text{Fraction } X/Y \times 100.$$

Here, X is the number of amino acid residues of the identical matching score based on program alignment of A and B in the sequence alignment program BLAST, while Y is the total number of amino acid residues. It will be understood that when the length of amino acid sequence A differs from the length of the amino acid sequence B, the sequence identity of A with respect to B is different from the sequence identity (%) of B with respect to A. Unless otherwise specified, all of the percent amino acid sequence identity values herein are obtained using the BLAST computer program, as indicated by the preceding paragraph.

Amino acid mutations in the antigen-binding fragment of SEQ ID NO: 7 of the invention include a "substitution" which is replacement with another amino acid, a "deletion" wherein the amino acid is removed, or an "insertion" in which another amino acid is added. One type of "substitution" is a "conservative amino acid substitution". A conservative amino acid substitution is determined based on similarity with the properties of polarity, charge, solubility, hydrophobicity, hydrophilicity and/or amphiphilicity of the residue in question. For example, non-polar (hydrophobic) amino acids include alanine (Ala, A), leucine (Leu, L), isoleucine (Ile, I), valine (Val, V), proline (Pro, P), phenylalanine (Phe, F), tryptophan (Trp, W) and methionine (Met, M); polar neutral amino acids include glycine (Gly, G), serine (Ser, S), threonine (Thr, T), cysteine (Cys, C), tyrosine (Tyr, Y), asparagine (Asn, N) and glutamine (Gln, Q); positively charged (basic) amino acids include arginine (Arg, R), lysine (Lys, K) and histidine (His, H); and negatively charged (acidic) amino acids include aspartic acid (Asp, D) and glutamic acid (Glu, E). The terms "substitution", "insertion" and "deletion" all have a preferred range of 1 to 25, 1 to 20, 1 to 15, 1 to 10 or 1 to 5 amino acids. Mutation may be introduced by using a recombinant DNA method for systematic or random substitution of amino acids of a polypeptide molecule and carrying out assay of recombinant mutants obtained by activation.

The herpes simplex virus (HSV virus) to be used for the invention includes herpes simplex virus 1-type (HSV-1) (also sometimes referred to as "HHV-1") and herpes simplex virus 2-type (HSV-2) (also sometimes referred to as "HHV-2"). HSV-1 and HSV-2 used for an embodiment of the invention include all lines classified as such (for example, KOS, F, 17, VR3, HF, HF10 and SC16 which are classified as HSV-1, and 186, G and 333 which are classified as HSV-2), as well as all those derived from their sublines. That is, using HSV-1 as an example, it may be KOS of which the full-length genome sequence is disclosed as GenBank No. JQ673480.1 (https://www.ncbi.nlm.nih.gov/nuccore/JQ673480.1) (SEQ ID NO: 29), or HSV-1 belonging to another line classified as HSV-1, or its subline.

HSV has envelope glycoproteins (such as gB, gC, gD, gH, gL and gK) which cause membrane fusion between the host cell membrane and the viral envelope and are important for intracellular entry and intercellular propagation of the virus. First, HSV is adsorbed onto the cell membrane by binding of gB and gC with heparan sulfate expressed on the cell membrane surface. Next, gD binds to herpesvirus entry mediator (HVEM), nectin-1 or 3-O-sulfated heparan sulfate (3-OS-HS) (Montgomery et al., Cell 1996; Geraghty et al., Science 1998; Shukla et al., Cell 1999), whereby gD undergoes a structural change (Carfi et al., Mol Cell 2001; Fusco et al., Proc Natl Acad Sci USA 2005; Krummenacher et al., EMBO J 2005), and signal transduction occur to gH/gL complex (Atanasiu et al., J Virol 2010; Atanasiu et al., Mbio 2013; Gianni et al., J Biol Chem 2009). The gH/gL complex activates gB and establishes membrane fusion (Atanasiu et al., Proc Natl Acad Sci USA 2007; Atanasiu et al., J Virol 2010; Chowdary et al., Nat Struct Mol Biol 2010).

According to one embodiment, the IL13RA2-targeting herpes simplex virus of the invention has anti-IL13RA2 antibody or its antigen-binding fragment incorporated in the envelope protein and targets IL13RA2 expressed on cell surfaces, preferably the anti-IL13RA2 antibody or its antigen-binding fragment being incorporated into the envelope protein gD (SEQ ID NO: 12, for example), the envelope protein gB (SEQ ID NO: 13, for example; see PLoS Pathog 2017; 13(4): e1006352), the envelope protein gC (see Cancer Gene Ther 2010; 17(9): 655-663, for example) or the envelope protein gH (see PLoS Pathog 2015; 11(5): e1004907, for example), and more preferably being incorporated into the envelope protein gD. The amino acid sequences of the aforementioned envelope proteins are examples and are not intended to be limitative.

According to one embodiment, the IL13RA2-targeting herpes simplex virus of the invention may have the anti-IL13RA2 antibody or its antigen-binding fragment incorporated into the deleted portion of the amino acids from position 2 to position 24 of SEQ ID NO: 12 of envelope protein gD, or between position 24 and position 25, into the deleted portion of the amino acids from position 6 to position 38, or into the deleted portion of the amino acids from position 61 to position 218, of SEQ ID NO: 12.

According to one embodiment, the IL13RA2-targeting herpes simplex virus of the invention may have the envelope protein gD with an amino acid mutation that weakens infectivity into cells that express Nectin-1, HVEM and/or 3-OS-HS, or with the mutation described in International Patent Publication No. WO2020/090871, for example.

According to one embodiment of the invention, a "gD mutation" is a nucleotide or amino acid mutation in the gD gene or gD protein, gD being an envelope glycoprotein of the HSV virus that is responsible for infiltration into cells, that includes a mutation that causes loss (or reduction) of binding capacity with HVEM, a mutation that causes loss (or reduction) of binding capacity with nectin-1, a mutation that causes loss (or reduction) of binding capacity with 3-OS-HS or a mutation that imparts binding capacity with a tumor antigen (for example, an insertion mutation of DNA coding for scFv against a tumor antigen). Such a mutation weakens infectivity into cells expressing Nectin-1, HVEM and/or 3-OS-HS, compared to HSV virus lacking the mutation.

The amino acid sequence of gD protein of strain KOS is listed as SEQ ID NO: 12 (without the signal peptide consisting of 25 amino acid residues from the N-terminus).

Specific examples of a "gD mutation" will be described below for strain KOS, but the invention also applies as appropriate for other strains as well. The KOS gD protein is encoded by nucleotide position Nos. 138279..139463 (forward (rightward) direction) in the KOS genomic sequence registered as GenBank No. JQ673480.1.

Many gD mutations causing loss of binding capacity between gD and HVEM, nectin-1 or 3-OS-HS have been disclosed in published literature, such as Yoon et al., J Virol. 77:9221-9231 2003, Spear et al., Virology 344:17-24 2006, Connolly et al., J Virol. 79:1282-1295 2005, Uchida et al., J Virol. 83:2951-2961 2009, Uchida et al., J Virol. 84:12200-12209 2010 and Shibata et al., Gene Ther. 23:479-488 2016, and a person skilled in the art can select proper mutations as appropriate.

Examples include, without being particularly limited to, mutations that cause loss of binding capacity with Nectin-1, and specifically regarding positions of amino acid mutations that weaken infectivity into cells that express Nectin-1 as amino acid positions of gD protein, and in terms of the amino acid nos. in SEQ ID NO: 12: Δ6-38 (deletion of amino acids at amino acid positions 6 to 38) (Menotti et al., J Virol. 82:10153-10161 2008), Δ61-218 (deletion of amino acids at amino acid positions 61 to 218) (Menotti et al., Proc Natl Acad Sci USA. 106:9039-9044 2009), mutations of R222 and F223 (mutation of amino acids at amino acid position 222 and position 223) such as R222 N/F223I (Uchida et al., J Virol. 83:2951-2961 2009), mutations of A3 and/or Y38 (mutation of amino acids at amino acid position 3 and/or position 38) such as Y38C, A3C or ΔA3/Y38C (Connolly et al., J Virol. 79:1282-1295 2005, Uchida et al., J Virol. 83:2951-2961 2009), or a mutation where the amino acid at position 3 is deleted or substituted to cysteine or the amino acid at position 38 is substituted to cysteine.

Additional examples include mutations that cause loss of binding capacity with HVEM, and specifically regarding positions of amino acid mutations that weaken infectivity into cells that express HVEM as amino acid positions of gD protein, and in terms of the amino acid nos. in SEQ ID NO: 12: deletion of all or some of the amino acids at amino acid positions 2 to 38, such as Δ7-32 (deletion of amino acids at amino acid positions 7 to 32) (Yoon et al., J Virol. 77:9221-9231 2003), Δ2-24 (deletion of amino acids at amino acid positions 2 to 24) (Shibata et al., Gene Ther. 23:479-488 2016), Δ7-11 (deletion of amino acids at amino acid positions 7 to 11) (Uchida et al., J Virol. 84:12200-12209 2010) and Δ6-38 (Menotti et al., J Virol. 82:10153-10161 2008), or Δ61-218 (deletion of amino acids at amino acid positions 61 to 218) (Menotti et al., Proc Natl Acad Sci USA. 106:9039-9044 2009), and mutations of Q27, T29 and D30 (amino acid mutation at amino acid position 27, amino acid mutation at position 29 and amino acid mutation at position 30), such as Q27A, Q27P, Q27R, T29A and D30A (Spear et al., Virology 344:17-24 2006).

Most mutations that inhibit binding with HVEM also inhibit infiltration into cells via 3-OS-HS (Yoon and Spear, Proc Natl Acad Sci USA. 101:17252-17257 2004).

Sequences exhibiting at least 70%, at least 80%, at least 85%, at least 90%, at least 95%, at least 98%, at least 99% or 100% sequence identity with the amino acid sequence of the KOS gD protein listed as SEQ ID NO: 12, and such sequences with the aforementioned specified mutations, are also within the scope of the invention.

According to one embodiment, in the herpes simplex virus of the invention, the envelope protein gB, envelope protein gK, UL20 protein and/or UL24 protein of the herpes simplex virus have an amino acid mutation that promotes infiltration into target cells and/or an amino acid mutation that promotes multinucleated giant cell formation.

The amino acid mutation that promotes multinucleated giant cell formation is a mutation to be introduced into the gB gene (or gB protein), gK gene (or gK protein), UL20 gene (or UL20 protein: envelope protein UL20) and/or UL24 gene (or UL24 protein: nuclear protein UL24) of HSV, as a mutation promoting membrane fusion activity by HSV. The amino acid mutation that promotes infiltration into target cells is also a mutation to be introduced into the gB gene (or gB protein) of HSV, as a mutation promoting efficiency of infiltration into target cells by HSV. The amino acid sequence of KOS gB protein, the amino acid sequence of KOS gK protein, the amino acid sequence of KOS UL20 protein and the amino acid sequence of KOS UL24 protein are shown as SEQ ID NO: 13, SEQ ID NO: 14, SEQ ID NO: 15 and SEQ ID NO: 16, respectively.

Specific examples of amino acid mutations that promote infiltration into target cells and/or amino acid mutations that promote multinucleated giant cell formation will be described using the KOS strains specified below, but the invention is likewise applicable for other strains. The KOS gB protein is encoded by nucleotide position Nos. 53022..55736 (reverse (leftward) direction), the gK protein is encoded by nucleotide position Nos. 112101..113117 (forward (rightward) direction), UL20 protein is encoded by nucleotide position Nos. 40763..41431 (reverse (leftward) direction) and UL24 protein is encoded by nucleotide position Nos. 47678..48487 (forward (rightward) direction), in the genomic sequence registered as GenBank No. JQ673480.1. Amino acid mutations that promote infiltration into target cells and/or amino acid mutations that promote multinucleated giant cell formation are disclosed in numerous publications, and a person skilled in the art can select proper mutations as appropriate.

Major amino acid mutations of gB protein that promote infiltration into target cells, referenced by the amino acid number in SEQ ID NO: 13 as the amino acid positions of gB protein, are D285 mutations such as D285N, and A549 mutations such as A549T. Major amino acid mutations of gB protein that promote multinucleated giant cell formation, referenced by the amino acid number in SEQ ID NO: 13, as the amino acid positions of gB protein, include R796 mutations such as R796C, R800 mutations such as R800W, T813 mutations such as T8131, L817 mutations such as L817H and L817P, S854 mutations such as S854F, A855 mutations such as A855V, R858 mutations such as R858C and R858H, insertions between E816 and L817 (VN (2-amino acid insertion) and VNVN (4-amino acid insertion)), S869 nonsense mutation, and T877 nonsense mutation.

Major amino acid mutations of gK protein that promote multinucleated giant cell formation, referenced by the amino acid number in SEQ ID NO: 14, as the amino acid positions of gK protein, include P33 mutations such as P33S, A40 mutations such as A40V and A40T, L86 mutations such as L86P, D99 mutations such as D99N, A111 mutations such as A111V, T121 mutations such as T121I, C243 mutations such as C243Y, L304 mutations such as L304P, and R310 mutations such as R310L.

Major amino acid mutations of UL20 protein that promote multinucleated giant cell formation, referenced by the amino acid number in SEQ ID NO: 15, as the amino acid positions of UL20 protein, include Y49, S50 and R51 mutations such as singular Y49A mutation and Y49A/S50A/R51A mutation, R209, T212 and R213 mutations such as singular R209A mutation and R209A/T212A/R213A mutation, C-terminal deletions after N217, and deletion of the full-length UL20 protein.

Major amino acid mutations of UL24 protein that promote multinucleated giant cell formation, referenced by the amino acid number in SEQ ID NO: 16 as the amino acid position of UL24 protein, include T62, R63 and V64 mutations such as T62G/R63V/V64S.

The major amino acid mutations that promote infiltration into target cells and/or amino acid mutations that promote multinucleated giant cell formation are summarized in Table 1.

[Table 1]

| Gene | Mutation | Mutation-identifying report |
|---|---|---|
| gK | P33S | Dolter et al., J Virol. 68:8277-81 1994 |
| | A40V | Debroy et al., Virology 145:36-48 1985<br>Dolter et al., J Virol. 68:8277-81 1994<br>Israyelyan et al., Hum Gene Ther. 18:457-73 2007 |
| | A40T | Debroy et al., Virology 145:36-48 1985 |
| | L86P | Dolter et al., J Virol. 68:8277-81 1994 |
| | D99N | Dolter et al., J Virol. 68:8277-81 1994 |
| | A111V | Dolter et al., J Virol. 68:8277-81 1994 |
| | T121I | Dolter et al., J Virol. 68:8277-81 1994 |

(continued)

| Gene | Mutation | Mutation-identifying report |
|---|---|---|
| | C234Y | Terry-Allison et al., J Virol. 72:5802-10 1998 |
| | L304P | Dolter et al., J Virol. 68:8277-81 1994 |
| | R310L | Dolter et al., J Virol. 68:8277-81 1994 |
| gB | D285N | Uchida et al., J Virol 84:12200-9 2010 |
| | A549T | Uchida et al., J Virol 84:12200-9 2010 |
| | R796C | Gage et al., J Virol. 67:2191-2201 1993 |
| | R800W | Gage et al., J Virol. 67:2191-2201 1993 |
| | T8131 | Gage et al., J Virol. 67:2191-2201 1993 |
| | Insertion of 2 (VN) or 4 (VNVN) amino acids between E816 and L817 | Cai et al., J Virol. 62:2596-2604 1988 |
| | L817H | Engel et al., Virology 192:112-120 1993 |
| | L817P | Diakidi-Kosta et al., Virus Res. 93: 99-108 203 |
| | S854F | Walev et al., Virus Genes 8:83-86 1994 |
| | A855V | Engel et al., Virology 192:112-120 1993 |
| | R858C | Gage et al., J Virol. 67:2191-2201 1993 |
| | R858H | Bzik et al., Virology 137:185-190 1984 |
| | S869STOP (C-terminal deletion) | Foster et al., Virology 287:18-29 2001 |
| | T877STOP (C-terminal deletion) | Baghian et al., J Virol. 67:2396-2401 1993 |
| UL20 | Y49A/S50A/R51A | Melancon et al., J Virol. 78: 7328-43 2004 |
| | Y49A | Melancon et al., J Virol. 78: 7328-43 2004 |
| | N217STOP (C-terminal deletion) | Melancon et al., J Virol. 78: 7328-43 2004 |
| | R209A/T212A/R213A | Melancon et al., J Virol. 78: 7328-43 2004 |
| | R209A | Melancon et al., J Virol. 78: 7328-43 2004 |
| | Deletion of UL20 itself | Baines et al., J Virol. 65:6414-24 1991 |
| UL24 | T62G/R63V/V64S | Jacobson et al., Virology 242:161-69 1998 |

Sequences exhibiting at least 70%, at least 80%, at least 85%, at least 90%, at least 95%, at least 98%, at least 99% or 100% sequence identity with the amino acid sequence of the KOS gK protein listed as SEQ ID NO: 14, the KOS gB protein listed as SEQ ID NO: 13, the KOS UL20 protein listed as SEQ ID NO: 15 and the KOS UL24 protein listed as SEQ ID NO: 16, and such sequences with the aforementioned specified mutations, are also within the scope of the invention.

In addition to retaining the aforementioned syn mutation, the herpes simplex virus of the invention may also retain an exogenous gene that promotes HSV membrane fusion (here also referred to as "membrane fusion-promoting exogenous gene"), in an expressible manner. The position of the exogenous gene incorporated into the HSV genome is not particularly restricted, and may be any position in the HSV genome that does not inhibit functioning of the HSV virus as an oncolytic virus.

The membrane fusion-promoting exogenous gene may be, but is not limited to, gibbon ape leukemia virus (GALV)-derived fusogenic membrane glycoprotein (FMG). Preparation of HSV that retains the GALV FMG gene in an expressible manner in the genome can be easily carried out by a person skilled in the art with reference to Simpson et al., Cancer Res. 66:4835-4842 2006 and Nakamori et al., Clin Cancer Res. 9:2727-2733 2003, for example.

The herpes simplex virus of the invention may also have a mutation that attenuates proliferation of normal cells (also referred to herein as "restricted growth modification"), either instead of or in addition to the mutations described above. Restricted growth modification may be a mutation that inactivates expression of functional ICP34.5, for example. ICP34.5 (the amino acid sequence listed as SEQ ID NO: 30 (strain KOS), for example) is a neurotoxic factor possessed by herpes simplex virus. In cancer cells, the signal transduction system involved in activation of protein kinase R (PKR) is abnormal, resulting in increased tolerance to viruses compared to normal cells, or sometimes in greater resistance to

external interferon. On the other hand, ICP34.5 protein which is a virus gene product that functions to neutralize PKR activity is conserved in herpes simplex virus. It is known that herpes simplex virus having a mutation that has inactivated expression of functional ICP34.5 has severely inhibited growth in normal cells, but proliferates well in an almost unaffected manner in cancer cells. Therefore, herpes simplex virus having a mutation that has inactivated expression of functional ICP34.5 may be expected to exhibit higher safety since it proliferates only in cancer cells while having inhibited proliferation in normal cells. According to one embodiment, the herpes simplex virus of the invention may have a mutation that inactivates expression of functional ICP34.5, and for example, it may have an arbitrary mutation (such as a nonsense mutation) or deletion or substitution that renders ICP34.5 non-functional, or a deletion of all or part of the gene coding for ICP34.5 which inactivates expression of functional ICP34.5, or the ICP34.5 mutations mentioned in International Patent Publication No. WO2020/090871, Japanese Patent Public Inspection No. 2006-528484, Chou et al. 1990, Science 250: 1262-1266; and Maclean et al. 1991, J. Gen. Virol. 72: 631-639. Without being limitative, it may be one with inactivated expression of a functional ICP34.5 protein having at least 70%, at least 80%, at least 85%, at least 90%, at least 95%, at least 98%, at least 99% or 100% sequence identity with the amino acid sequence of the ICP34.5 protein of strain KOS listed as SEQ ID NO: 30, or one with an arbitrary mutation (such as a nonsense mutation), a deletion or a substitution in such an amino acid sequence that renders it non-functional, or with a deletion of all or part of the gene coding for the same, whereby expression of functional ICP34.5 is inactivated. The term "functional ICP34.5" used herein means ICP34.5 protein that can exhibit the function of the wild type ICP34.5 (for example, the function of neutralizing PKR activity), while "expression of functional ICP34.5 is inactivated" means that the function of neutralizing PKR activity is not exhibited, or that the function is attenuated compared to the wild type ICP34.5. In addition to ICP34.5, the restricted growth modification to be used for the invention may be restricted growth modification introduced by any of various methods such as the one described in a paper by Peters et al. (Mol Ther Oncolytics. 2015; 2. pii: 15010. Epub 2015 Jul 22), as well as other papers citing that paper, and for example, it may be a modification (mutation, deletion and/or substitution) of one or more genes selected from among ICP6/UL39, Thymidine kinase/UL23, UL2/UNG, ICP47/Us12 and Us11, Us3, ICP0, UL56, VP16/UL48, and UL24.

The HSV of the invention may also have, incorporated into the genome in an expressible manner, a reporter gene and an optional therapeutic gene (for example, a gene that increases the killing effect on cancer cells, a gene that suppresses tumor vascularization or a gene that promotes antitumor immunoreaction) (see Peters et al., Mol Ther Oncolytics. 2015; 2. pii: 15010. Epub 2015 Jul 22 for details). The position of the gene incorporated into the HSV genome is not particularly restricted, and may be any position in the HSV genome that does not inhibit functioning of the HSV virus as an oncolytic virus.

The reporter gene is not particularly restricted and may be the LacZ gene (Mineta et al., Nat Med. 1:938-943 1995), Luc gene (Yamamoto et al., Gene Ther. 13:1731-1736 2006), GFP gene (Adusumilli et al., FASEB J. 20:726-728 2006) or NIS gene (Li et al., Cancer Gene Ther. 20:478-485 2013).

The therapeutic gene is also not particularly restricted and may be the CD gene (Nakamura et al., Cancer Res. 61:5447-5452 2001) or TRAIL gene (Tamura et al., Mol Ther. 21:68-77 2013), as genes coding for cytotoxicity molecules, the GM-CSF gene (Liu et al., Gene Ther. 10:292-303 2003) or IL-12 gene (Roth et al., Ther Clin Dev. 25:16-27 2014), as genes coding for immunoactivating molecules, or the Angiostatin gene (Zhang et al., Mol Ther. 20:37-45 2012), PF4 gene (Liu et al., Mol Ther. 14:789-797 2006) and Chondroitinase-ABC gene (Dmitrieva et al., Clin Cancer Res. 17:1362-1372 2011), as genes coding for microenvironment-regulating molecules.

The present invention also encompasses nucleic acid (a nucleic acid molecule) encoding the herpes simplex virus described herein.

The herpes simplex virus of the invention can be produced by a standard method known to those skilled in the field of HSV virology. The invention also provides a vector having the genomic nucleic acid of the recombinant virus of the invention, in order to facilitate manipulation of the HSV genome and production of the vector of the invention. A preferred vector is a bacterial artificial chromosome ("BAC") having the genome of the recombinant virus of the invention which facilitates manipulation of HSV in bacterial systems. An example of an HSV-BAC construct to be used is KOS-37 BAC (Even et al., 2006 Virus Res.119(2):195), allowing mutations to be introduced by homologous recombination (for example, Red homologous recombination: Tischer et al., 2006. Biotechniques. 40(2):191).

HSV vectors and methods for constructing them are described in, for example, US Patent No. 7,078,029, No. 6,261,552, No. 5,998,174, No. 5,879,934, No. 5,849,572, No. 5,849,571, No. 5,837,532, No. 5,804,413 and No. 5,658,724, as well as International Patent Applications WO91/02788, WO96/04394, WO98/15637 and WO99/06583, which are incorporated herein in their entirety by reference. The sequence of HSV is publicly known (NCBI Accession No. NC_001806; see also McGoechetal., J. Gen. Virol, 69 (PT7), 1531(1988)), and allows the recombinant virus vector of the invention to be easily designed. In some cases, the oncolytic recombinant virus of the invention will be herpes simplex virus (HSV-1), including the gD gene having Δ2-24 and Y38C mutations, the gB gene having a D285N/A549T mutation (NT mutation) and R858H mutation, and the gK gene having an A40T mutation, and further having a nucleic acid sequence encoding anti-IL13RA2 antibody or its antigen-binding fragment fused with the gD gene (for example, the Δ2-24 portion).

The genome of the herpes simplex virus of the invention may further include a non-virus gene (exogenous expression cassette) for a reporter protein such as green fluorescent protein (GFP) or luciferase or a payload molecule.

The reporter protein can facilitate verification of target cells infected with the recombinant virus of the invention, with lysing of tumor cells being monitored using the reporter protein released from lysed tumor cells as the indicator.

The present invention also encompasses a method for producing the herpes simplex virus described herein. After infection of producing cells with the herpes simplex virus of the invention, the herpes simplex virus may be recovered from the culture supernatant or producing cells. The producing cells may be, but are not limited to, RPMI7951, U87, CHO, 293, COS, BHK, HeLa or Vero cells, or a subline of these cell lines expressing IL13RA2 (for example, Vero-IL13RA2 cells), or a subline of these cell lines expressing gD (for example, VD60 cells). Herpes simplex virus can be purified by a method such as centrifugal separation.

The present invention also encompasses cells that produce the herpes simplex virus described herein. The cells that produce the herpes simplex virus may be cells that produce the herpes simplex virus temporarily, having nucleic acid (a nucleic acid molecule) coding for the herpes simplex virus described herein transferred into the cells, or they may be cells that produce the herpes simplex virus described herein in a continuous manner, having nucleic acid (a nucleic acid molecule) coding for the herpes simplex virus described herein incorporated into the genome of the cells. The cells that produce the herpes simplex virus described herein may be, but are not limited to, RPMI7951, U87, CHO, 293, COS, BHK, HeLa or Vero cells, or a subline of these cell lines expressing IL13RA2 (for example, Vero-IL13RA2 cells), or a subline of these cell lines expressing gD (for example, VD60 cells).

A specific mode of the invention relates to a stock and composition comprising the herpes simplex virus described herein. According to several aspects, the invention relates to a virus stock comprising a recombinant virus as described herein. According to several embodiments, the virus stock is stock of the same species. Preparation and analysis of the virus stocks are well known in the technical field. For example, a virus stock can be produced with a roller bottle containing cells transfected with a viral vector. The virus stock may then be purified in a continuous Nycodenz gradient, separated into aliquots, and stored until needed. Virus stocks have significant differences in titer which depend on the viral genotype and the protocols or cell lines used for their preparation.

According to one specific embodiment, the titer of a virus stock designed according to the present invention (for example, virus stock of herpes simplex virus) is preferably at least ~$10^5$ plaque forming units (pfu)/mL, more preferably at least ~$10^6$ pfu/mL, and even more preferably at least ~$10^7$ pfu/mL. According to a specific embodiment, the titer may be at least ~$10^8$ pfu/mL or at least ~$10^9$ pfu/mL, with the stock most preferably having a high titer of at least ~$10^{10}$ pfu/mL or at least ~$10^{11}$ pfu/mL.

Pharmaceutical composition

The present invention also provides a pharmaceutical composition comprising the herpes simplex virus described herein and a pharmaceutically acceptable carrier. The expression "pharmaceutically acceptable" refers to a molecular entity or composition that does not cause allergy or a similar adverse reaction when administered to a subject (such as a human). The term "pharmaceutical composition" used herein refers to a formulation of one or more herpes simplex viruses described herein, that can be administered or delivered to a subject and/or cells. The formulation will generally include the herpes simplex virus of the invention as the active ingredient, and also optionally a physiologically acceptable carrier, diluent and/or excipient. The term "pharmaceutical composition" is a composition of one or more drugs that can be administered or delivered to a patient and/or a subject and/or cells for treatment of a specific disease or disorder. The pharmaceutical composition may also include a nucleic acid molecule encoding the herpes simplex virus described herein, instead of the herpes simplex virus of the invention.

The composition disclosed herein may be formulated in neutral or salt form. The expression "pharmaceutically acceptable salt" includes both acid addition salts and base addition salts. Pharmaceutically acceptable salts include acid addition salts (formed by free amino groups of proteins), which may be formed by inorganic acids such as hydrochloric acid, hydrobromic acid, sulfuric acid, nitric acid or phosphoric acid, and organic acids including, but not limited to, acetic acid, 2,2-dichloroacetic acid, adipic acid, alginic acid, ascorbic acid, aspartic acid, benzenesulfonic acid, benzoic acid, 4-acetamidebenzoic acid, camphoric acid, camphor-10-sulfonic acid, capric acid, caproic acid, caprylic acid, carbonic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactaric acid, gentisic acid, glucoheptonic acid, gluconic acid, glucuronic acid, glutamic acid, glutaric acid, 2-oxoglutaric acid, glycerophosphoric acid, glycolic acid, hippuric acid, isobutyric acid, lactic acid, lactobionic acid, lauric acid, maleic acid, malic acid, malonic acid, mandelic acid, methanesulfonic acid, mucic acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, pamoic acid, propionic acid, pyroglutamic acid, pyruvic acid, salicylic acid, 4-aminosalicylic acid, sebacic acid, stearic acid, succinic acid, tartaric acid, thiocyanic acid, p-toluenesulfonic acid, trifluoroacetic acid and undecylenic acid. Salts formed with free carboxyl groups can likewise be derived from inorganic bases, and may be sodium, potassium, lithium, ammonium, calcium, magnesium, iron, zinc, copper, manganese or aluminum salts, for example. Salts derived from organic bases include, but are not limited to, primary, secondary and tertiary amines, substituted amines including naturally substituted amines, cyclic amines and basic ion exchange

resins, with examples including salts of ammonia, isopropylamine, trimethylamine, diethylamine, triethylamine, tripropylamine, diethanolamine, ethanolamine, deanol, 2-dimethylaminoethanol, 2-diethylaminoethanol, dicyclohexylamine, lysine, arginine, histidine, caffeine, procaine, hydrabamine, choline, betaine, benethamine, benzathine, ethylenediamine, glucosamine, methylglucamine, theobromine, triethanolamine, tromethamine, purine, piperazine, piperidine, N-ethylpiperidine and polyamine resins. Especially preferred organic bases include isopropylamine, diethylamine, ethanolamine, trimethylamine, dicyclohexylamine, choline and caffeine. After formulation, the solution is administered in a therapeutically effective dose by a method suited for the formulation to be administered. The formulation can be easily administered in various forms such as an injection or drug release capsules.

The term "carrier" used herein includes the concepts of solvent, dispersing medium, medium, coating, diluent, antibacterial or antifungal agent, isotonic or delayed absorption agent, buffering agent, carrier solution, suspension and colloid. The use of media and drugs as pharmaceutically active substances are well known in the technical field. Any conventional medium or drug can be used in a therapeutic composition unless it is unsuitable as an active component. Auxiliary active components may also be incorporated into the composition.

The expression "pharmaceutically acceptable carrier" as used herein encompasses, but is not limited to, any physiologically suitable adjuvant, carrier, excipient, glidant, sweetener, diluent, preservative, dye/coloring agent, flavor enhancer, surfactant, moistening agent, dispersing agent, suspending agent, stabilizer, isotonic agent, solvent, surfactant, dispersing medium, coating, antibacterial or antifungal agent, or isotonic or delayed absorption agent which includes a pharmaceutically acceptable cell culture medium and/or emulsifying agent approved by the U.S. Food and Drug Administration (FDA) as acceptable for use in humans and/or livestock. Typical pharmaceutically acceptable carriers include, but are not limited to, sugars such as lactose, glucose and sucrose, starches such as corn starch and potato starch, cellulose and its derivatives such as carboxymethylcellulose sodium, ethyl cellulose and cellulose acetate, tragacanth, malt, gelatin, talc, cocoa butter, waxes, animal and vegetable fats, paraffin, silicone, bentonite, silicic acid, zinc oxide, oils such as peanut oil, cottonseed oil, safflower oil, sesame oil, olive oil, corn oil and soybean oil, glycols such as propylene glycol, polyols such as glycerol, sorbitol, mannitol and polyethylene glycol and esters such as ethyl oleate and ethyl laurate, agar, buffering agents such as magnesium hydroxide and aluminum hydroxide, alginic acid, pyrogenic-free water, isotonic saline, Ringer's solution, ethyl alcohol, phosphate buffer, and any other suitable substances used in medical formulations. Any conventional medium and/or drug can be used in a therapeutic composition unless it is unsuitable as a drug according to the present disclosure. Auxiliary active components may also be incorporated into the composition.

Moistening agents, emulsifying agents and lubricants such as sodium lauryl sulfate and magnesium stearate, as well as coloring agents, release agents, coating agents, sweeteners, flavoring agents, aromatics, preservatives and antioxidants, may also be included in the composition.

Examples of pharmaceutically acceptable antioxidants include (1) water-soluble antioxidants such as ascorbic acid, cysteine hydrochloride, sodium bisulfate, sodium metabisulfite and sodium sulfite, (2) oil-soluble antioxidants such as ascorbyl palmitate, butylated hydroxyanisole (BHA), butylated hydroxytoluene (BHT), lecithin, propyl gallate and $\alpha$-tocopherol, and (3) metal chelating agents such as citric acid, ethylenediaminetetraacetic acid (EDTA), sorbitol, tartaric acid and phosphoric acid.

According to one embodiment, the pharmaceutical composition containing a carrier is suitable for parenteral administration such as intravascular (intravenous or intraarterial), intrathoracic, intraperitoneal, intracerebral or into another organ, intratumoral, subcutaneous or intramuscular administration. Pharmaceutically acceptable carriers include sterilized water solutions or dispersions for immediate preparation of solutions for sterile injection or dispersions, and sterile powders. The use of media and drugs for pharmaceutically active substances are well known in the technical field. Any conventional medium or drug may be used for the pharmaceutical composition of the invention, excluding those that are unsuitable for recombinant viruses or nucleic acid molecules.

The composition of the invention may contain one or more polypeptides, polynucleotides, vectors containing them, or cells infected with the recombinant virus of the invention, as described herein, either alone or formulated as a pharmaceutically acceptable or physiologically acceptable solution for administration to cells or a subject (such as a human) in combination with one or more other treatment methods. It will be appreciated that the composition of the invention may be administered as necessary in combination with antibodies, cytokines, growth factors, hormones, small molecules or various pharmaceutically active agents, in the same manner as other drugs. Such additional drugs may likewise be included in the composition, without any restrictions so long as they do not adversely affect, in any substantial manner, the potency of the composition being delivered for the intended treatment.

Pharmaceutically acceptable excipients and carrier solution formulations for the pharmaceutical composition of the invention are well known to those skilled in the art, as are appropriate methods of administration for use of the specific compositions described herein in various treatment plans, and the development of treatment plans. After formulation, the solution is administered in an amount that provides a therapeutic effect for amelioration or curing of symptoms, by a method suited for the administered formulation. The formulation is easily administerable in various dosage forms such as an ingestible solution or drug release capsules. Changes may be made to the manner of administration depending on the

condition of the subject during treatment. The person in charge of administration may determine the appropriate dosage for each individual subject. For administration to a human, the formulation should satisfy general standards of safety and purity, including sterileness as required by criteria established by the Center for Biologics Evaluation and Research of the U.S. Food and Drug Administration (FDA).

Under certain conditions, the pharmaceutical composition, recombinant virus and nucleic acid molecule disclosed herein are preferably delivered by a parenteral (such as intravascular (intravenous or intraarterial), intrathoracic, intraperitoneal, intracerebral, other organ, intratumoral, subcutaneous or intramuscular) route, as described in US Patent No. 5,543,158, US Patent No. 5,641,515 and US Patent No. 5,399,363 (all of which are incorporated herein in their entirety by reference). The solution of an active compound as a free base or pharmaceutically acceptable salt may be prepared using water in suitable admixture with a surfactant such as hydroxypropyl cellulose. A dispersion may likewise be prepared in glycerol or liquid polyethylene glycol, or a mixture thereof, or in an oil. Under normal storage and usage conditions, such preparations will usually contain preservatives to prevent growth of microorganisms.

Drug forms suited for injection include solutions for sterile injection, sterilized water solutions for immediate preparation of dispersions, or dispersions and sterile powders (US Patent No. 5,466,468, the entirety of which is explicitly incorporated herein by reference). In all cases, the dosage form should be aseptic, and should be fluid enough to allow easy passage through an injection needle. It should also be stable under production and storage conditions, and protected from contaminants including microorganisms such as bacteria and fungi. The carrier may be water, ethanol a polyol (such as glycerol, propylene glycol or liquid polyethylene glycol), appropriate mixtures of the same, and/or a solvent or dispersing medium containing a vegetable oil. Suitable fluidity may be maintained by, in the case of a coating such as a dispersion using lecithin, for example, maintaining the necessary particle sizes, or maintenance using a surfactant. Suppression of microorganic activity can be promoted by antibacterial or antifungal agents such as paraben, chlorobutanol, phenol, sorbic acid or thimerosal. In most cases it is preferred to include an isotonic agent such as sugar or sodium chloride. Long-lasting absorption of an injectable composition can be achieved by use of the composition with a medicine that delays absorption, such as aluminum monostearate or gelatin. Preparation of aqueous compositions containing proteins as active components is well understood in the technical field. Such a composition will typically be prepared as an injection either in liquid solution or suspension form, which may also be in a solid form suitable for preparation as a liquid solution or suspension prior to injection. The formulation may also be emulsified.

For parenteral administration as an aqueous solution, the solution should be appropriately buffered as necessary, and the liquid diluent should first be brought to isotonicity with a sufficient amount of physiological saline or glucose. The specified aqueous solution is particularly suitable for parenteral administration (such as intravascular (intravenous or intraarterial), intrathoracic, intraperitoneal, intracerebral or into another organ, intratumoral, subcutaneous or intramuscular administration). In this regard, suitable sterile aqueous media will be apparent to a person skilled in the art in light of the present disclosure. For example, a single dose may be dissolved in 1 ml of isotonic NaCl solution, adding this to 1000 ml of subcutaneous injection solution, or injecting it into the intended injection site (see Remington: The Science and Practice of Pharmacy, 20th Edition. Baltimore, MD: Lippincott Williams & Wilkins, 2000, for example). It will be necessary to make several changes to the dose depending on the condition of the subject during treatment. In each case, the person in charge of administration will determine the appropriate dosage for each individual subject. For administration to a human, the formulation should satisfy general standards of sterileness and pyrogenicity, as well as safety and purity, as required by criteria established by the Office of Biological Therapeutic Products of the U.S. Food and Drug Administration (FDA).

The solution for sterile injection may be prepared by adding a necessary amount of the active compound to a suitable solvent together with the various other components mentioned above as necessary, and then carrying out filtered sterilization. A dispersion is generally prepared by incorporating various sterilized active components into a sterilized medium containing a basic dispersing medium and necessary other components selected from among those mentioned above. In the case of sterile powder for preparation of a solution for sterile injection, the preferred method of preparation is vacuum drying or freeze-drying, whereby in addition to the active component powder, any other optional desired components can be obtained from the pre-sterilized filtered solution.

According to a specific embodiment, the composition can be delivered by intranasal spraying, inhalation and/or via another aerosol delivery medium. Methods for direct delivery of polynucleotide and peptide compositions via nasal aerosol spray are described, for example, in US Patent No. 5,756,353 and US Patent No. 5,804,212 (both of which are incorporated herein in their entirety by reference). Methods of delivering drugs using intranasal fine particulate resins (Takenaga et al., 1998) and lysophosphatidyl-glycerol compounds US Patent No. 5,725,871, which is incorporated herein in its entirety by reference) are also well known in the pharmaceutical field. Transmucosal drug delivery in the form of a polytetrafluoroethylene-supporting matrix is described in US Patent No. 5,780,045 (which is incorporated herein in its entirety by reference).

According to a specific embodiment, the delivery may be carried out using liposomes, nanocapsules, microparticles, microspheres, lipid particles or vesicles in admixture with CPP polypeptide, for introduction of the composition of the invention into the appropriate host cells. The composition of the invention can be formulated by encapsulating lipid

particles, liposomes, vesicles, nanospheres or nanoparticles, for delivery. Formulation and use of the delivery medium may be carried out according to known conventional techniques. The formulation and composition of the invention may be obtained by formulating one or more polypeptides, polynucleotides or small molecules mentioned herein, in a pharmaceutically acceptable or physiologically acceptable solution (such as medium) for administration to cells or a subject (such as a human), either alone or in combination with one or more other treatment methods. It will be understood that the composition of the invention may be administered as necessary in combination with cells, other proteins or polypeptides, or various pharmaceutically active agents, in the same manner as other drugs.

According to a specific embodiment, the formulation or pharmaceutical composition of the invention includes cells that have been contacted with a combination of the various polynucleotides referred to herein or the herpes simplex virus described herein.

Typical formulation methods for *ex-vivo* delivery to deliver viral vector-containing polynucleotides into cells also include the use of various transfection agents known in the technical field, such as calcium phosphate, electroporation, heat shock or liposome preparations (i.e. lipid-mediated transfection). Liposomes are lipid bilayers encapsulating an aqueous fluid fraction. The DNA spontaneously binds to the outer surfaces of cationic liposomes (based on electrical charge), with the liposomes interacting with the cell membrane.

A specific embodiment of the invention also includes other formulations such as those well known in the pharmaceutical field, described in Remington: The Science and Practice of Pharmacy, 20th Edition. Baltimore, MD: Lippincott Williams & Wilkins, 2000, for example.

According to a specific aspect, the invention provides a pharmaceutically acceptable composition comprising a therapeutically effective dose of one or more viral vectors or polynucleotides described herein, formulated together with one or more pharmaceutically acceptable carriers (additives) and/or diluents (such as a pharmaceutically acceptable cell culture medium). The expression "therapeutically effective dose" as used herein refers to an amount of the composition or recombinant virus described herein that is necessary to achieve a desired physiological and/or biological result. A "therapeutically effective dose" of a virus, virus stock or composition may be adjusted according to the condition, age, gender and body weight of the individual, as well as other factors such as the ability of stem cells and precursor cells to elicit the desired response in the individual. The therapeutically effective dose is also such that the beneficial therapeutic effect outweighs any toxicity or harmful effects of the virus or transformed therapeutic cells. The expression "therapeutically effective dose" also includes the concept of an effective dose that "cures" the subject (such as a patient). The therapeutically effective dose may be quantified as the total number of plaque forming units (pfu) (for example, at least $\sim 10^1$ to at least $\sim 10^{20}$, specifically $\sim 10^4$ to $\sim 10^{15}$, and more specifically $\sim 10^6$ to $\sim 10^{12}$ pfu), or number of viral genomes (for example, at least $\sim 10^1$ to at least $\sim 10^{20}$, and specifically $\sim 10^4$ to $\sim 10^{15}$, and more specifically $\sim 10^6$ to $\sim 10^{12}$ viral genomes). A person skilled in the art will appreciate that the therapeutically effective dose may be adjusted based on the type of virus to be administered, the properties of the formulation, the route of administration, the nature and/or severity of the disease to be treated, and/or the general condition and health of the subject.

Several aspects of the invention encompass a method of killing cancerous cells, wherein the cancerous cells are exposed to the herpes simplex virus or its composition as described herein under sufficient conditions for the herpes simplex virus to infect and replicate in the cancerous cells, with replication of the herpes simplex virus in the cancerous cells leading to cell death. According to several embodiments, the cancerous cells to be killed by the method are *in vivo* cells. According to a specific embodiment, the cancerous cells to be killed by the method are intratumoral cells.

The term "administration" as used herein refers to introduction of IL13RA2-targeting herpes simplex virus, virus stock or its composition, or a nucleic acid molecule encoding the recombinant virus or its composition, into a subject, and according to one aspect it refers to contacting the herpes simplex virus, virus stock or its composition, or the nucleic acid molecule encoding the virus or its composition, as described herein, with cells and/or tissue. The administration may be carried out by infusion, perfusion, inhalation, ingestion, electro-osmosis, hemodialysis, ion introduction, or another method known in the technical field. The route of administration will naturally differ depending on the location and nature of the disease to be treated, and for example it may be through the auricle, cheeks, conjunctiva, skin or teeth, or transcervical, intratunnel, endotracheal, enteral, epidural, interstitial, intra-articular, intra-arterial, intraperitoneal, intra-auricular, intrabiliary, endobronchial, intracystic, intracavitary, intracerebral, intrasulcular, intracorneal, intracranial, intracoronal, intracoronary, intracranial, intradermal, intradiscal, intraductal, intraduodenal, intradural, intrapericardial, intraepidermal, intraesophageal, intragastric, intragingival, intrahepatic, intraileal, intralesional, intralingual, intra-cavity, intralymphatic, intramammary, intramedullary, intrameningeal, intramuscular, intranasal, intranodular, intraocular, intrareticular, intraovarian, intrapericardial, intrapleural, intraprostatic, intrapulmonary, intraluminal, intraspinal, intrasynovial, intratendinous, intratesticular, intracapsular, intrathoracic, intratumoral, intratympanic, intrauterine, intravascular, intracerebroventricular, intravesical, intravestibular, intravenous, intravitreal, laryngeal, nasal, intranasal, gastric, oral, ocular, oropharyngeal, parenteral, percutaneous, periarticular, epidural, perineural, periodontal, respiratory, post-tubular, rectal, spinal, subarachnoid, subconjunctival, subcutaneous, subdermal, subgingival, sublingual, submucosal, subretinal, local, transdermal, transendocardial, transmucosal, transplacental, transtracheal, transtympanic, ureteral, urethral and/or by vaginal flow, washing, direct injection and oral administration.

The terms "treatment" or "therapy" as used herein refer to administering to the subject a therapeutically effective dose of the virus or its composition as described herein so as to ameliorate the disease or condition of the subject, or the symptoms of the disease or condition. Amelioration is any improvement or healing of the disease or condition, or the symptoms of the disease or condition. The amelioration may also be visible or measurable improvement, or it may be subjective improvement in overall health of the subject. A person skilled in the art will therefore appreciate that treatment will sometimes improve the symptoms, while not completely curing the disease. The term "preventative dose" refers to an amount of the virus, virus stock or composition that is effective for achieving a desired preventative result. The term "preventative" as used herein may refer to complete prevention of symptoms of the disease, delay of appearance of the disease symptoms, or alleviation of the severity of late-onset morbidity. While not always the case, since a preventative dose is used for a subject before the initial stages or during an initial stage of the disease, a preventative dose is generally less than a therapeutically effective dose.

The pharmaceutical composition comprising the IL13RA2-targeting herpes simplex virus of the invention can serve as a therapeutic agent for tumors expressing IL13RA2. Examples of tumors expressing IL13RA2 include melanoma, osteosarcoma, leukemia, lymphoma, prostate cancer, lung cancer (including malignant mesothelioma), gliomas (such as glioblastoma, astrocytoma and meduloblastoma), head and neck cancer, renal carcinoma, Kaposi's sarcoma, colorectal cancer, pancreatic carcinoma, adrenal cancer, breast cancer and ovarian cancer. Such cancer types may therefore be treatment targets for the invention.

<u>Antibody and its antigen-binding fragment</u>

One aspect of the anti-IL13RA2 antibody or its antigen-binding fragment according to the invention is an antibody that specifically recognizes tumor cells expressing IL13RA2, or its antigen-binding fragment.

One aspect of the anti-IL13RA2 antibody or its antigen-binding fragment according to the invention is one wherein the heavy chain CDRs (HCDR1, HCDR2, HCDR3) and light chain CDRs (LCDR1, LCDR2, LCDR3) have the amino acid sequences:

(i) HCDR1: (SEQ ID NO: 1: RHGIH)
(ii) HCDR2: (SEQ ID NO: 2: VIWAGGSTNYNSALMS)
(iii) HCDR3: (SEQ ID NO: 3: DHFDSFDY)
(iv) LCDR1: (SEQ ID NO: 4: TASSSVSSSYLH)
(v) LCDR2: (SEQ ID NO: 5: STSNLAS) and
(vi) LCDR3: (SEQ ID NO: 6: HQYHRSPLT),

or these amino acid sequences having a mutation of one or more amino acids.

Another aspect of the anti-IL13RA2 antibody or its antigen-binding fragment according to the invention is one wherein the heavy chain CDRs (HCDR1, HCDR2, HCDR3) have the respective amino acid sequences:

(i) HCDR1: (SEQ ID NO: 1: RHGIH)
(ii) HCDR2: (SEQ ID NO: 2: VIWAGGSTNYNSALMS)
(iii) HCDR3: (SEQ ID NO: 3: DHFDSFDY),

or these amino acid sequences with one or more (one, two or three) amino acid mutations.

Yet another aspect of the anti-IL13RA2 antibody or its antigen-binding fragment according to the invention is one wherein the light chain CDRs (LCDR1, LCDR2, LCDR3) have the respective amino acid sequences:

(i) LCDR1: (SEQ ID NO: 4: TASSSVSSSYLH)
(ii) LCDR2: (SEQ ID NO: 5: STSNLAS)
(iii) LCDR3: (SEQ ID NO: 6: HQYHRSPLT),

or these amino acid sequences with one or more (one, two or three) amino acid mutations.

Yet another aspect of the anti-IL13RA2 antibody or its antigen-binding fragment according to the invention may be an anti-IL13RA2 antibody or its antigen-binding fragment comprising a sequence having at least 80% (such as at least 85%, at least 90%, at least 95%, at least 98%, at least 99%, or 100%) sequence identity with the amino acid sequence of the $V_L$ region listed as SEQ ID NO: 8, and a sequence having at least 80% (such as at least 85%, at least 90%, at least 95%, at least 98%, at least 99% or 100%) sequence identity with the amino acid sequence of the $V_H$ region listed as SEQ ID NO: 10.

For this aspect, the anti-IL13RA2 antibody of the invention may include a mouse constant region (IgG1, IgG2a, IgG2b, IgG3, IgM, IgD, IgE or IgA). According to a partial aspect, the antibody may be a chimeric antibody, humanized

antibody or human antibody having a human constant region (IgG1, IgG2, IgG3, IgG4, IgM, IgD, IgE, IgA1, IgA2). According to yet another partial aspect, the antibody may be an antibody with double specificity, triple specificity or multi specificity (such as a bispecific antibody, trispecific antibody or multispecific antibody), in order to increase the affinity or avidity.

According to a partial aspect, the anti-IL13RA2 antibody or its antigen-binding fragment according to the invention is a humanized antibody. The term "humanized", as used in relation to an antibody, refers to an antibody having at least a CDR region derived from a non-human source, manipulated so as to have a structure and immunological function more similar to a true human antibody than the original source. For example, humanization may involve grafting a CDR derived from a non-human antibody such as a mouse antibody onto a human antibody. Humanization may also involve selecting an amino acid substitution that makes a non-human sequence more like a human sequence, as is done with known technology.

According to a partial aspect, the invention is an antigen-binding fragment of anti-IL13RA2 antibody. The antigen-binding fragment may be any antigen-binding fragment of an antibody described throughout the present specification. Antigen-binding fragments include Fab, Fab', F(ab')2, Fv, scFv, dsFv, diabodies, triabodies and bis-scFv, with no limitation to these so long as the antigen-binding fragment is derived from anti-IL13RA2 antibody. Diabodies (dimers), triabodies (trimers) and tetrabodies (tetramers) are known technologies. Exemplary references include Kortt et al., Biomol. Eng. 2001, 18:95-108 (2001) and Todorovska et al., J. Immunol Methods. 248:47-66 (2001).

The fragment scFv comprises an antibody light chain variable (V) domain linked to an antibody heavy chain V domain, via a synthetic peptide (linker), and can be formed using common recombinant DNA technology (see Janeway et al., Immunobiology, 2nd Edition, Garland Publishing, New York (1996), for example). Examples of linkers include flexible linkers that comprise $(Gly_4Ser)_3$. For scFv, the order of linkage of the $V_L$ region and $V_H$ region connected by the linker is not particularly restricted. A disulfide-stabilized variable region fragment (dsFv) may likewise be prepared by recombinant DNA technology (Reiter et al., Protein Engineering, 7, 697-704(1994), for example).

According to a partial aspect of the invention, the antigen-binding fragment of the anti-IL13RA2 antibody of the invention may be, for example, an antigen-binding fragment containing a sequence having at least 90%, at least 91%, at least 92%, at least 93%, at least 94%, at least 95%, at least 96%, at least 97%, at least 98%, at least 99%, or 100% sequence identity to the sequence listed as SEQ ID NO: 7. According to a partial aspect of the disclosure, the antigen-binding fragment of the anti-IL13RA2 antibody of the invention may be an antigen-binding fragment wherein the heavy chain CDRs (HCDR1, HCDR2, HCDR3) and light chain CDRs (LCDR1, LCDR2, LCDR3) have the amino acid sequences:

(i) HCDR1: (SEQ ID NO: 1: RHGIH)
(ii) HCDR2: (SEQ ID NO: 2: VIWAGGSTNYNSALMS)
(iii) HCDR3: (SEQ ID NO: 3: DHFDSFDY)
(iv) LCDR1: (SEQ ID NO: 4: TASSSVSSSYLH)
(v) LCDR2: (SEQ ID NO: 5: STSNLAS) and
(vi) LCDR3: (SEQ ID NO: 6: HQYHRSPLT),

or these amino acid sequences having a mutation of one or more amino acids,
and having at least 80%, at least 85%, at least 90%, at least 95%, at least 98%, at least 99%, or 100% sequence identity with the sequence listed as SEQ ID NO: 7.

In an antigen-binding fragment having the amino acid sequence of SEQ ID NO: 7, as one aspect of the invention, the amino acid mutations include a "substitution" which is replacement with another amino acid, a "deletion" wherein the amino acid is removed, or an "insertion" in which another amino acid is added. One aspect of "substitution" is a "conservative amino acid substitution". A conservative amino acid substitution is determined based on similarity with the properties of polarity, charge, solubility, hydrophobicity, hydrophilicity and/or amphiphilicity of the residue in question. For example, non-polar (hydrophobic) amino acids include alanine (Ala, A), leucine (Leu, L), isoleucine (Ile, I), valine (Val, V), proline (Pro, P), phenylalanine (Phe, F), tryptophan (Trp, W) and methionine (Met, M); polar neutral amino acids include glycine (Gly, G), serine (Ser, S), threonine (Thr, T), cysteine (Cys, C), tyrosine (Tyr, Y), asparagine (Asn, N) and glutamine (Gln, Q); positively charged (basic) amino acids include arginine (Arg, R), lysine (Lys, K) and histidine (His, H); and negatively charged (acidic) amino acids include aspartic acid (Asp, D) and glutamic acid (Glu, E). The terms "substitution", "insertion" and "deletion" may all have a preferred range of 1 to 25, 1 to 20, 1 to 15, 1 to 10 or 1 to 5 amino acids. Mutation may be introduced by using a recombinant DNA method for systematic or random substitution of amino acids of a polypeptide molecule and carrying out assay of recombinant mutants obtained by activation.

One aspect of the anti-IL13RA2 antibody or its antigen-binding fragment according to the invention is an anti-IL13RA2 antibody or its antigen-binding fragment that at least partially competes with an anti-IL13RA2 antibody or its antigen-binding fragment that includes the amino acid sequences of the heavy chain CDRs (HCDR1, HCDR2, HCDR3) and light chain CDRs (LCDR1, LCDR2, LCDR3) listed as SEQ ID NO: 1 to 6, for binding with human IL13RA2. That the antibody or its antigen-binding fragment "competes" means that when IL13RA2 protein is reacted with an antibody or

antigen-binding fragment of interest, for example, and then subsequently reacted with the anti-IL13RA2 antibody or antigen-binding fragment including the amino acid sequences of the heavy chain CDRs (HCDR1, HCDR2, HCDR3) and light chain CDRs (LCDR1, LCDR2, LCDR3) listed as SEQ ID NO: 1 to 6, the antibody or its antigen-binding fragment of interest decreases binding of the anti-IL13RA2 antibody or antigen-binding fragment including the amino acid sequences of the heavy chain CDRs (HCDR1, HCDR2, HCDR3) and light chain CDRs (LCDR1, LCDR2, LCDR3) listed as SEQ ID NO: 1 to 6, to IL13RA2 protein. Whether or not the antibody or its antigen-binding fragment of interest competes with the anti-IL13RA2 antibody or antigen-binding fragment including the amino acid sequences of the heavy chain CDRs (HCDR1, HCDR2, HCDR3) and light chain CDRs (LCDR1, LCDR2, LCDR3) listed as SEQ ID NO: 1 to 6 can be verified by a common method such as the ELISA method, RIA method, FACS method or Biacore™ method.

The anti-IL13RA2 antibody and its antigen-binding fragment according to the invention may have any level of affinity or avidity for IL13RA2. The dissociation constant (KD) may be any of the dissociation constants mentioned as examples herein for binding units. The coupling constant, which includes the dissociation constant, can be determined by a known method using a technique which utilizes the principle of surface plasmon resonance, for example, or utilizing a Biacore™ system.

According to an exemplary aspect, the KD is between about 0.0001 nM and about 1000 nM. According to one embodiment, the KD is about 0.0001 nM or greater, about 0.001 nM or greater, about 0.01 nM or greater, about 0.1 nM or greater, about 1 nM or greater or about 10 nM or greater, and about 1000 nM or lower, about 100 nM or lower, about 10 nM or lower, about 1 nM or lower, about 0.1 nM or lower, about 0.01 nM or lower or about 0.001 nM or lower.

According to an exemplary aspect, the antibody may be in any form of immunoglobulin that is known in the relevant technology. For example, the antibody may be any isotype such as IgA, IgD, IgE, IgG or IgM. The antibody may be a monoclonal or polyclonal antibody. The antibody may be a naturally occurring antibody, isolated from a mammal such as a mouse, rabbit, goat, horse, chicken, hamster, camel or human, and/or a purified antibody. In this regard, the antibody may be treated as a mammal antibody, such as mouse antibody, rabbit antibody, goat antibody, horse antibody, chicken antibody, hamster antibody or human antibody. The term "isolated", as used herein, means removed from its natural environment. The term "purified", as used herein, means isolation of a molecule or compound in a form substantially containing no contaminants normally related to molecules or compounds in the native environment or natural environment, with the purity increasing as a result of separation of the original composition from other components. Here, "purity" is a relative term, and should not necessarily be interpreted as absolute purity, absolute concentration or absolute selection. According to a partial aspect, the purity is at least about 50%, at least about 60%, at least about 70%, at least about 80% or at least about 90% (such as at least about 91%, at least about 92%, at least about 93%, at least about 94%, at least about 95%, at least about 96%, at least about 97%, at least about 98%, at least about 99%, or approximately 100%).

According to a preferred aspect, the anti-IL13RA2 antibody or its antigen-binding fragment according to the invention is internalized in tumor cells expressing IL13RA2. The condition of being internalized in tumor cells expressing IL13RA2 can be examined by contacting the anti-IL13RA2 antibody or its antigen-binding fragment that has been directly or indirectly labeled using fluorescence or similar labeling, with tumor cells expressing IL13RA2, and conducting observation by (phase contrast) microscopy. As an alternative method, internalization in tumor cells expressing IL13RA2 can be evaluated using DT3C, a protein comprising the catalytic region of diphtheria toxin (DT) and three protein G antibody binding regions. DT3C stably binds specifically to the Fc portion of the antibody, and when incorporated into cells induces cell death by inhibiting protein synthesis. By using this system it is possible to simultaneously observe internalization of the antibody and the cytotoxic effect of immunotoxins, allowing screening of antibodies having an internalizing effect (Yamaguchi, M., Hamada, H., et al., Biochemical and Biophysical Research Communications 454 (2014) 600-603). The anti-IL13RA2 antibody or its antigen-binding fragment according to the invention is internalized into IL13RA2-expressing cells at a level of at least 5-fold, at least 10-fold or at least 50-fold, with respect to the level of internalization in non-IL13RA2 expressing cells.

Since the anti-IL13RA2 antibody or its antigen-binding fragment according to the invention thus has the property of not only specifically recognizing tumor cells expressing IL13RA2, but also being internalized in tumor cells expressing IL13RA2, the anti-IL13RA2 antibody or its antigen-binding fragment is suitable for use in a drug delivery system (DDS) for delivery of a drug, such as an anticancer agent into tumor cells. Specifically, the anti-IL13RA2 antibody or its antigen-binding fragment according to the invention may be used in an antibody-drug conjugate (ADC). According to one embodiment, therefore, the invention relates to an antibody-drug conjugate (ADC) comprising an anti-IL13RA2 antibody or its antigen-binding fragment.

Methods suitable for preparing antibodies are well-known technology. For example, the standard hybridoma method is described in Harlow and Lane (ed.), Antibodies: A Laboratory Manual, CSH Press (1988) and CA. Janeway et al. (ed.), Immunobiology, 5th Edition, Garland Publishing, New York, NY (2001).

The monoclonal antibody for use according to the invention may be prepared using any method that allows continuous production of an antibody molecule by culturing of a cell line. Such methods include, but are not limited to, the hybridoma method described first by Koehler and Milstein (Nature, 256:495-497, 1975), the human B cell hybridoma method (Kosbor et al., Immunol. Today, 4:72, 1983; Cote et al., Proc. Natl. Acad. Sci. 80:2026-2030, 1983) and the EBV-hybridoma method (Cole et al., Monoclonal Antibodies and Cancer Therapy, Alan R Liss Inc, New York, N.Y., pp.77-96 (1985).

Briefly, a polyclonal antibody can be prepared by immunizing an animal with a desired immunogen (for example, a recombinant human IL13RA2 polypeptide (such as a polypeptide having the amino acid sequence listed as SEQ ID NO: 17 or a portion thereof (without the signal peptide, for example)); or a membrane fraction of cells expressing human IL13RA2 (for example, A375 cells)), and sampling antiserum from the immunized animal. A wide range of animal species can be used for production of antiserum. According to a partial aspect, the animal used for production of anti-IL13RA2 antiserum is a non-human animal such as a rabbit, mouse, rat, hamster, goat, sheep, camel, pig or horse. Since the volume of rabbit blood is relatively large, according to one exemplary aspect a rabbit is preferably selected for creation of the polyclonal antibody. In a method for producing polyclonal antiserum with immunoreactivity against a selected IL13RA2 epitope, 50 μg of IL13RA2 antigen is emulsified with Freund's complete adjuvant for use in immunization of a rabbit. For booster injection, 50 μg of epitope is emulsified with Freund's incomplete adjuvant at an interval of 21 days, for example. After allowing a period for antibody production, blood is sampled from the animal and a serum sample can be prepared from the whole blood to easily obtain polyclonal antiserum.

For production of a monoclonal antibody, briefly as one exemplary embodiment, a mouse is immunized with a desired immunogen that is to elicit antibodies against it (for example, a recombinant human IL13RA2 polypeptide (such as a polypeptide having the amino acid sequence listed as SEQ ID NO: 17 or a portion thereof (without the signal peptide, for example)); or a membrane fraction of cells expressing human IL13RA2 (for example, A375 cells) (using Freund's complete adjuvant, for example)). Cells ($1 \times 10^8$) obtained from the spleen of the immunized mouse are combined with myeloma cells (such as NS-1 cells or P3U1 cells) and cell fusion is carried out using PEG. Hybridomas producing the desired monoclonal antibody in the culture supernatant are screened by ELISA. An Isostrip system (Boehringer Mannheim, Indianapolis, Ind.), for example, may be used to determine the isotype of the monoclonal antibody produced by the hybridomas. The myeloma cells used may be P3-X63/Ag8, P3-X63-Ag8.653, NS1/1.Ag41, Sp210-Ag14, FO, NSO/U, MPC-11, MPC11-X45-GTG1.7 or S194/15XX0Bul, for mouse splenocytes; or R210.RCY3, Y3-Ag1.2.3, IR983F or 4B210, for rat; or U-266, GM1500-GRG2, LICR-LON-HMy2 or UC729-6, all of which are useful for cell fusion.

Depending on the host cell type, various types of adjuvants may be used to increase the immune response. Such adjuvants include, but are not limited to, Freund's adjuvant, minerals such as aluminum hydroxide, lysolecithin, PLURONIC™ polyol, surface-active substances such as polyanions, peptides, oil-based emulsions, and keyhole limpet hemocyanine and dinitrophenol. BCG (Bacillus Calmette-Guerin) and *Corynebacterium parvum* are potentially useful as human adjuvants.

Examples of other alternative methods known in the art include the EBV-hybridoma method (Haskard and Archer, J. Immunol. Methods, 74(2), 361-67(1984) and Roder et al. Methods Enzymol., 121, 140-67(1986)), and bacteriophage vector expression systems (see Huse et al., Science, 246, 1275-81(1989), for example). Methods of producing antibodies from non-human animals may also be used, as described in US Patent No. 5,545,806, No. 5,569,825 and No. 5,714,352, and U.S. Patent Application Publication No. 2002/0197266Al, for example.

Antibodies may also be produced by inducing *in vivo* production by lymphocyte groups, or by screening a library or panel of highly specific recombinant immunoglobulins as disclosed by Orlandi et al. (Proc. Natl. Acad. Sci. 86:3833-3837; 1989) and Winter and Milstein (Nature, 349:293-299, 1991).

Phage display may also be used to produce antibodies of the disclosure. A phage library encoding the antigen-binding variable (V) domain of the antibody can be produced using standard molecular biology and recombinant DNA techniques (for example, see Sambrook et al. (ed.), Molecular Cloning, A Laboratory Manual, 3rd Edition, Cold Spring Harbor Laboratory Press, New York(2001)). Phages encoding the variable region with desired specificity for specific binding to the desired antigen are selected and used to reconstitute the complete or partial antibody containing the selected variable domain. A nucleic acid sequence coding for the reconstituted antibody is introduced into a suitable cell line such as myeloma cells to be used for hybridoma creation, and thus antibodies with the feature of the monoclonal antibody are secreted by the cells (for example, Janeway et al., Huse et al. and US Patent No. 6,265,150). Related methods are described in US Patent No. 5,403,484, No. 5,571,698, No. 5,837,500 and No. 5,702,892. The techniques described in US Patent No. 5,780,279, No. 5,821,047, No. 5,824,520, No. 5,855,885, No. 5,858,657, No. 5,871,907, No. 5,969,108, No. 6,057,098 and No. 6,225,447 are also considered useful for preparing the antibody of the invention.

Antibodies can also be produced by transgenic mice having gene transfer of specific heavy chain and light chain immunoglobulin genes. Such methods are known technology and are described in US Patent No. 5,545,806 and No. 5,569,825 and Janeway et al., for example.

Methods for producing humanized antibodies are also known technology and are described in detail in Janeway et al., US Patent No. 5,225,539, No. 5,585,089 and No. 5,693,761, EP No. 0239400BI, and UK Patent No. 2188638, for example. Humanized antibodies can also be produced using the antibody resurfacing methods described in US Patent No. 5,639,641 and Pedersen et al., J. Mol. Biol., 235:959-973(1994).

Another technique that may be used, which was developed for creation of "chimeric antibodies", is splicing of a mouse antibody gene onto a human antibody gene to obtain a molecule with appropriate antigen specificity and bioactivity

(Morrison et al., Proc. Natl. Acad. Sci. 81:6851-6855, 1984; Neuberger et al., Nature, 312:604-608, 1984; and Takeda et al., Nature, 314:452-454; 1985). Alternatively, a technique described for creation of single-chain antibodies (US Patent No. 4,946,778) may be applied to create single-chain antibodies specific for IL13RA2.

Methods for creating "humanized antibodies" are well known technology (see US Patent No. 5,585,089 and No. 5,693,762). A humanized antibody generally has one or more amino acid residues introduced into the CDR region and/or framework region from a non-human source. Humanization can be accomplished, for example, by using at least a portion of the complementarity determining region (CDR) of a rodent instead of the corresponding region of the human antibody, using the methods described in Jones et al. (Nature, 321:522-525, 1986), Riechmann et al. (Nature, 332:323-327, 1988) and Verhoeyenet al. (Science, 239:1534-1536, 1988). Numerous techniques for preparing manipulated antibodies are described in Owens and Young, J. Immunol. Meth., 168:149-165(1994), for example. Further changes may then be introduced into the antibody framework to adjust the affinity or immunogenicity.

The framework region (FR) of a mouse antibody is humanized by using a suitable human framework region selected from among a large database of human antibody variable sequences comprising more than 1200 human VH sequences and more than 1000 VL sequences. An antibody sequence database to be used for comparison may be downloaded from the KabatMan web page of Andrew C.R. Martin (http://www.rubic.rdg.ac.uk/abs/). The Kabat method for identifying CDR provides means for determining CDR and FR by defining the approximate CDR and framework regions of the human antibody and comparing them with mouse antibody sequences based on similarity. The maximum matching human VH and VL sequences are selected based on high overall matching of frameworks, lengths of similar CDRs and minimum mismatch between standard residues and VH/VL contacting residues. The human framework region with the greatest similarity to the mouse sequence is inserted between the mouse CDRs. Alternatively, the mouse framework region may be modified by amino acid substitution of all or a portion of the native framework region that is even more closely similar to the framework region of the human antibody.

A "conservative" amino acid substitution is determined based on similarity with the properties of polarity, charge, solubility, hydrophobicity, hydrophilicity and/or amphiphilicity of the residue in question. For example, non-polar (hydrophobic) amino acids include alanine (Ala, A), leucine (Leu, L), isoleucine (Ile, I), valine (Val, V), proline (Pro, P), phenylalanine (Phe, F), tryptophan (Trp, W) and methionine (Met, M); polar neutral amino acids include glycine (Gly, G), serine (Ser, S), threonine (Thr, T), cysteine (Cys, C), tyrosine (Tyr, Y), asparagine (Asn, N) and glutamine (Gln, Q); positively charged (basic) amino acids include arginine (Arg, R), lysine (Lys, K) and histidine (His, H); and negatively charged (acidic) amino acids include aspartic acid (Asp, D) and glutamic acid (Glu, E). The terms "substitution", "insertion" and "deletion" all have a preferred range of 1 to 25, 1 to 20, 1 to 15, 1 to 10 or 1 to 5 amino acids. Mutation may be introduced by using a recombinant DNA method for systematic substitution of amino acids of a polypeptide molecule and carrying out assay of recombinant mutants obtained by activation. Changes in nucleic acid may be at sites with different nucleic acids for different species (variable locations), or highly conserved regions (constant regions).

The anti-IL13RA2 antibody and its antigen-binding fragment of the invention have high affinity for IL13RA2. The suitability of the antibody and its antigen-binding fragment for recognizing antigen expressed on the cell surfaces of IL13RA2-expressing cells and targeting IL13RA2-expressing tumor cells *in vivo* will now be demonstrated. Since IL13RA2-expressing tumor cells are specifically recognized, it is possible to reduce side-effects caused by damage to normal cells. The anti-IL13RA2 antibody and its antigen-binding fragment are expected to be highly effective and cost-efficient for image diagnosis of tumors that overexpress various forms of IL13RA2, for delivery of antibody-radioactive nuclide conjugates, for bioassay for detection of IL13RA2, and as a carrier for therapeutic agents.

In addition to affinity for IL13RA2, the anti-IL13RA2 antibody and its antigen-binding fragment of the invention also has the property of being internalized in tumor cells that express IL13RA2, and can therefore reliably deliver a therapeutic agent into target tumor cells. The anti-IL13RA2 antibody and its antigen-binding fragment of the invention can therefore be effectively utilized in a drug delivery system (DDS), in the form of ADC, for example. Since a therapeutic agent can be reliably delivered into target tumor cells it is expected that the therapeutic effect can thereby be increased.

According to one embodiment, the invention relates to cells that produce the anti-IL13RA2 antibody or its antigen-binding fragment. According to one aspect, the cells are hybridoma cells that have been deposited at the NITE Patent Microorganisms Depositary (NPMD) at the Biotechnology Center of the independent administrative National Institute of Technology and Evaluation of Japan (address: Room 122, 2-5-8 Kazusa Kamatari, Kisarazu City, Chiba Prefecture, Japan) on July 4, 2022, as Accession No. NITE BP-03673. According to one embodiment, the invention relates to nucleic acid coding for an anti-IL13RA2 antibody or its antigen-binding fragment, or cells producing the anti-IL13RA2 antibody or its antigen-binding fragment, which contain the nucleic acid.

According to one embodiment, the invention relates to a method for producing the anti-IL13RA2 antibody or its antigen-binding fragment. The aforementioned cells are preferably used in the production method.

According to one embodiment, the invention relates to a pharmaceutical composition comprising the anti-IL13RA2 antibody or its antigen-binding fragment. According to one aspect, the pharmaceutical composition comprises an antibody-drug conjugate (ADC) comprising an anti-IL13RA2 antibody or its antigen-binding fragment. In other words, the anti-IL13RA2 antibody or its antigen-binding fragment is included in a pharmaceutical composition in the form of an

antibody-drug conjugate (ADC). The antibody-drug conjugate (ADC) is not particularly restricted, so long as it can bind to the anti-IL13RA2 antibody or its antigen-binding fragment, and it may be a drug that can exhibit the desired therapeutic effect (for example, an anticancer agent (such as a low molecular compound or protein)), a compound comprising a radioactive isotope, or liposomes, and it may also be used in a form bound to a substance that can be used as an ADC.

The pharmaceutical composition is preferably a pharmaceutical composition for treatment of IL13RA2-expressing tumors. Such a tumor is selected from the group consisting of melanoma, osteosarcoma, leukemia, lymphoma, prostate cancer, lung cancer (including malignant mesothelioma), glioma, head and neck cancer, renal carcinoma, Kaposi's sarcoma, colorectal cancer, pancreatic carcinoma, adrenal cancer, breast cancer and ovarian cancer.

Any components, formulations and forms of administration known in the field of antibody-containing pharmaceutical compositions may be used in a pharmaceutical composition comprising the anti-IL13RA2 antibody or its antigen-binding fragment of the embodiment, and the descriptions herein may also be applied for the pharmaceutical composition comprising herpes simplex virus.

## EXAMPLES

The present invention will now be explained in greater detail by Examples. However, the invention is in no way limited by the Examples.

<Example 1>

### 1. Materials and methods

#### 1-1. Cells

The human malignant melanoma cell lines A2058 (ATCC CRL-11147) and A375 (ATCC CRL-1619), as well as PLAT-A cells (provided by Toshio Kitamura of University of Tokyo) were cultured using Dulbecco's modified Eagle medium (DMEM, Thermo Fisher Scientific) with addition of 10% FBS (Thermo Fisher Scientific, MA, USA). The monkey kidney cell line Vero (ATCC CCL-81) and the gD-supplemented Vero cell subline VD60 (Ligas et al., J Virol. 1988 May; 62(5):1486-94) (provided by David Johnson of Oregon Health and Science University) were cultured in 5% FBS-added DMEM. The subline (Vero-IL13RA2) obtained by introducing human IL13RA2 into Vero cells was established by infecting Vero cells with a human IL13RA2-expressing retrovirus vector prepared by transfection of PLAT-A cells with pMXc-puro-hIL13RA2, and using 5% FBS-added DMEM medium with further addition of 4 μg/mL puromycin (Thermo Fisher Scientific) for drug selection. The pMXc-puro-hIL13RA2 was prepared by inserting the ORF of human IL13RA2 into the multicloning site of pMXc-puro (provided by Toshio Kitamura of University of Tokyo). The human malignant glioma cell line U87 (ATCC HTB-14) and its sublines, as well as human malignant melanoma cell line RPMI7951 (ATCC HTB-66), were cultured using 10% FBS-added Eagle's Minimum Essential Medium (E-MEM; FujiFilm Wako Pure Chemical Corporation, Osaka, Japan). Among the U87 cells, cell groups binding by anti-IL13RA2 antibody SHM38 (BioLegend, CA, USA) and non-binding cell groups were separated using a cell sorter FACSAria (BD Biosciences, NJ, USA), to establish U87(+) cells and U87(-) cells. The mouse myeloma cell line P3U1 (ATCC CRL-1597) and human non-small-cell lung cancer cell line H1299 (ATCC CRL-5803) were cultured using 10% FBS-added Roswell Park Memorial Institute 1640 medium (RPMI 1640, Thermo Fisher Scientific). H1299(+) cells and H1299(-) cells were established from H1299 cells using the same method as for the U87(+) cells and U87(-) cells. Human umbilical vein endothelial cells HUVEC (PromoCell, Heidelberg, Germany) were cultured using Endothelial Cell Growth Medium 2 (PromoCell) or KBM VEC-1 (Kohjin Bio, Saitama, Japan). The CHO-K1, CHO-HVEM, CHO-nectin-1, J1.1-2, J-HVEM and J-nectin-1 cells used were previously reported lines (Okubo et al., J Virol. 2016Nov 28; 90(24):11096-11105). J-13R2 cells were established by selecting pcDNA3.1-puro-hIL13RA2-transfected J1.1-2 cells using 10% FBS-added DMEM medium with further addition of 16 μg/mL puromycin, and using FACSAria to separate out the IL13RA2-high-expressing populations. The pcDNA3.1-puro-hIL13RA2 was constructed by inserting the ORF of human IL13RA2 into the multicloning site of pcDNA3.1-puro constructed by replacing the G418 resistance gene of pcDNA3.1 (Thermo Fisher Scientific) with a puromycin resistance gene. CHO-13R2 cells were established by selecting pCAcc-puro-hIL13RA2-transfected CHO-K1 cells using 10% FBS-added F-12K medium (Thermo Fisher Scientific) with further addition of 4 μg/mL puromycin, and using FACSAria to separate out the IL13RA2-high-expressing populations. J-13R1/4R cells and CHO-13R1/4R cells were established by drug selection of J1.1-2 cells and CHO-K1 cells co-transfected with pCAcc-puro-hIL13RA1 and pCAcc-puro-hIL4R by culturing in the same manner as J-13R2 and CHO-13R2, and using a FACSAria to separate out the cell populations with high expression of both IL13RA1 and IL4R. The pCAcc-puro-hIL13RA2, pCAcc-puro-hIL13RA1 and pCAcc-puro-hIL4R were constructed by inserting the ORFs of human IL13RA2, human IL13RA1 and human IL4R, respectively, into the multicloning site of pCAcc-puro which was constructed by inserting a puromycin resistance gene expression cassette into pCAcc (Yoshida et al., Biochem Biophys Res Commun. 230, 426-430 (1997)).

1-2. Creation of anti-IL13RA2 antibody-producing hybridomas

After using 2 mM EDTA to collect A375 cells and washing with PBS(-), a hypotonic solution (20 mM Tris-HCl, 50 mM NaCl, 2 mM EDTA) was used to prepare a cell suspension at $5 \times 10^7$ cells/mL. A Dounce homogenizer was used for disruption of the cells on ice, and then the cells were centrifuged for 10 minutes at 4°C, 1,000 × g and the supernatant was collected. The collected supernatant was centrifuged for 1 hour at 4°C, 80,000 × g, the supernatant was discarded, and RPMI 1640 was added for resuspension to prepare a cell membrane fraction solution. The cell membrane fraction solution prepared from $1 \times 10^7$ cells was subcutaneously administered a total of 6 times to female BALB/c mice (Tokyo Laboratory Animals Co., Ltd., Tokyo, Japan), together with 12 μg of AbISCO-100 (Funakoshi Corp., Tokyo, Japan). At 3 days after the final administration, the mice were euthanized and the spleens were extracted. The spleen cells were mixed with P3U1 cells for cell fusion, using polyethylene glycol (PEG1500, Roche, Basel, Switzerland). The created hybridomas were cultured for 6 days using RPMI 1640 medium with addition of 10% Super Low IgG-FBS (Thermo Fisher Scientific), 1× HAT Media Supplement Hybri-Max (Sigma, MO, USA), 5% Briclone (NICB, Dublin, Ireland) and 128 μM 2-mercaptoethanol (Nacalai Tesque, Inc., Kyoto, Japan), after which the medium was exchanged. At 2 days after medium exchange, the culture supernatant was collected, and the culture supernatant, together with 4 μg/mL antibody-bound diphtheria toxin (DT3C) (Yamaguchi, et al., Biochem Biophys Res Commun. 2014 Nov 28; 454(4):600-3) in an equal amount to the culture supernatant, were added to a 96-well plate and incubated for 30 minutes at room temperature. The A375 cell suspension was added in the same amount as the culture supernatant-DT3C mixture, and after culturing for 3 days, the form was observed. The cells in the wells of the culture supernatants observed to have high cytotoxicity were cloned as monoclones by carrying out limiting dilution 3 times. The antibody (4E6 antibody) produced from the hybridoma cells was purified and an IsoStrip (Roche) was used to identify them as subclasses IgG1 and κ. The hybridoma cells producing 4E6 antibody were deposited at the NITE Patent Microorganisms Depositary (NPMD) of the independent administrative National Institute of Technology and Evaluation of Japan (address: No. 122, 2-5-8 Kazusa Kamatari, Kisarazu City, Chiba Prefecture, Japan) on July 4, 2022, as Accession No. NITE BP-03673.

1-3. Construction of anti-IL13RA2 single-chain antibody

RNA extracted from 4E6 antibody-producing hybridomas using an RNeasy Mini Kit (Qiagen, CA, USA) was used as template for RT-PCR using SuperScript III One-step RT-PCR System with Platinum Taq High Fidelity DNA polymerase (Thermo Fisher Scientific), to amplify the antibody gene light chain and heavy chain. The light chain was amplified using the primers 5'- GGAAGATCTGAYATTGTGMTSACMCARWCTMCA-3' (SEQ ID NO: 18) and 5'-CCGAATTCGGATACAGT TGGTGCAGCATC-3' (SEQ ID NO: 19), and the heavy chain was amplified using 5'-GGAAGATCTSARGTNMAGCTGSAGSAGTC-3' (SEQ ID NO: 20), 5'-GGAAGATCTSARGTNMAGCTGSAGSAGTCWGG-3' (SEQ ID NO: 21) and 5'-C CGAATTCATAGACAGATGGGGGTGTCGTTTTGGC-3' (SEQ ID NO: 22) (Wang et al., J Immunol Methods 233 2000. 167-177). The fragment obtained by RT-PCR was cloned in pTAC-2 vector (BioDynamics Laboratory, Inc., Tokyo, Japan), and the sequences of the light chain and heavy chain were determined by sequence analysis. The determined sequences were linked via $(Gly_4Ser)_3$-containing spacer sequences (linker sequences) to construct scFv. In the primer sequences shown above, Y represents T or C, M represents A or C, S represents G or C, R represents G or A, W represents A or T, and N represents A, G, C or T.

1-4. HSV-BAC recombination

All of the HSV-BAC constructs used in this experiment were from KOS-37 BAC (provided by David Leib of Dartmouth Medical School) (Gierasch et al., J Virol Methods. 2006 Aug; 135(2):197-206). All of the recombination protocols were carried out based on the Red homologous recombination system (Tischer et al., Biotechniques. 2006 Feb; 40(2):191-7). Plasmids pKGN-sc13R2, pKGN-IL13 and pKGN-IL13zr were created using a transfer construct prepared by PCR using pgD:224/Y38C-sc13R2kan, pgD:224/Y38C-IL13kan and pgD:dSP-32/V34S-IL13kan as template, with modification of pKGN gD in the same manner as a previously reported method (Shibata, et al., Gene Ther. 2016 23(6):479-88). Plasmids pgD:224/Y38C-sc13R2kan, pgD:224/Y38C-IL13kan and pgD:dSP-32/V34S-IL13kan were created by inserting the I-SceI recognition sequence, the kanamycin resistance gene, and a 50 bp homologous sequence for removal of the kanamycin resistance gene, into the AflII site of pgD:224/Y38C-sc13R2, pgD:224/Y38C-IL13 and pgD:dSP-32/V34S-IL13 by the same method as previously reported (Shibata, et al., Gene Ther. 2016 23(6):479-88). Plasmid pgD:224/Y38C-sc13R2 was created by inserting the scFv sequence constructed from 4E6 antibody-producing hybridomas by the same method as previously reported (Shibata, et al., Gene Ther. 2016 23(6):479-88), into the deleted region of amino acids from position 2 to position 24 of gD of pgD:224/Y38C (Uchida, et al., Mol Ther. 2013 Mar; 21(3):561-9). Plasmid pgD:224/Y38C-IL13 was created by inserting the BamHI fragment of DNA amplified using primers 5'-AAGAATTCGCTAGCGGTGGTGGTGGATCCGGCCCTGTGCCTCCCTCTA-3' (SEQ ID NO: 23) and 5'-CCGGGAT CCTGAACCTCCACCACCGTTGAACTGTCCCTCGCGAAAAAG-3' (SEQ ID NO: 24), into the deleted region of amino

acids from position 2 to position 24 of gD of pgD:224/Y38C, with pGEM-T Easy-hIL13 encoding the ORF of human IL13 as template. Plasmid pgD:dSP-32/V34S-IL13 was created by inserting the fragment from the AflII site to the BamHI site of DNA amplified using primers 5'-CCCTTAAGGTCTCTTTTGTGTGGTGCGTTCCGGTATGGCGCTTTTGTTGACCACG GTC ATTGCTCTCACTTGCCTTGGCGGCTTTGCCTCCCCAGGCCCTGTGCCTCCCTCTAC-3' (SEQ ID NO: 25) and 5'-TTGGATCCGGTACCGTTGAACTGTCCCTCGCGAAAAAGTT-3' (SEQ ID NO: 26), with the IL13 ORF as template, and the fragment from the BamHI site to the BspEI site of DNA amplified using primers 5'-ATCGGATCCCGGCGCGT GTACCACATCCAGG-3' (SEQ ID NO: 27) and 5'-TCCGGACGTCTTCGGAGGCCCC-3' (SEQ ID NO: 28), with the ORF of wild type gD as template, into the sequence from the AflII site to the BspEI site of pgD:224/Y38C, including the gD N-terminal end. Plasmid pKGN-sc13R2-BhKt was created by modifying the anti-EGFR single-chain antibody-inserted gD of pKGNE-BhKt to the single-chain antibody-inserted gD of 4E6, by the same method as for modification of pKGN to pKGN-sc13R2.

1-5. Virus

The KGN used was as previously reported, having the EGFP expression cassette inserted between the UL3 gene and UL4 gene, and having an intracellular entry-promoting double mutation (gB:D285 N/A549T) in gB (Shibata, et al., Gene Ther. 2016 23(6):479-88). The KGN, KGN-IL13 and KGN-IL 13zr used in Fig. 2 and Fig. 3 were prepared either by infecting VD60 cells with KGN, or by cotransfecting pKGN-IL13 or pK GN-IL13zr with the Cre recombinant enzyme-expressing plasmid pxCANCre (provided by Izumu Saito of University of Tokyo). After infecting Vero cells with these wild type gD-supplemented viruses and inactivating the viruses remaining outside the cell by acid treatment using pH 3.0 glycine buffer, the viruses were purified by a previously reported method to prepare purified viruses with the VD60-derived wild type gD removed (Uchida, et al., J Virol. 2009 Apr; 83(7):2951-61). The viral genome titer (gc/mL) was measured by qPCR for the gD gene, as previously reported (Miyagawa et al., Proc Natl Acad Sci USA. 2015 Mar 31; 112(13):E1632-41). The KGN-IL13, KGN-sc13R2 and KGN-sc13R2-BhKt not used in Fig. 2 and Fig. 3 were prepared by cotransfection of Vero-IL13RA2 cells with pKGN-IL13, pKGN-sc13R2 or pKGN-sc13R2-BhKt, with pxCANCre. These viruses were cloned as single clones by limiting dilution twice using Vero-IL13RA2. A previously reported method was used to confirm removal of the BAC sequence in the cloned single clones (Miyagawa et al., Proc Natl Acad Sci USA. 2015 Mar 31; 112(13):E1632-41). The viruses and KGN isolated as single clones using Vero-IL13RA2 were propagated, and purified virus was prepared by purifying the viruses by the method described above. The plaque-forming unit titer (pfu/mL) of the purified virus was measured by a previously reported method using Vero-IL13RA2 (Uchida et al., J Virol. 2013 Feb; 87(3):1430-42).

1-6. Flow cytometry analysis

Flow cytometry analysis was carried out using LSRFortessa (BD Biosciences). The primary antibodies used were 4E6 antibody, mouse anti-IL13RA2 monoclonal antibody SHM38 (BioLegend), mouse anti-IL13RA1 monoclonal antibody GM-1E7 (Santa Cruz Biotechnology, Dallas, TX), mouse anti-IL4R monoclonal antibody G077F6 (BioLegend) and the isotype-matched control antibodies MG1-45 and MOPC-173 (BioLegend). The secondary antibody used was Alexa Fluor 488-labeled goat anti-mouse IgG (H+L) polyclonal antibody (Thermo Fisher Scientific).

1-7. Entry assay, infectious center assay, plaque formation assay and cell killing assay

An entry assay (Shibata, et al., Gene Ther. 2016 23(6):479-88), infectious center assay (Uchida, et al., J Virol. 2009 Apr; 83(7):2951-61), plaque formation assay (Shibata, et al., Gene Ther. 2016 23(6):479-88) and cell killing assay (Uchida, et al., Mol Ther. 2013 Mar; 21(3):561-9) were carried out in the same manner as previously reported methods.

1-8. Animal experiment

All of the animal experiments were conducted while carefully following the implementation rules and manual as approved by the Animal Experimentation Committees of the University of Tokyo or Tokyo University of Pharmacy and Life Sciences. U87(+) or H1299(+) cells suspended in Hank's balanced salt solution (Thermo Fisher Scientific) were subcutaneously transplanted at $1 \times 10^7$/100 μL into the left abdomen of 6- to 8-week-old, female severe-combined immunodeficient mice SCID-Beige (CB17.Cg-Prkdc$^{scid}$Lyst$^{bg-J}$/CrlCrlj; Charles River Laboratories, Japan Inc., Kanagawa, Japan). When the average tumor volume reached ~290 mm$^3$ (U87(+)) or ~150 mm$^3$ (H1299(+)), the mice were grouped according to approximate average tumor volume, and PBS or the virus solution suspended in PBS was administered into the tumor at a volume of 30 μL, or into the tail vein at a volume of 200 μL. The tumor volume was determined by the formula (length $\times$ short diameter$^2$)/2 (Tomayko et al., Cancer Chemother Pharmacol. 1989; 24(3):148-54). Mice with tumor volumes exceeding 10% body weight were considered to have succumbed to the tumor

and were euthanized.

1-9. Statistical analysis

Statistical analysis of plaque formation assay was carried out by the two-tailed Mann-Whitney U test. Statistical analysis of tumor volume was carried out by two-way repeated measurement distributed analysis. In all of the statistical analyses, a P value of $<0.05$ was judged to be statistically significant.

2. Results

2-1. Creation of HSV with IL-13 inserted in targeted gD platform

Zhou and Roizman have developed RR-oHSV (R5141) that can infect via IL13RA2, by substituting IL-13 for the gD signal peptide and the amino acids following it from position 1 to position 32, and mutating valine at position 34 to serine (Zhou and Roizman, Proc Natl Acad Sci USA 2006 Apr 4; 103(14):5508-13). In order to examine whether or not differences in the IL-13-inserted gD construct leads to differences in target specificity for intracellular entry, the targeted gD platform reported by Uchida et al. of our research group, i.e. a virus (KGN-IL13) having IL-13 inserted into a gD mutant with deletion of amino acids from position 2 to position 24 and mutation of tyrosine at position 38 to cysteine, was constructed, and a virus (KGN-IL13zr) having the IL-13-inserted gD mutant as reported by Zhou and Roizman was also constructed as a comparative control (Fig. 1).

2-2. KGN-IL13 entered cells via IL13RA2 but virtually failed to enter cells via HVEM or nectin-1.

J1.1-2 cells, as a cell line lacking expression of gD receptor, and a subline of J1.1-2 with forced expression of gD receptor or IL13RA2 (J-HVEM cells, J-nectin-1 cells and J-13R2 cells) were infected with two IL-13-inserted HSVs (KGN-IL13 and KGN-IL13zr), and non-targeted HSV (KGN) as their parent virus, having the same EGFP expression cassette and an intracellular entry enhanced mutation (gB:NT mutation). At 8 hours after start of infection, establishment of intracellular entry of the virus was evaluated using the EGFP fluorescent signal as the indicator (Fig. 2). KGN infiltrated the two types of gD receptor-expressing cells, but could not infiltrate J1.1-2 and J-13R2 cells. With KGN-IL13zr, intracellular entry was detected into J-13R2 cells only under conditions of 300 gc/cell. While intracellular entry into J-HVEM cells was not detected, a greater degree of intracellular entry was detected into J-13R2 cells than into J-nectin-1 cells. These observations do not match reporting by Zhou and Roizman who found that R5141 having the same gD construct as the IL-13-inserted gD of KGN-IL13zr virtually failed to infiltrate J-nectin-1 cells but did infiltrate J-13R2 cells. It is conjectured that this divergence is due to the fact that in R5141, unlike KGN-IL13zr, the N-terminal region of gC which is responsible for adsorption of HSV into cells is replaced with IL-13, and the polylysine (pK) region of gB which is also responsible for adsorption is deleted (Zhou and Roizman, Proc Natl Acad Sci USA 2006 Apr 4; 103(14):5508-13). In other words, it is possible that the IL13RA2 selectivity of R5141 is imparted primarily at the adsorption stage.

In comparison to KGN-IL13zr, KGN-IL13 infiltrated J-13R2 cells more efficiently than KGN-IL13zr. In addition it was shown that KGN-IL13 not only fails to enter J-HVEM cells, but also had virtually no detectable infiltration into J-nectin-1 cells, even under conditions of 300 gc/cell. A similar result was observed when using CHO-K1 cells and their sublines, as separate cell lines lacking expression of gD receptor (Fig. 3). The results suggested that KGN-IL13 is more suitable than KGN-IL13zr for targeting of IL13RA2.

2-3. KGN-IL13 infiltrated IL13RA1-expressing cells as well, but KGN-sc13R2 specifically infiltrated only into IL13RA2-expressing cells.

IL-13 binds to IL13RA1, though with lower affinity than IL13RA2. It has also been reported that when IL13RA1 forms a complex with IL4R (IL13RA1/IL4R), it binds with higher affinity than IL13RA1 alone. It was then examined whether IL13RA1/IL4R functions as a receptor for KGN-IL13. As a control virus there was created KGN-sc13R2 in which an anti-IL13RA2 single-chain antibody (sc13R2), prepared alone by our research group using the following method, was inserted into the same targeted gD platform as KGN-IL13.

First, a cell membrane fraction of the malignant melanoma cell line A375, known to express IL13RA2, was used to immunize mice, from which spleen cells were extracted and fused with the mouse myeloma cell line P3U1, to prepare a hybridoma cell library. The antibody-binding modified diphtheria toxin (DT3C) reported by Yamaguchi et al. (Yamaguchi, et al., Biochem Biophys Res Commun. 2014 Nov 28; 454(4):600-3) was used to select from a hybridoma cell library the hybridoma cells producing antibodies with high cytotoxicity against A375 cells, and they were cloned as single clones. A protein extract was prepared from the A375 cells, the antibody produced by the clones (4E6 antibody) was used for immunoprecipitation, and mass spectrometry analysis of the precipitate showed the presence of a peptide fragment

possibly from IL13RA2. Based on these results, flow analysis was carried out to confirm the binding affinity of 4E6 antibody to CHO-K1 cells and to a subline of CHO-K1 with forced expression of IL13RA2 (CHO-13R2), and it was found that the 4E6 antibody did not bind to CHO-K1 cells but did bind to CHO-13R2 cells. It was therefore judged that the antigen of 4E6 antibody is IL13RA2. The antibody gene was cloned from the 4E6 antibody-producing hybridomas, and a gD mutant having the scFv sequence derived from it inserted into the targeted gD platform was incorporated to construct KGN-sc13R2.

Entry assay was carried out using the J-13R2, J-HVEM and J-nectin-1 cells used in Fig. 2, and also a subline of J1.1-2 with forced expression of IL13RA1/IL4R (J-13R1/4R), and the specificity of intracellular entry of KGN-sc13R2 was compared with KGN-IL13 (Fig. 4). KGN-IL13 and KGN-sc13R2 both infiltrated J-13R2 cells, but of these two viruses only KGN-IL13 was found to infiltrate J-13R1/4R cells. At MOI 10, KGN-IL13 infiltrated J-nectin-1 cells to a slight extent, while KGN-sc13R2 completely failed to infiltrate. Neither of the two viruses infiltrated J-HVEM cells. Similar results were also observed in an entry assay using CHO-K1 cells and their sublines (Fig. 5). These results suggest that KGN-IL13 utilizes not only IL13RA2 but also IL13RA1/IL4R as a receptor for intracellular entry, whereas KGN-sc13R2 utilizes IL13RA2 alone as a receptor for intracellular entry.

2-4. KGN-IL13 killed vascular endothelial cells but KGN-sc13R2 did not exhibit cytotoxicity.

Vascular endothelial cells are normal cells thought to come into viral contact immediately after intravenous administration of oHSV. The infectivity of KGN-IL13 and KGN-sc13R2 for vascular endothelial cells was therefore examined in a comparative manner. First, IL-13 receptor and IL4R expression in human umbilical vein endothelial cells (HUVEC) was examined by flow analysis. The anti-IL13RA1 antibody and anti-IL4R antibody bound to HUVEC, but the anti-IL13RA2 antibody did not bind to HUVEC (Fig. 6(a)). This result suggested that IL13RA1 and IL4R are expressed in HUVEC but not in IL13RA2.

An entry assay and cell killing assay were carried out to ascertain whether KGN-IL13 infiltrates HUVEC and exhibits cytotoxicity. As seen in Fig. 6(b) and Fig. 6(c), of the three viruses examined, KGN infiltrated with highest efficiency into HUVEC, exhibiting notable cytotoxicity (Fig. 6(b), Fig. 6(c)). KGN-IL13 infiltrated HUVEC and exhibited cytotoxicity when the virus was added at relatively high MOI. In all of the experiments, however, KGN-sc13R2 failed to infiltrate into HUVEC or exhibit cytotoxicity even when the virus was added at the maximum MOI. In the Vero-IL13RA2 cells used as positive control cells, KGN-sc13R2 entered the cells at about the same efficiency as KGN-IL13, and exhibited about the same or higher cytotoxicity. These results suggested that systemic administration of KGN-IL13 can kill vascular endothelial cells, but that this is probably unlikely to be true for KGN-sc13R2.

Based on the results described above, it was concluded that insertion of anti-IL13RA2 single-chain antibody into the targeted gD platform prepared here is suitable as a gD construct for development of IL13RA2-targeting RR-oHSV with higher target specificity.

2-5. KGN-sc13R2 infiltrated and propagated only in cells expressing IL13RA2.

In order to evaluate infectivity of the anti-IL13RA2 single-chain antibody-inserted RR-oHSV into human cancer cells, the expression of IL13RA2 in several human cancer cell lines was confirmed by flow analysis using commercially available anti-IL13RA2 antibody (SHM38 antibody). Two of the examined cell lines, the malignant glioma cell line U87 and the non-small cell lung cancer cell line H1299, consisted of a population expressing IL13RA2 on the cell surfaces and a non-expressing population (Fig. 7). In order to separately examine the infectivity of the anti-IL13RA2 single-chain antibody-inserted RR-oHSV for each cell population, the cell lines were separated into an IL13RA2 high-expressing population and a non-expressing population, by cell sorting using SHM38 antibody, referred to below as U87(+) or H1299(+) for the high-expressing population hereunder and U87(-) or H1299(-) for the non-expressing population. The binding capacity of the 4E6 antibody for these cell lines and the malignant melanoma cell lines RPMI7951 and A2058 were examined by flow analysis (Fig. 8(a)). As a result, the 4E6 antibody bound to U87(+) and H1299(+), as well as to RPMI7951 cells, but did not bind to U87(-), H1299(-) or A2058 cells. Since 4E6 antibody was found to bind only to the IL13RA2-positive cell population sorted with SHM38 antibody, this suggests that 4E6 antibody has high antigen specificity.

In recent years the present inventors have developed a virus (RRsyn-oHSV) having a mutation (syncytial mutation) that induces multinucleated giant cell formation in infected cells, incorporated into the two different glycoproteins gB and gK of RR-oHSV (Okubo et al., J Virol. 2016 Nov 28; 90(24):11096-11105). Since these syncytial mutations (gB:R858H/gK:A40T and BhKt) enhanced intercellular propagation efficiency and cytotoxicity without impairing the target specificity of EGFR targeting RR-oHSV, this suggests that it is possible to enhance the efficacy of RR-oHSV while maintaining its safety. Therefore, KGN-sc13R2-BhKt was created having BhKt introduced into KGN-sc13R2, and its target specificity and infection efficiency were examined in comparison to KGN-sc13R2.

To examine the target specificity for intracellular entry into cancer cells, first an entry assay was carried out with an IL13RA2-positive or negative human cancer cell line (Fig. 8(b)). The KGN used as a control infiltrated into all of the cells,

whereas KGN-sc13R2 and KGN-sc13R2-BhKt infiltrated only into the IL13RA2-positive cancer cell line. An infectious center assay was carried out next to evaluate specificity of intercellular propagation (Fig. 8(c)). KGN formed plaques in all of the cell lines, but KGN-sc13R2 and KGN-sc13R2-BhKt formed plaques only with the IL13RA2-positive cells. Moreover the plaques formed in those cell lines by KGN-sc13R2-BhKt exhibited a multinucleated giant cell morphology. The results suggested that the anti-IL13RA2 single-chain antibody-inserted RR-oHSV has high target specificity for intracellular entry and intercellular propagation, regardless of the presence of BhKt.

2-6. Intercellular propagation and cell killing of anti-IL13RA2 single-chain antibody-inserted RR-oHSV for cancer cells increased by introduction of BhKt.

In order to examine the efficacy of the anti-IL13RA2 single-chain antibody-inserted RR-oHSV *in vitro,* the plaque areas in IL13RA2-positive cancer cell lines were compared. The results showed that all of the plaque areas in IL13RA2-positive cancer cell lines were significantly larger with KGN-sc13R2-BhKt than with KGN-sc13R2 (approximately 7-fold in U87(+) cells, approximately 50-fold in H1299(+) cells and approximately 9-fold in RPMI7951 cells), suggesting that KGN-sc13R2-BhKt propagates more efficiently than KGN-sc13R2 (Fig. 9(a)). When cytotoxicity for IL13RA2-positive cancer cell lines was examined by cell killing assay, KGN-sc13R2-BhKt exhibited a lower $ED_{50}$ value than KGN-sc13R2 in all of the cell lines (approximately 1/4 in U87(+) cells, approximately 1/200 in H1299(+) cells and approximately 1/10 in RPMI7951 cells), suggesting that KGN-sc13R2-BhKt has higher cytotoxicity than KGN-sc13R2 (Fig. 9(b)).

These results showed that introduction of BhKt may have enhanced intercellular propagation and cell killing by KGN-sc13R2, without impairing the target specificity of RR-oHSV, suggesting that KGN-sc13R2-BhKt is the virus that can exhibit the highest safety and efficacy, among the IL13RA2-targeting RR-oHSV examined in this experiment.

2-7. KGN-sc13R2-BhKt exhibited powerful antitumor effect against IL13RA2-positive tumors *in vivo.*

In order to examine whether or not KGN-sc13R2-BhKt exhibits a powerful antitumor effect *in vivo,* U87(+) cells, derived from U87 cells which have been commonly used in previous preclinical testing of oncolytic HSV, were evaluated for antitumor effect in a subcutaneous tumor model. At 9 days after subcutaneous grafting of the U87(+) cells into severe immunodeficient mice SCID-Beige, KGN-sc13R2-BhKt was intratumorally injected once at $10^2$ pfu (Fig. 10(a)). While increase in tumor size was seen in all individuals in the PBS treatment group, complete tumor regression was seen in all individuals by the 17th day after virus administration in the KGN-sc13R2-BhKt treatment group ($p < 0.0001$). These results clearly demonstrated that KGN-sc13R2-BhKt exhibits a very powerful antitumor effect even with administration of the virus in a very small amount of $10^2$ pfu.

In order to examine whether or not a high antitumor effect is seen even in subcutaneous tumor models of cell lines other than U87(+) cells, KGN-sc13R2-BhKt was intratumorally administered once at $10^2$ or $10^4$ pfu, 9 days after subcutaneous grafting of H1299(+) cells into SCID-Beige mice (Fig. 10(b)). In this model, there was only one individual with complete tumor regression in the $10^4$ pfu-administered group, but an antitumor effect was seen with KGN-sc13R2-BhKt in a dose-dependent manner, with a significant antitumor effect being exhibited even with administration of the virus in a small amount of $10^2$ pfu ($p < 0.0001$). These results suggested the possibility that KGN-sc 13R2-BhKt can exhibit a powerful antitumor effect against numerous types of cancer even with a very small amount of virus, though to a degree dependent on the type of cancer cell.

These observations demonstrate that infection of KGN-sc13R2-BhKt established in only part of a tumor can propagate throughout the entire tumor. Since it was thought that the virus may therefore exhibit a powerful antitumor effect even when administered intravenously, this possibility was also tested (Fig. 10(c)). When KGN-sc13R2-BhKt was intravenously administered at $10^6$ pfu to the U87(+) cell subcutaneous tumor model, the tumor had undergone complete regression in all of the individuals by the 17th day after administration ($p < 0.0001$). This demonstrated that KGN-sc13R2-BhKt can exhibit a powerful antitumor effect even by intravenous administration.

The results suggested that KGN-sc13R2-BhKt has high target specificity for IL13RA2 and can serve as a cancer therapeutic agent exhibiting a powerful antitumor effect.

3. Discussion

In this experiment, with a comparative test carried out on targeted gD platforms used for insertion of IL-13 as a cancer targeting module into gD for targeting of IL13RA2, it was demonstrated that the gD construct developed by Uchida et al. of our research group is more suitable than the gD construct previously developed by Zhou and Roizman, for excellent target specificity of intracellular entry and greater efficiency of infiltration via target molecules. However, it was shown that the IL-13-inserted gD mutant also establishes intracellular entry via IL13RA1/IL4R, and infects normal cells expressing IL13RA1/IL4R. On the other hand, the anti-IL13RA2 single-chain antibody inserted gD mutant established intracellular entry only via IL13RA2, without establishing intracellular entry via IL13RA1/IL4R. Moreover the BhKt mutation enhanced plaque formation ability and cytotoxicity of KGN-sc13R2 without loss of infection specificity. It was demonstrated that KGN-sc13R2-BhKt exhibits an antitumor effect by intratumoral administration to mouse models with tumors of

numerous IL13RA2-positive human cancer cell lines, and exhibits a powerful antitumor effect even when intravenously administered.

Since KGN-IL13 infiltrated cells expressing IL13RA1, this demonstrated that when it is used as a cancer targeting module for inserting IL-13 into gD, the IL13RA1 receptor functions as a receptor for HSV. It has been suggested by several research groups that the binding capacity of IL-13 and IL13RA1 can be reduced by introducing amino acid mutations such as E13K or E13Y into IL-13 (Debinski, et al., Nat Biotechnol. 1998 May; 16(5):449-53.; Kahlon, et al., Cancer Res. 2004 Dec 15; 64(24):9160-6). Consequently, by creating RR-oHSV, having IL-13 with these introduced mutations incorporated as a cancer targeting module into gD, it may be possible to avoid infection of IL13RA1-expressing normal cells. For actual application in cancer treatment, however, it is necessary to consider the possibility that mutations may be acquired during viral proliferation that restore bindability with IL13RA1, consequently leading to spread of infection to IL13RA1-expressing normal cells. From this viewpoint as well, it is possible that anti-IL13RA2 single-chain antibody is more suitable than IL-13 as a cancer targeting module for development of RR-oHSV specifically targeting IL13RA2.

KGN-sc13R2-BhKt exhibited an antitumor effect against immune-deficient cancerous mice by single dose of an exceedingly low viral level of $10^2$ pfu, and also produced no definite toxicity. This suggested that KGN-sc13R2-BhKt can serve as a cancer treatment agent with very high efficacy and safety, not only by intratumoral administration but also by intravenous administration. This property is considered to be especially beneficial when treating cancer patients with distant metastasis.

<Example 2>

A virus with deletion of the ICP34.5 gene of the KGN-sc13R2-BhKt prepared in Example 1 was also created (KGΔN-sc13R2-BhKt). The method for creating a virus with deletion of the ICP34.5 gene of the parent virus KGN-sc13R2-BhKt can be carried out with reference to the method described in International Patent Publication No. WO2020/090871. Specifically, since the ICP34.5 gene has two copies in the herpesvirus genome, the procedure for deleting the two copies was carried out in two stages, one for each copy, using the method described in detail by Suzuki et al. (Mol Ther Oncolytics. 2021; 22:265-276). The created construct pKGΔN-sc13R2-BhKt was used in cotransfection together with pxCANCre, similar to the method described in Example 1 ("1-5. Virus"). These viruses were cloned as single clones by limiting dilution twice using Vero-IL13RA2. A previously reported method was used to confirm removal of the BAC sequence in the cloned single clones (Miyagawa et al., Proc Natl Acad Sci USA. 2015 Mar 31; 112(13):E1632-41). The virus isolated as a single clone using Vero-IL13RA2 was propagated, and purified virus was prepared by purifying the viruses by the method described above. The plaque-forming unit titer (pfu/mL) of the purified virus was measured by a previously reported method using Vero-IL13RA2 (Uchida et al., J Virol. 2013 Feb; 87(3):1430-42).

When the parent virus KGN-sc13R2-BhKt and the created KGΔN-sc13R2-BhKt were each intratumorally administered once ($10^2$ pfu) into a U87-IL13RA2+ subcutaneous tumor model, a remarkable antitumor effect was exhibited in both of the virus treatment groups (Fig. 11). Moreover tumor regression was seen in almost all of the individuals of the KGΔN-sc13R2-BhKt treatment group. This suggested that the excellent antitumor effect of KGN-sc13R2-BhKt is virtually unimpaired even with deletion of ICP34.5, i.e. with restricted growth modification.

[Table 2-1]

| SEQ ID NO: | Description | Sequence |
|---|---|---|
| SEQ ID NO: 1 | 4E6_HCDR1 | RHGIH |
| SEQ ID NO: 2 | 4E6_HCDR2 | VIWAGGSTNYNSALMS |
| SEQ ID NO: 3 | 4E6 HCDR3 | DHFDSFDY |

(continued)

| SEQ ID NO: | Description | Sequence |
|---|---|---|
| SEQ ID NO: 4 | 4E6 LCDR1 | TASSSVSSSYLH |
| SEQ ID NO: 5 | 4E6 LCDR2 | STSNLAS |
| SEQ ID NO: 6 | 4E6 LCDR3 | HQYHRSPLT |
| SEQ ID NO: 7 | 4E6_scFv | DIVLTQTPAIMSASLGERVTMTCTASSSVSSSYLHWYQQKPGSSPKLWIYSTS NLASGVPLRFSGSGSGTSYSLTISSMEAEDAATYYCHQYHRSPLTFGAGTKLE LKRAASGGGGSGGGGSGGGGSEVKLEESGPGLVAPSQSLSIACTVSGFSLTRH GIHWVRQPPGKNLEWLGVIWAGGSTNYNSALMSRLSIIKDNSKSQVFLKMNS LQTVDTAMYYCARDHFDSFDYWGQGTTLTVSSAGGGGSGS |
| SEQ ID NO: 8 | 4E6_scFv-VL | DIVLTQTPAIMSASLGERVTMTCTASSSVSSSYLHWYQQKPGSSPKLWIYSTS NLASGVPLRFSGSGSGTSYSLTISSMEAEDAATYYCHQYHRSPLTFGAGTKLE LKRA |
| SEQ ID NO: 9 | 4E6_scFv_linker_sequence 1 | ASGGGGSGGGGSGGGGS |
| SEQ ID NO: 10 | 4E6_scFv-VH | EVKLEESGPGLVAPSQSLSIACTVSGFSLTRHGIHWVRQPPGKNLEWLGVIWA GGSTNYNSALMSRLSIIKDNSKSQVFLKMNSLQTVDTAMYYCARDHFDSFDY WGQGTTLTVSSA |
| SEQ ID NO: 11 | 4E6_scFv_linker_sequence 2 | GGGGSGS |
| SEQ ID NO: 12 | gD | KYALADASLK MADPNRFRGK DLPVLDQLTD PPGVRRVYHI QAGLPDPFQP PSLPITVYYAVLERACRSVL LNAPSEAPQI VRGASEDVRK QPYNLTIAWF RMGGNCAIPI TVMEYTECSYNKSLGACPIR TQPRWNYYDS FSAVSEDNLG FLMHAPAFET AGTYLRLVKI NDWTEITQFILEHRAKGSCK YALPLRIPPS ACLSPQAYQQ GVTVDSIGML PRFIPENQRT VAVYSLKIAGWHGPKAPYTS TLLPPELSET PNATQPELAP EDPEDSALLE DPVGTVAPQI PPNWHIPSIQDAATPYHPPA TPNNMGLIAG AVGGSLLAAL VICGIVYWMH RRTRKAPKRI RLPHIREDDQPSSHQPLFY |

(continued)

| SEQ ID NO: | Description | Sequence |
|---|---|---|
| SEQ ID NO: 13 | gB | MHQGAPSWGR RWFVVWALLG LTLGVLVASA APSSPGTPGV AAATQAANGG PATPAPPALGAAPTGDPKPK KNKKPKNPTP PRPAGDNATV AAGHATLREH LRDIKAENTD ANFYVCPPPTGATVVQFEQP RRCPTRPEGQ NYTEGIAVVF KENIAPYKFK ATMYYKDVTV SQVWFGHRYSQFMGIFEDRA PVPFEEVIDK INAKGVCRST AKYVRNNLET TAFHRDDHET DMELKPANAATRTSRGWHTT DLKYNPSRVE AFHRYGTTVN CIVEEVDARS VYPYDEFVLA TGDFVYMSPFYGYREGSHTE HTSYAADRFK QVDGFYARDL TTKARATAPT TRNLLTTPKF TVAWDWVPKRPSVCTMTKWQ EVDEMLRSEY GGSFRFSSDA ISTTFTTNLT EYPLSRVDLG DCIGKDARDAMDRIFARRYN ATHIKVGQPQ YYLANGGFLI AYQPLLSNTL AELYVREHLR EQSRKPPNPTPPPPGASANA SVERIKTTSS IEFARLQFTY NHIQRHVNDM LGRVAIAWCE LQNHELTLWNEARKLNPNAI ASVTVGRRVS ARMLGDVMAV STCVPVAADN VIVQNSMRIS SRPGACYSRPLVSFRYEDQG PLVEGQLGEN NELRLTRDAI EPCTVGHRRY FTFGGGYVYF EEYAYSHQLSRADITTVSTF IDLNITMLED HEFVPLEVYT RHEIKDSGLL DYTEVQRRNQ LHDLRFADIDTVIHADANAA MFAGLGAFFE GMGDLGRAVG KVVMGIVGGV VSAVSGVSSF MSNPFGALAVGLLVLAGLAA AFFAFRYVMR LQSNPMKALY PLTTKELKNP TNPDASGEGE EGGDFDEAKLAEAREMIRYM ALVSAMERTE HKAKKKGTSA LLSAKVTDMV MRKRRNTNYT QVPNKDGDADEDDL |

[Table 2-2]

| | | |
|---|---|---|
| SEQ ID NO: 14 | gK | MLAVRSLQHL STVVLITAYG LVLVWYTVFG ASPLHRCIYA VRPTGTNNDT ALVWMKMNQTLLFLGAPTHP PNGGWRNHAH ICYANLIAGR VVPFQVPPDA TNRRIMNVHE AVNCLETLWYTRVRLVVVGW FLYLAFVALH QRRCMFGVVS PAHKMVAPAT YLLNYAGRIV SSVFLQYPYTKITRLLCELS VQRQNLVQLF ETDPVTFLYH RPAIGVIVGC ELMLRFVAVG LIVGTAFISRGACAITYPLF LTITTWCFVS TIGLTELYCI LRRGPAPKNA DKAAAPGRSK GLSGVCGRCCSIILSGIAMR LCYIAVVAGV VLVALHYEQE IQRRLFDV |
| SEQ ID NO: 15 | UL20 | MTMRDDLPLV DRDLVDEAAF GGEEGELPLE EQFSLSSYGT SDFFVSSAYS RLPPHTQPVFSKRVILFLWS FLVLKPLEMV AAGMYYGLTG RVVAPACILA AIVGYYVTWA VRALLLYVNIKRDRLPLSAP VFWGMSVFLG GTALCALFAA AHETFSPDGL FHFIATNQML PPTDPLRTRALGIACAAGAS MWVAAADSFA ASANFFLARF WTRAILNAPV AF |
| SEQ ID NO: 16 | UL24 | MAARTRSLVE RRRVLMAGVR SHTRFYKALA KEVREFHATK ICGTLLTLLS GSLQGRSVFEATRVTLICEV DLGPRRPDCI CVFEFANDKT LGGVCVIIEL KTCKYISSGD TASKREQRATGMKQLRHSLK LLQSLAPPGD KIVYLCPVLV FVAQRTLRVS RVTRLVPQKV SGNITAVVRMLQSLSTYTVP MEPRTQRARR RRGGAARGSA SRPKRSHSGA RDPPEPAARQ VPPADQTPASTEGGGVLKRI AALFCVPVAT KTKPRAASE |

(continued)

| | | |
|---|---|---|
| SEQ ID NO: 17 | human IL13RA2 | MAFVCLAIGCLYTFLISTTFGCTSSSDTEIKVNPPQDFEIVDPGYLGYLYLQWQPPLSLDHFKECTVEYELKYRNIGSETWKTIITKNLHYKDGFDLNKGIEAKIHTLLPWQCTNGSEVQSSWAETTYWISPQGIPETKVQDMDCVYYNWQYLLCSWKPGIGVLLDTNYNLFYWYEGLDHALQCVDYIKADGQNIGCRFPYLEASDYKDFYICVNGSSENKPIRSSYFTFQLQNIVKPLPPVYLTFTRESSCEIKLKWSIPLGPIPARCFDYEIEIREDDTTLVTATVENETYTLKTTNETRQLCFVVRSKVNIYCSDDGIWSEWSDKQCWEGEDLSKKTLLRFWLPFGFILILVIFVTGLLLRKPNTYPKMIPEFFCDT |
| SEQ ID NO: 18 | light chain forward primer | GGAAGATCTGAYATTGTGMTSACMCARWCTMCA |
| SEQ ID NO: 19 | light chain reverse primer | CCGAATTCGGATACAGTTGGTGCAGCATC |
| SEQ ID NO: 20 | heavy chain forward primer 1 | GGAAGATCTSARGTNMAGCTGSAGSAGTC |
| SEQ ID NO: 21 | heavy chain reverse primer 2 | GGAAGATCTSARGTNMAGCTGSAGSAGTCWGG |

[Table 2-3]

| | | |
|---|---|---|
| SEQ ID NO: 22 | heavy chain reverse primer | CCGAATTCATAGACAGATGGGGGTGTCGTTTTGGC |
| SEQ ID NO: 23 | human IL13 forward primer 1 | AAGAATTCGCTAGCGGTGGTGGTGGATCCGGCCCTGTGCCTCCCTCTA |
| SEQ ID NO: 24 | human IL13 reverse primer 1 | CCGGGATCCTGAACCTCCACCACCGTTGAACTGTCCCTCGCGAAAAAG |
| SEQ ID NO: 25 | human IL13 forward primer 2 | CCCTTAAGGTCTCTTTTGTGTGGTGCGTTCCGGTATGGCGCTTTTGTTGACCACGGTCATTGCTCTCACTTGCCTTGGCGGCTTTGCCTCCCCAGGCCCTGTGCCTCCCTCTAC |
| SEQ ID NO: 26 | human IL13 reverse primer 2 | TTGGATCCGGTACCGTTGAACTGTCCCTCGCGAAAAAGTT |
| SEQ ID NO: 27 | human IL13 forward primer 3 | ATCGGATCCCGGCGCGTGTACCACATCCAGG |
| SEQ ID NO: 28 | human IL13 reverse primer 3 | TCCGGACGTCTTCGGAGGCCCC |
| SEQ ID NO: 29 | human herpesvirus 1 s train KOS complete genome | GenBank no. JQ673480.1 (see https://www.ncbi.nlm.nih.gov/nuccore/JQ673480.1) |

(continued)

| | | |
|---|---|---|
| SEQ ID NO: 30 | ICP34.5 (human herpesvirus 1 strain KOS) | MARRRHRGPRRPRPPGPTGAVPTAQSQVTSTPNSEPVVRSAPAA APPPPPASGPPPSCSLLLRQWLHVPESASDDDDDDWPDSPPPEPAP EARPTAAAPRPRSPPPGAGPGGGANPSHPPSRPFRLPPRLALRLRV TAEHLARLRLRRAGGEGAPKPPATPATPATPTRVRFSPHVRVRHL VVWASAARLARRGSWARERADRARFRRRVAEAEAVIGPCLGPE ARARALARGAGPANSV |

[Accession No.] NITE BP-03673

| | | |
|---|---|---|
| 0-1 | Form PCT/RO/134<br>Indications for deposited microorganisms or other biological materials (PCT Rule 13.2) were according to right column. | |
| 0-1-1 | | JPO-PASi480 |
| 0-2 | International Application Number | |
| 0-3 | Applicant's or Agent's File Reference | P230524WO |

| | | |
|---|---|---|
| 1 | The following relate to microorganisms or biological materials mentioned in the Detailed Description of the Invention. | |
| 1-1 | Paragraph No. | 0132 |
| 1-3 | Depositing | |
| 1-3-1 | Depositary institution name | NPMD (Independent) National Institute of Technology and Evaluation, NITE Patent Microorganisms Depositary (NPMD) |
| 1-3-2 | Depositary institution location | Room 122, 2-5-8 Kazusa Kamatari, Kisarazu City, Chiba Prefecture, Japan, 292-0818 |
| 1-3-3 | Deposit date | June 16, 2022 (16.06.2022) |
| 1-3-4 | Accession No. | NPMD NITE BP-03673 |
| 1-5 | Target countries | All listed countries |
| | for Receiving Office use | |
| 0-4 | Received together with international application (yes/no) | |
| 0-4-1 | Authorized staff member | Nagai, Tsuneo |
| | for International Bureau use | |
| 0-5 | Date received by International Bureau | |
| 0-5-1 | Authorized staff member | |

[Sequence Listing]

**Claims**

1. IL13RA2-targeting herpes simplex virus displaying anti-IL13RA2 antibody or its antigen-binding fragment on the virus surface.

2. The herpes simplex virus according to claim 1, wherein the heavy chain CDRs (HCDR1, HCDR2, HCDR3) and light chain CDRs (LCDR1, LCDR2, LCDR3) of the anti-IL13RA2 antibody or its antigen-binding fragment have the amino acid sequences:

(i) HCDR1: RHGIH (SEQ ID NO: 1)
(ii) HCDR2: VIWAGGSTNYNSALMS (SEQ ID NO: 2)
(iii) HCDR3: DHFDSFDY (SEQ ID NO: 3)
(iv) LCDR1: TASSSVSSSYLH (SEQ ID NO: 4)
(v) LCDR2: STSNLAS (SEQ ID NO: 5) and
(vi) LCDR3: HQYHRSPLT (SEQ ID NO: 6),

or these amino acid sequences having a mutation of one or more amino acids.

3. The herpes simplex virus according to claim 1 or 2, wherein the anti-IL13RA2 antibody or its antigen-binding fragment comprises:

a sequence having at least 80% sequence identity with the amino acid sequence of the $V_L$ region listed as SEQ ID NO: 8, and
a sequence having at least 80% sequence identity with the amino acid sequence of the $V_H$ region listed as SEQ ID NO: 10.

4. The herpes simplex virus according to any one of claims 1 to 3, wherein the antigen-binding fragment is Fab, Fab', F(ab')2, Fv, scFv, dsFv, bis-scFv, a diabody, a triabody or a tetrabody.

5. The herpes simplex virus according to any one of claims 1 to 4, wherein the antigen-binding fragment is scFv.

6. The herpes simplex virus according to any one of claims 1 to 5, wherein the antigen-binding fragment contains a sequence having at least 90% sequence identity with the sequence of SEQ ID NO: 7 (scFv sequence).

7. The herpes simplex virus according to any one of claims 1 to 6, wherein the anti-IL13RA2 antibody or its antigen-binding fragment is a chimerized or humanized anti-IL13RA2 antibody or its antigen-binding fragment.

8. The herpes simplex virus according to any one of claims 1 to 7, wherein the anti-IL13RA2 antibody or its antigen-binding fragment is incorporated into an envelope protein.

9. The herpes simplex virus according to claim 8, wherein the anti-IL13RA2 antibody or its antigen-binding fragment is incorporated into envelope protein gD, envelope protein gB, envelope protein gC or envelope protein gH.

10. The herpes simplex virus according to claim 8, wherein the anti-IL13RA2 antibody or its antigen-binding fragment is incorporated into envelope protein gD.

11. The herpes simplex virus according to claim 8, wherein the anti-IL13RA2 antibody or its antigen-binding fragment is incorporated into the deleted portion of the amino acids from position 2 to position 24 of SEQ ID NO: 12 of envelope protein gD, or between position 24 and position 25, into the deleted portion of the amino acids from position 6 to position 38, or into the deleted portion of the amino acids from position 61 to position 218, of SEQ ID NO: 12.

12. The herpes simplex virus according to any one of claims 1 to 11, wherein the envelope protein gD has an amino acid mutation that weakens infectivity into cells expressing Nectin-1, HVEM and/or 3-OS-HS.

13. The herpes simplex virus according to claim 12, wherein the amino acid mutation that weakens infectivity into cells expressing Nectin-1 is one or more selected from the group consisting of a deletion of all of the amino acids from position 6 to position 38 of SEQ ID NO: 12, a deletion of all of the amino acids from position 61 to position 218 of SEQ ID NO: 12, a mutation of the amino acid at position 3 and/or position 38 of SEQ ID NO: 12 and a mutation of the amino acid at position 222 and position 223 of SEQ ID NO: 12.

14. The herpes simplex virus according to claim 13, wherein the amino acid mutation at position 3 of SEQ ID NO: 12 is a deletion or a substitution to cysteine, the amino acid mutation at position 38 is a substitution to cysteine, the amino acid mutation at position 222 is a substitution to asparagine and the amino acid mutation at position 223 is a substitution to

isoleucine.

**15.** The herpes simplex virus according to any one of claims 12 to 14, wherein of the amino acid mutations of envelope protein gD, the amino acid mutation that weakens infectivity into cells expressing HVEM and/or 3-OS-HS is a deletion of all or some of the amino acids from position 2 to position 38 of SEQ ID NO: 12, a deletion of all of the amino acids from position 61 to position 218 of SEQ ID NO: 12, an amino acid mutation at position 27 of SEQ ID NO: 12, an amino acid mutation at position 29 of SEQ ID NO: 12, and/or an amino acid mutation at position 30 of SEQ ID NO: 12.

**16.** The herpes simplex virus according to claim 15, wherein a portion of the deletion of amino acids from position 2 to position 38 of SEQ ID NO: 12 is a deletion of amino acids from position 2 to position 24, a deletion of amino acids from position 7 to position 11, a deletion of amino acids from position 7 to position 32, or a deletion of amino acids from position 6 to position 38, the amino acid mutation at position 27 is a substitution to alanine, proline or arginine, the amino acid mutation at position 29 is a substitution to alanine, and the amino acid mutation at position 30 is a substitution to alanine.

**17.** The herpes simplex virus according to any one of claims 1 to 16, wherein the envelope protein gB, envelope protein gK, UL20 protein and/or UL24 protein of the herpes simplex virus have an amino acid mutation that promotes entry into target cells and/or an amino acid mutation that promotes multinucleated giant cell formation.

**18.** The herpes simplex virus according to claim 17, wherein the amino acid mutation that promotes entry into target cells and/or the amino acid mutation that promotes multinucleated giant cell formation is a mutation according to the following (a), (b), (c) and/or (d).

(a) an amino acid mutation at position 285, an amino acid mutation at position 549, an amino acid mutation at position 796, an amino acid mutation at position 800, an amino acid mutation at position 813, an amino acid mutation at position 817, an amino acid mutation at position 854, an amino acid mutation at position 855, an amino acid mutation at position 858, an insertion of an amino acid between position 816 and position 817, a nonsense mutation of the amino acid at position 869 and/or a nonsense mutation of the amino acid at position 877, of SEQ ID NO: 13,
(b) an amino acid mutation at position 33, an amino acid mutation at position 40, an amino acid mutation at position 86, an amino acid mutation at position 99, an amino acid mutation at position 111, an amino acid mutation at position 121, an amino acid mutation at position 243, an amino acid mutation at position 304 and/or an amino acid mutation at position 310, of SEQ ID NO: 14,
(c) an amino acid mutation at position 49, amino acid mutations at positions 49, 50 and 51, an amino acid mutation at position 209, amino acid mutations at positions 209, 212 and 213 or a nonsense mutation of the amino acid at position 217, of SEQ ID NO: 15, and/or a deletion of the entire amino acid sequence listed as SEQ ID NO: 15, and
(d) amino acid mutations at positions 62, 63 and 64 of SEQ ID NO: 16.

**19.** The herpes simplex virus according to claim 18, wherein

the amino acid mutation at position 285 of SEQ ID NO: 13 is a substitution to asparagine, the amino acid mutation at position 549 of SEQ ID NO: 13 is a substitution to threonine, the amino acid mutation at position 796 of SEQ ID NO: 13 is a substitution to cysteine, the amino acid mutation at position 800 of SEQ ID NO: 13 is a substitution to tryptophan, the amino acid mutation at position 813 of SEQ ID NO: 13 is a substitution to isoleucine, the amino acid mutation at position 817 of SEQ ID NO: 13 is a substitution to histidine or proline, the amino acid mutation at position 854 of SEQ ID NO: 13 is a substitution to phenylalanine, the amino acid mutation at position 855 of SEQ ID NO: 13 is a substitution to valine, and the amino acid mutation at position 858 of SEQ ID NO: 13 is a substitution to cysteine or histidine,
the amino acid mutation at position 33 of SEQ ID NO: 14 is a substitution to serine, the amino acid mutation at position 40 of SEQ ID NO: 14 is a substitution to valine or threonine, the amino acid mutation at position 86 of SEQ ID NO: 14 is a substitution to proline, the amino acid mutation at position 99 of SEQ ID NO: 14 is a substitution to asparagine, the amino acid mutation at position 111 of SEQ ID NO: 14 is a substitution to valine, the amino acid mutation at position 121 of SEQ ID NO: 14 is a substitution to isoleucine, the amino acid mutation at position 243 of SEQ ID NO: 14 is a substitution to tyrosine, the amino acid mutation at position 304 of SEQ ID NO: 14 is a substitution to proline, and the amino acid mutation at position 310 of SEQ ID NO: 14 is a substitution to leucine,
the amino acid mutation at position 49 of SEQ ID NO: 15 is a substitution to alanine, the amino acid mutation at position 50 of SEQ ID NO: 15 is a substitution to alanine, the amino acid mutation at position 51 of SEQ ID NO: 15 is a substitution to alanine, the amino acid mutation at position 209 of SEQ ID NO: 15 is a substitution to alanine, the

amino acid mutation at position 212 of SEQ ID NO: 15 is a substitution to alanine, and the amino acid mutation at position 213 of SEQ ID NO: 15 is a substitution to alanine, and
the amino acid mutation at position 62 of SEQ ID NO: 16 is a substitution to glycine, the amino acid mutation at position 63 is a substitution to valine and the amino acid mutation at position 64 is a substitution to serine.

**20.** The herpes simplex virus according to any one of claims 1 to 19, which has a restricted growth modification.

**21.** The herpes simplex virus according to claim 20, wherein the restricted growth modification is a mutation that inactivates expression of functional ICP34.5.

**22.** Nucleic acid coding for a herpes simplex virus according to any one of claims 1 to 21.

**23.** Cells producing a herpes simplex virus according to any one of claims 1 to 21.

**24.** A method for producing a herpes simplex virus according to any one of claims 1 to 21.

**25.** A pharmaceutical composition comprising a herpes simplex virus according to any one of claims 1 to 21.

**26.** The pharmaceutical composition according to claim 25, which is a therapeutic agent for a tumor that expresses IL13RA2.

**27.** The pharmaceutical composition according to claim 26, wherein the tumor is melanoma, osteosarcoma, leukemia, lymphoma, prostate cancer, lung cancer (including malignant mesothelioma), glioma, head or neck cancer, renal carcinoma, Kaposi's sarcoma, colorectal cancer, pancreatic carcinoma, adrenal cancer, breast cancer or ovarian cancer.

**28.** An anti-IL13RA2 antibody or its antigen-binding fragment, wherein the heavy chain CDRs (HCDR1, HCDR2, HCDR3) and light chain CDRs (LCDR1, LCDR2, LCDR3) have the amino acid sequences:

(i) HCDR1: RHGIH (SEQ ID NO: 1)
(ii) HCDR2: VIWAGGSTNYNSALMS (SEQ ID NO: 2)
(iii) HCDR3: DHFDSFDY (SEQ ID NO: 3)
(iv) LCDR1: TASSSVSSSYLH (SEQ ID NO: 4)
(v) LCDR2: STSNLAS (SEQ ID NO: 5) and
(vi) LCDR3: HQYHRSPLT (SEQ ID NO: 6),

or these amino acid sequences having a mutation of one or more amino acids.

**29.** The anti-IL13RA2 antibody or its antigen-binding fragment according to claim 28, wherein the anti-IL13RA2 antibody or its antigen-binding fragment comprises:

a sequence having at least 80% sequence identity with the amino acid sequence of the $V_L$ region listed as SEQ ID NO: 8, and
a sequence having at least 80% sequence identity with the amino acid sequence of the $V_H$ region listed as SEQ ID NO: 10.

**30.** The anti-IL13RA2 antibody or its antigen-binding fragment according to claim 28 or 29, wherein the antigen-binding fragment is Fab, Fab', F(ab')2, Fv, scFv, dsFv, bis-scFv, a diabody, a triabody or a tetrabody.

**31.** The anti-IL13RA2 antibody or its antigen-binding fragment according to any one of claims 28 to 30, wherein the antigen-binding fragment contains a sequence having at least 90% sequence identity with the sequence of SEQ ID NO: 7 (scFv sequence).

**32.** The anti-IL13RA2 antibody or its antigen-binding fragment according to any one of claims 28 to 31, which is chimerized or humanized.

**33.** Nucleic acid coding for an anti-IL13RA2 antibody or its antigen-binding fragment according to any one of claims 28 to 32.

**34.** Cells producing an anti-IL13RA2 antibody or its antigen-binding fragment, and containing nucleic acid according to claim 33.

**35.** Cell producing an anti-IL13RA2 antibody or its antigen-binding fragment according to any one of claims 28 to 32.

**36.** Cells deposited under Accession No. NITE BP-03673.

**37.** A method for producing an anti-IL13RA2 antibody or its antigen-binding fragment according to any one of claims 28 to 32 using cells according to any one of claims 33 to 35.

**38.** A pharmaceutical composition comprising an anti-IL13RA2 antibody or its antigen-binding fragment according to any one of claims 28 to 32.

**39.** The pharmaceutical composition according to claim 38, which is for treatment of a tumor that expresses IL13RA2.

**40.** The pharmaceutical composition according to claim 39, wherein the tumor is melanoma, osteosarcoma, leukemia, lymphoma, prostate cancer, lung cancer (including malignant mesothelioma), glioma, head or neck cancer, renal carcinoma, Kaposi’s sarcoma, colorectal cancer, pancreatic carcinoma, adrenal cancer, breast cancer or ovarian cancer.

**41.** The pharmaceutical composition according to any one of claims 38 to 40, wherein the anti-IL13RA2 antibody or its antigen-binding fragment is included in a pharmaceutical composition in the form of an antibody-drug conjugate.

Fig. 1

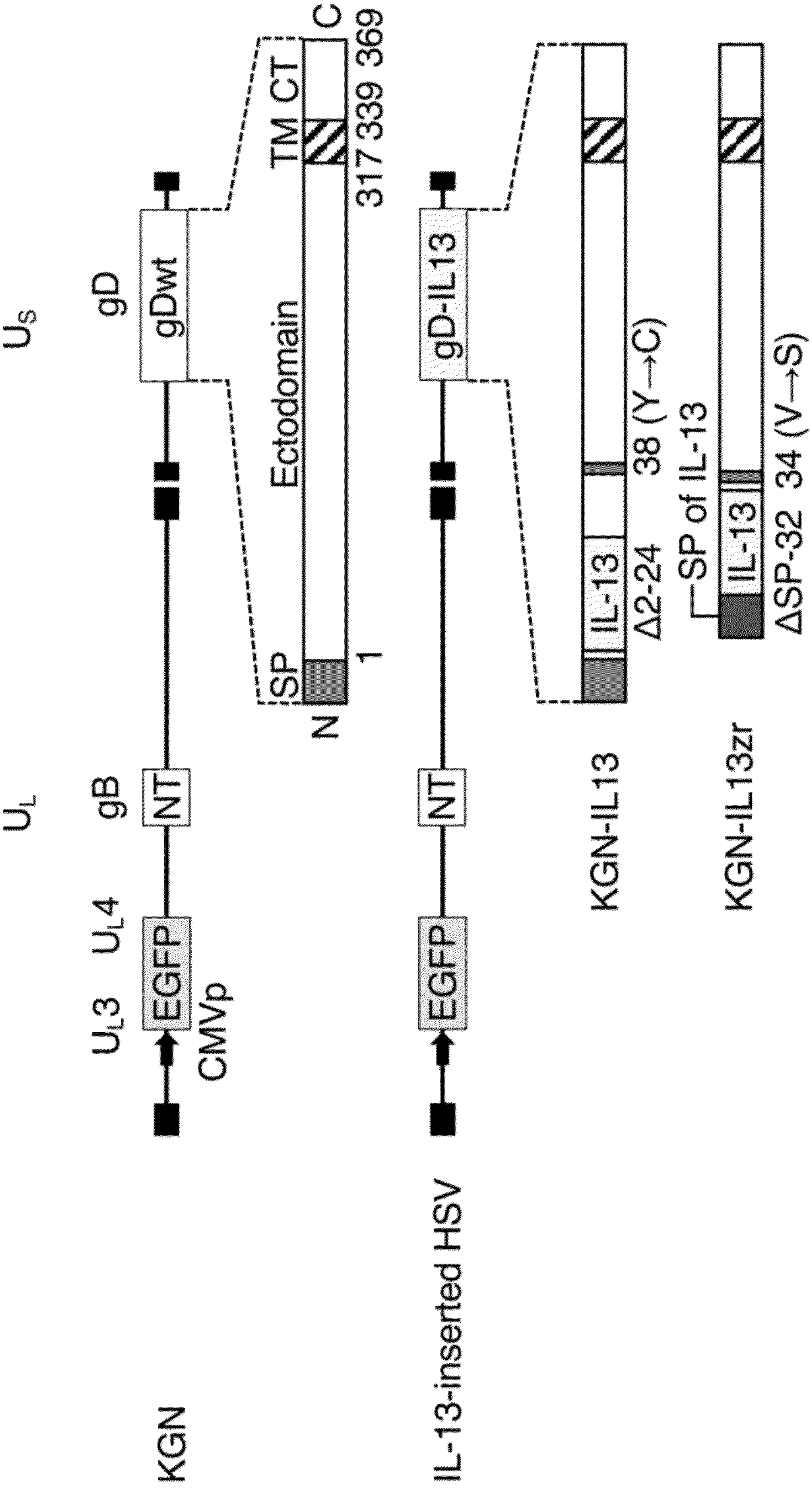
KGN
IL-13-inserted HSV
$U_L$
$U_S$
$U_L3$
$U_L4$
CMVp
EGFP
gB
NT
gD
gDwt
gD-IL13
SP
Ectodomain
TM
CT
N
C
1
317
339
369
KGN-IL13
KGN-IL13zr
IL-13
Δ2-24
38 (Y→C)
SP of IL-13
ΔSP-32
34 (V→S)

Fig. 2

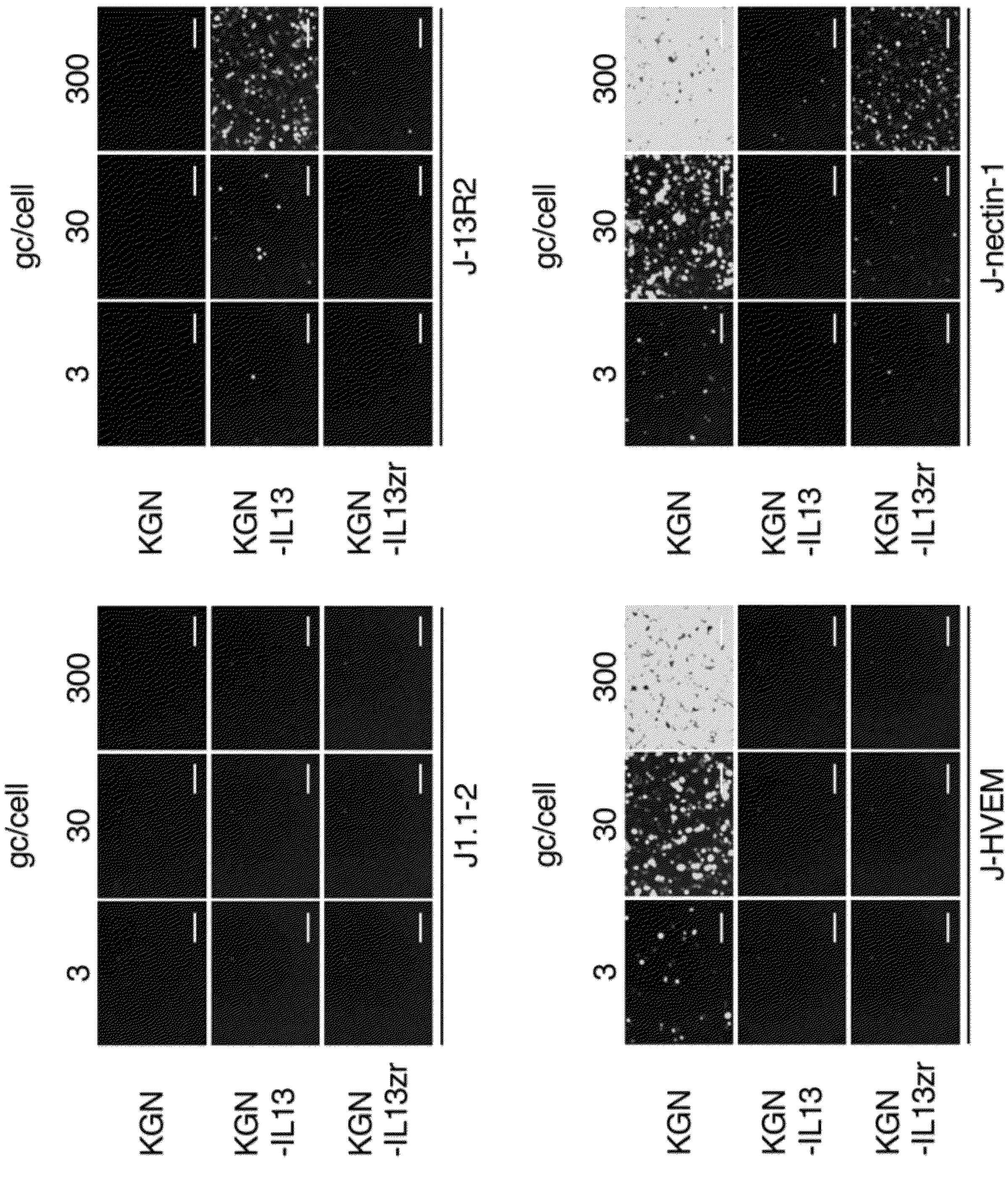
gc/cell
3
30
300
KGN
KGN -IL13
KGN -IL13zr
J1.1-2
J-13R2
J-HVEM
J-nectin-1

Fig. 3

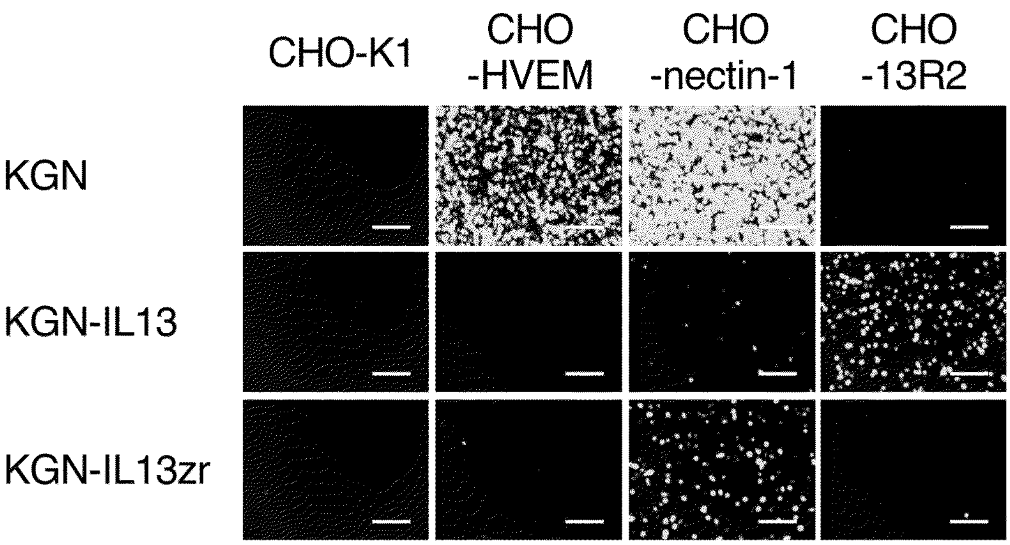

Fig. 4

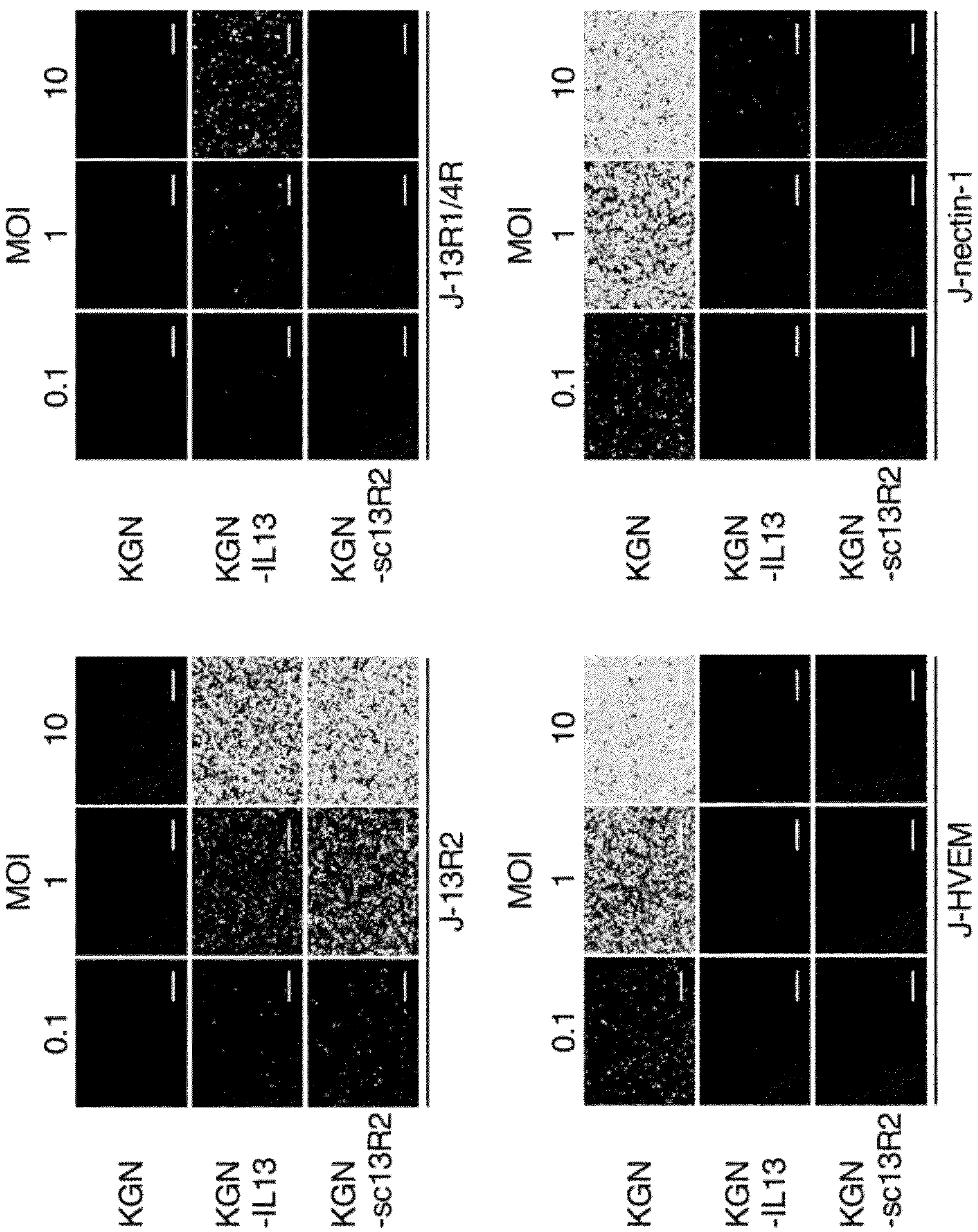
MOI
0.1
1
10
KGN
KGN -IL13
KGN -sc13R2
J-13R2
J-13R1/4R
J-HVEM
J-nectin-1

Fig. 5

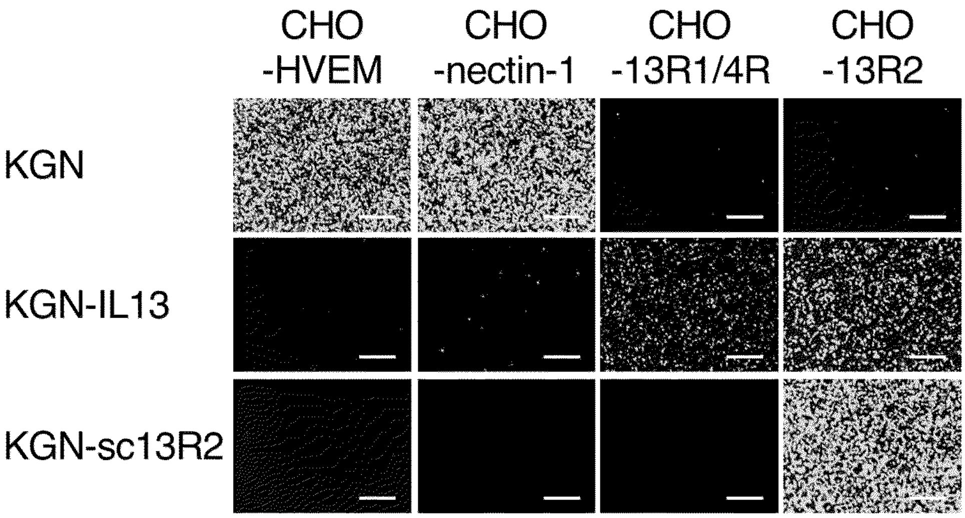
CHO
-HVEM
CHO
-nectin-1
CHO
-13R1/4R
CHO
-13R2
KGN
KGN-IL13
KGN-sc13R2

Fig. 6A

Fig. 6B

Fig. 6C

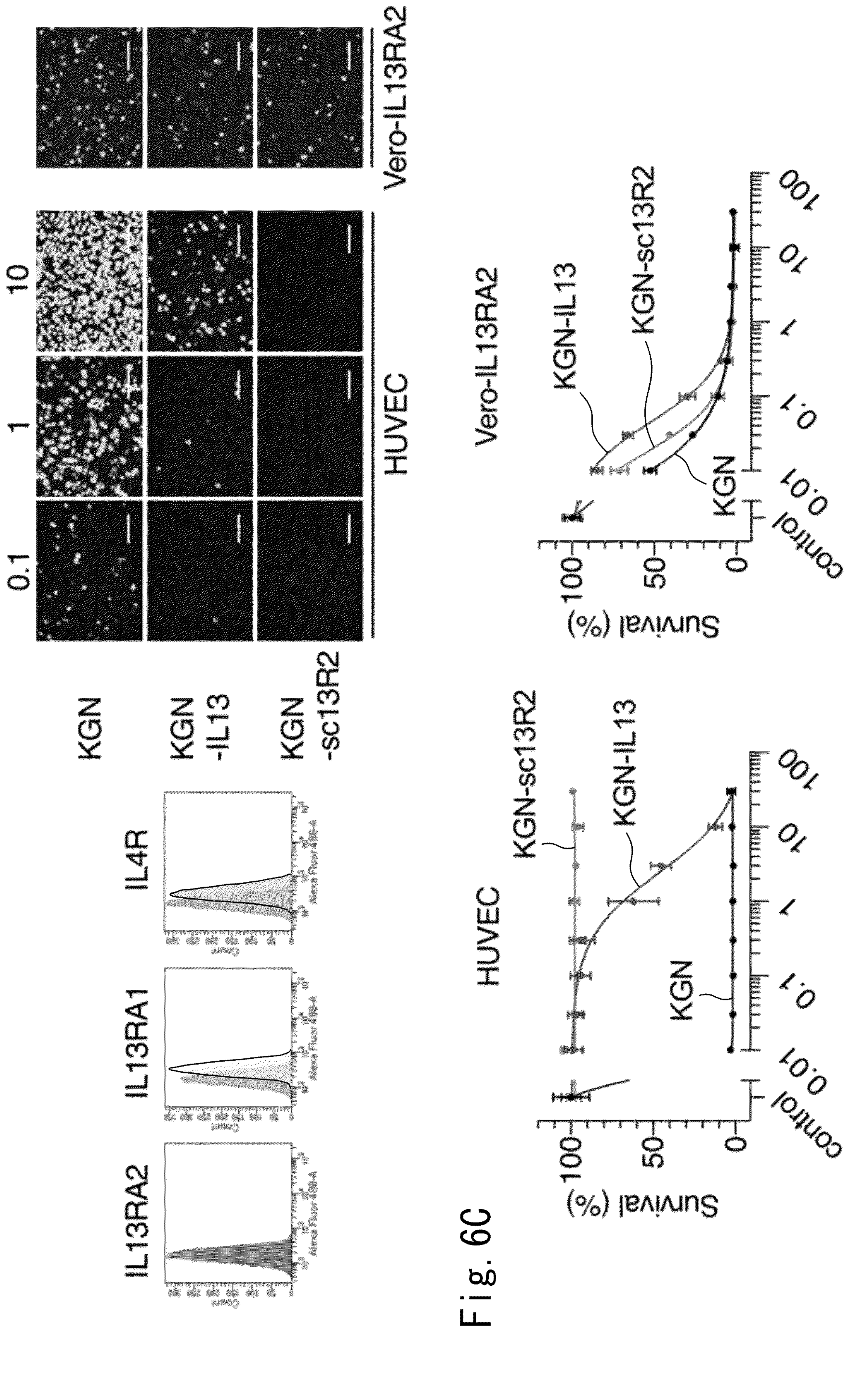

Fig. 7

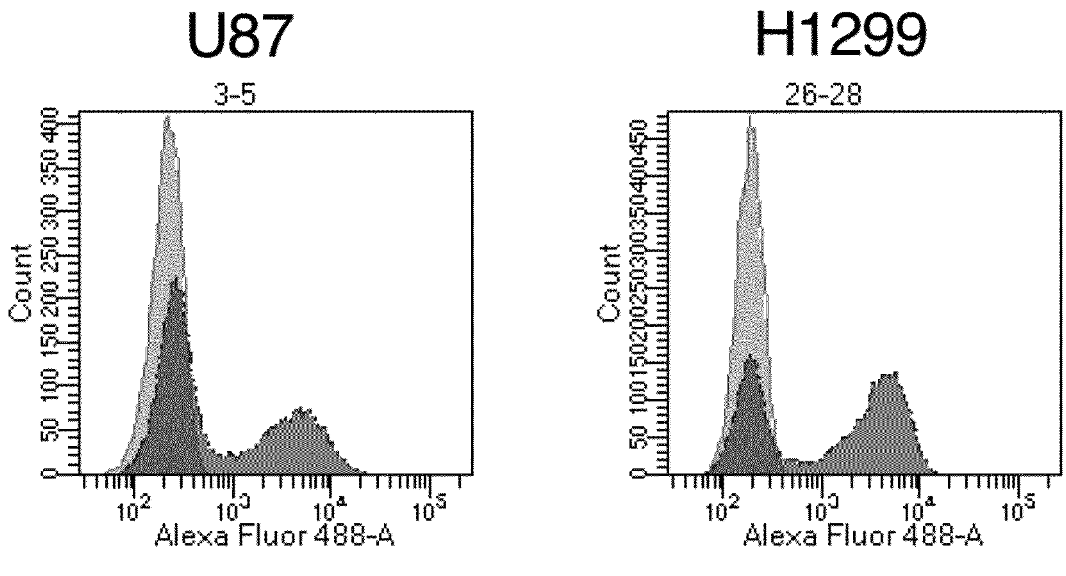
U87
3-5
Count
0 50 100 150 200 250 300 350 400
$10^2$ $10^3$ $10^4$ $10^5$
Alexa Fluor 488-A
H1299
26-28
Count
0 50 100 150 200 250 300 350 400 450
$10^2$ $10^3$ $10^4$ $10^5$
Alexa Fluor 488-A

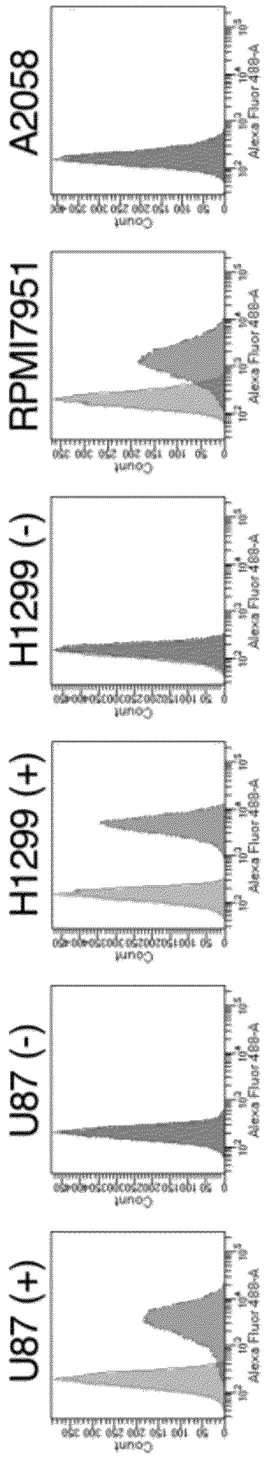


Fig. 8A

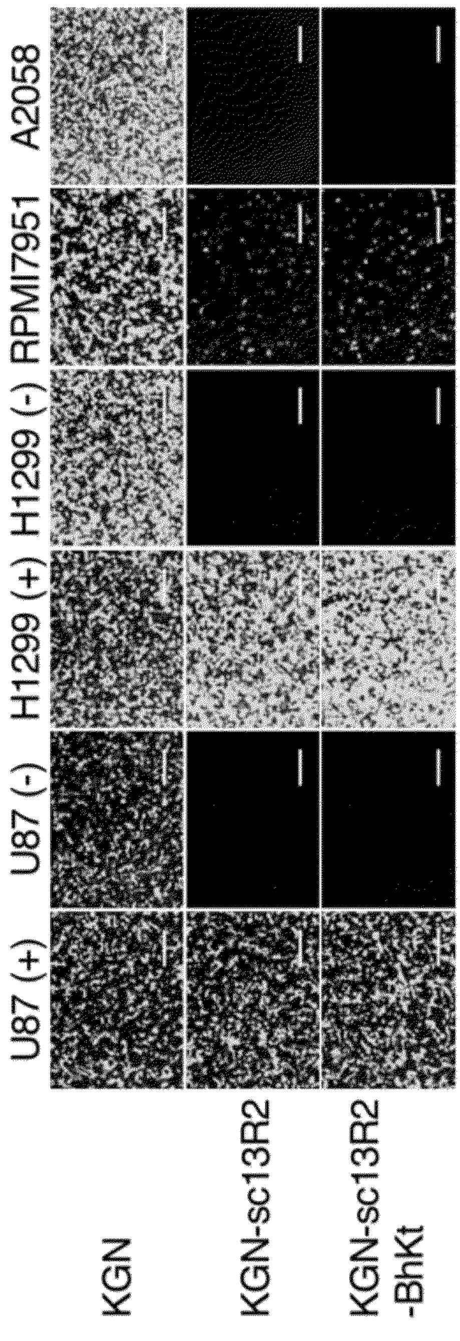


Fig. 8B

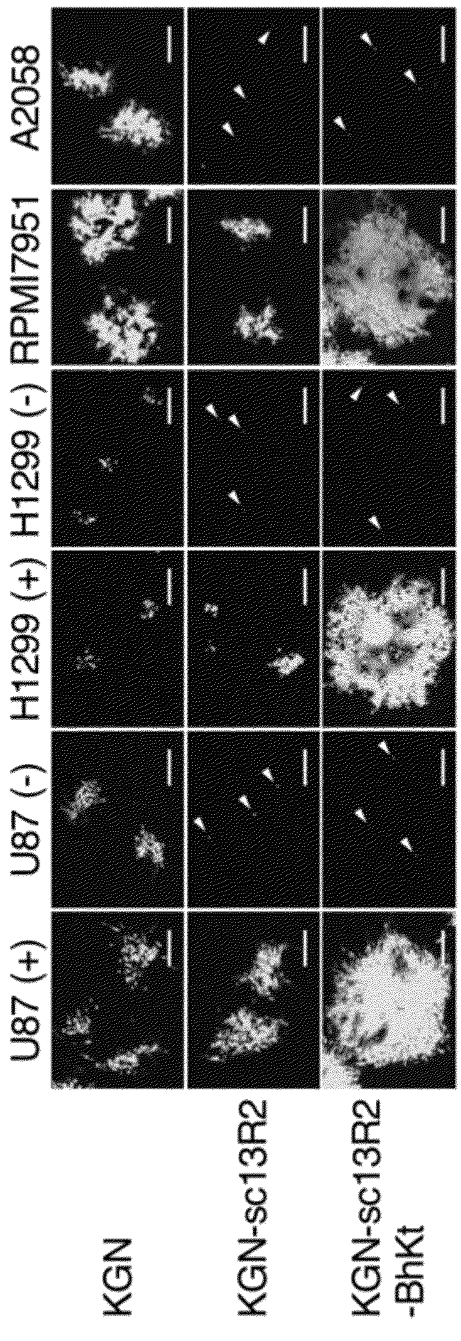


Fig. 8C

Fig. 9A

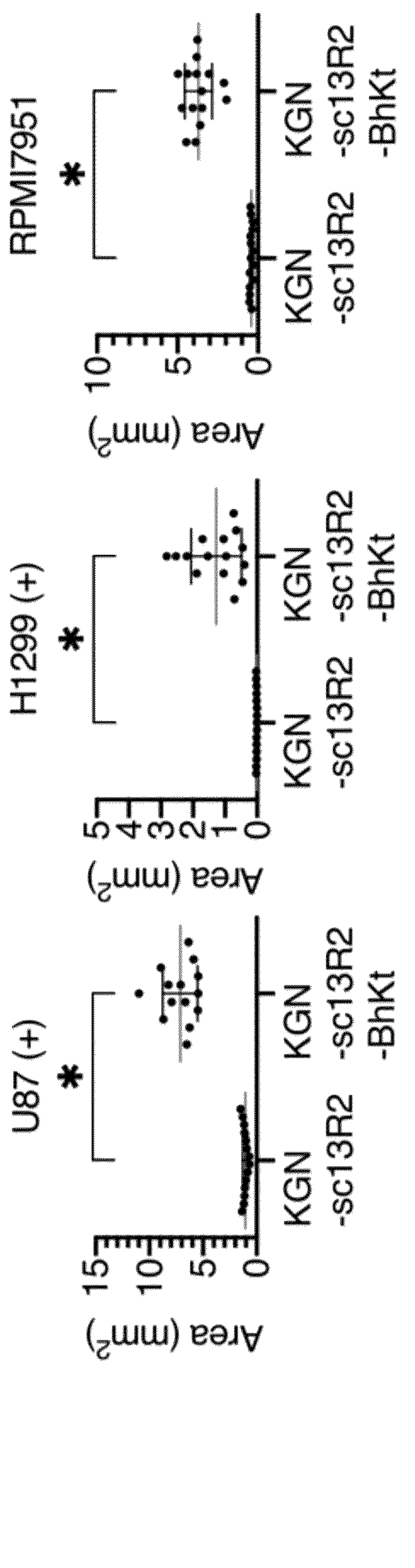
U87 (+)
H1299 (+)
RPMI7951
*
Area (mm²)
15
10
5
0
4
3
2
1
KGN -sc13R2
KGN -sc13R2 -BhKt

Fig. 9B

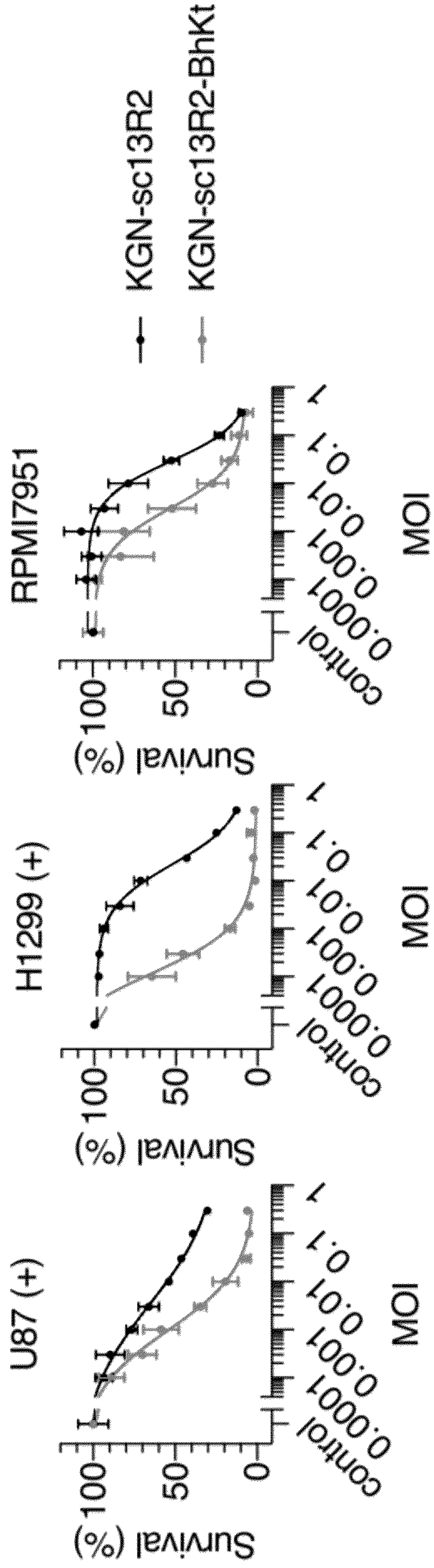
U87 (+)
H1299 (+)
RPMI7951
Survival (%)
100
50
0
control
0.0001
0.001
0.01
0.1
1
MOI
KGN-sc13R2
KGN-sc13R2-BhKt

Fig. 10A

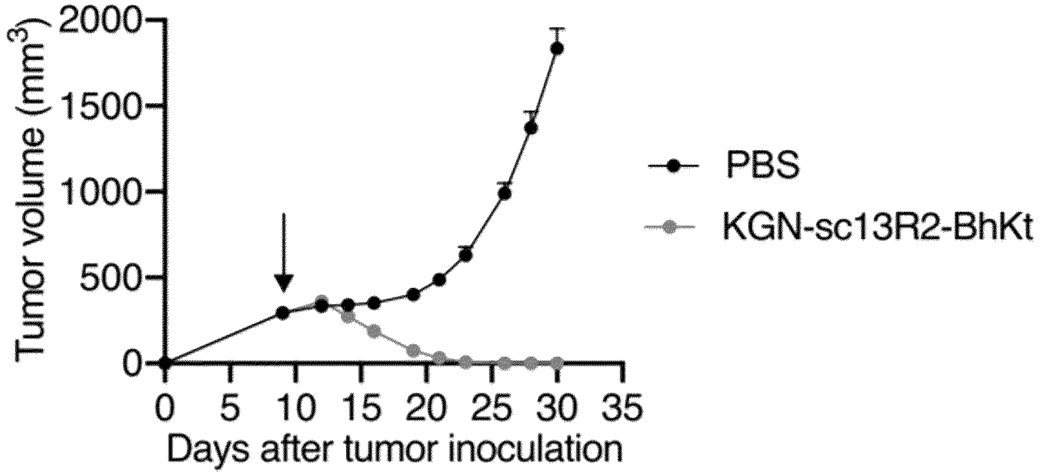


Fig. 10B

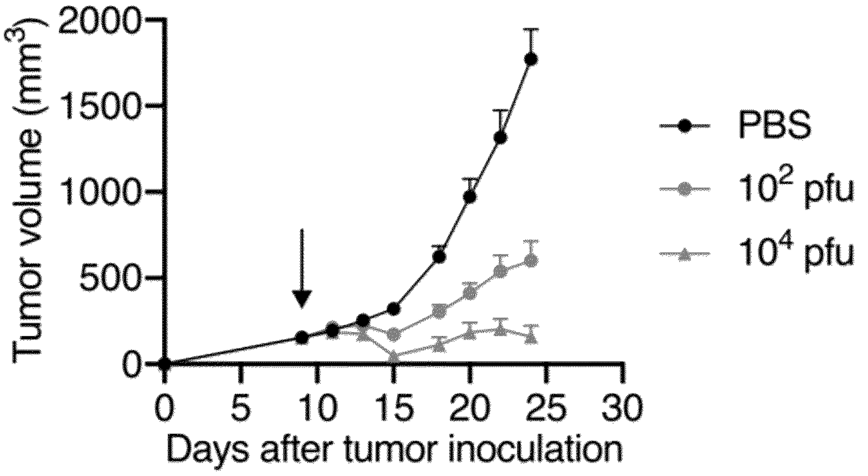


Fig. 10C

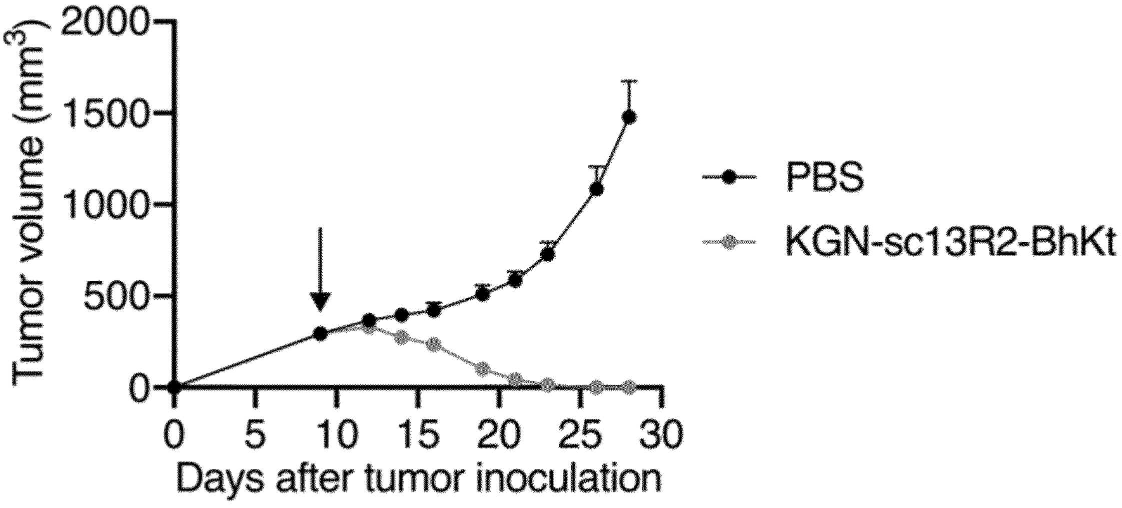

Fig. 11A

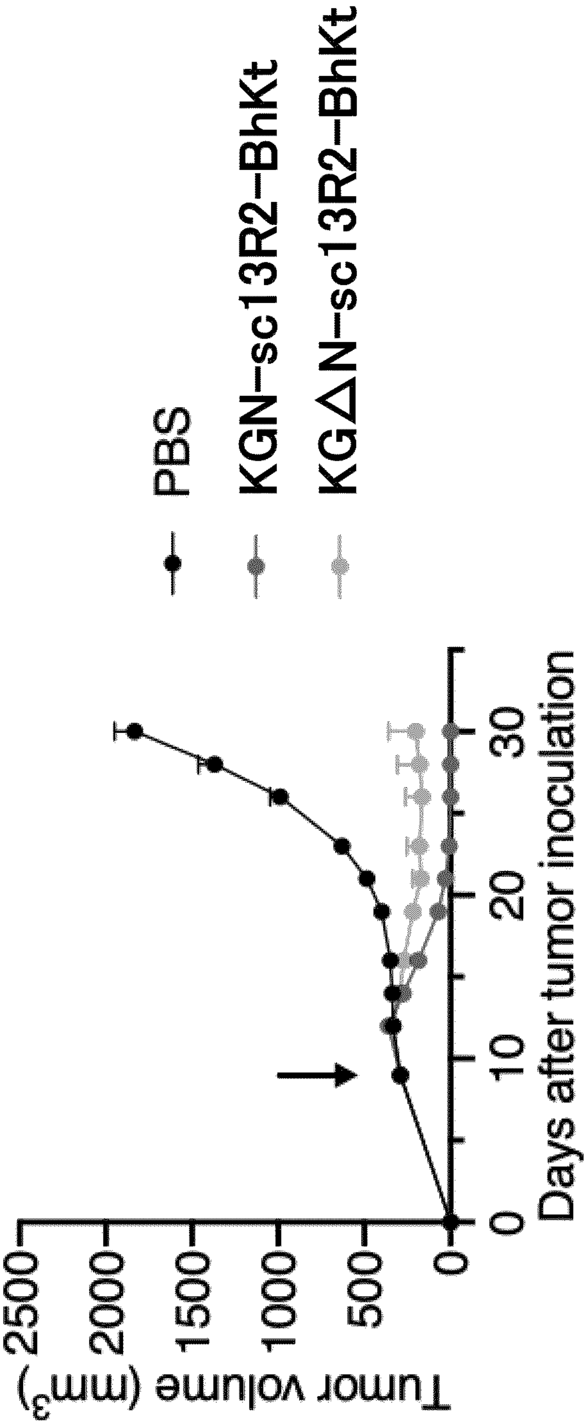


Fig. 11B

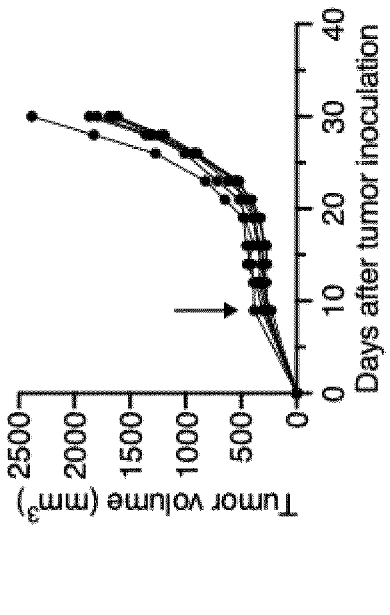


Fig. 11C

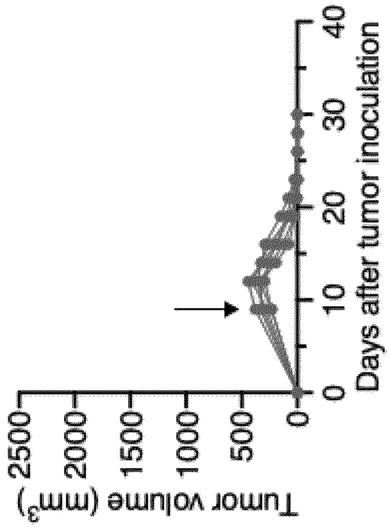


Fig. 11D

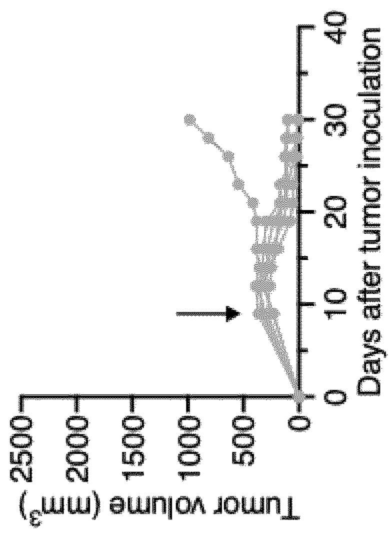

## INTERNATIONAL SEARCH REPORT

International application No. **PCT/JP2023/035689**

### A. CLASSIFICATION OF SUBJECT MATTER

***C12N 7/01***(2006.01)i; ***A61K 35/763***(2015.01)i; ***A61K 39/395***(2006.01)i; ***A61P 35/00***(2006.01)i; ***A61P 35/02***(2006.01)i; ***C07K 16/28***(2006.01)i; ***C07K 19/00***(2006.01)i; ***C12N 1/15***(2006.01)i; ***C12N 1/19***(2006.01)i; ***C12N 1/21***(2006.01)i; ***C12N 5/10***(2006.01)i; ***C12N 15/13***(2006.01)i; ***C12N 15/38***(2006.01)i; ***C12P 21/08***(2006.01)i

FI: C12N7/01 ZNA; A61K35/763; A61K39/395 N; A61P35/00; A61P35/02; C07K16/28; C07K19/00; C12N1/15; C12N1/19; C12N1/21; C12N5/10; C12N15/13; C12N15/38; C12P21/08

According to International Patent Classification (IPC) or to both national classification and IPC

### B. FIELDS SEARCHED

Minimum documentation searched (classification system followed by classification symbols)

C12N1/00-15/90; A61K35/763; A61K39/395; A61P35/00; C07K1/00-19/00; C12P21/08

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN); UniProt/GeneSeq; PubMed

### C. DOCUMENTS CONSIDERED TO BE RELEVANT

| Category* | Citation of document, with indication, where appropriate, of the relevant passages | Relevant to claim No. |
|---|---|---|
| X | JP 2016-503295 A (PFIZER INC) 04 February 2016 (2016-02-04)<br>claims, paragraphs [0003], [0040], examples, fig. 4 | 28-30, 32-35, 37-41 |
| Y | claims, paragraphs [0003], [0040], examples, fig. 4 | 1-5, 7-27 |
| A | entire text | 6, 31, 36 |
| X | WO 2021/041725 A1 (THE TRUSTEES OF THE UNIVERISTY OF PENNSYLVANIA) 04 March 2021 (2021-03-04)<br>claims, p. 1, line 32 to p. 2, line 6, table 1, example 1, fig. 8 | 28-35 |
| Y | claims, p. 1, line 32 to p. 2, line 6, table 1, example 1, fig. 8 | 1-27 |
| A | entire text | 36-41 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 November 2023** | **12 December 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/035689**

**C. DOCUMENTS CONSIDERED TO BE RELEVANT**

| Category* | Citation of document, with indication, where appropriate, of the relevant passages | Relevant to claim No. |
|---|---|---|
| Y | UCHIDA, H. et al. Effective treatment of an orthotopic xenograft model of human glioblastoma using an EGFR-retargeted oncolytic herpes simplex virus. Mol Ther. 2013, vol. 21, pp. 561-569, fig. S1<br>in particular, p. 561, left column, lines 1 to line 1 from the bottom, p. 561, right column, lines 14-1 from the bottom, p. 562, left column, line 9 from the bottom to p. 562, right column, line 13, p. 564, left column, line 13 from the bottom to p. 565, right column, line 1 from the bottom, p. 566, right column, line 26 to line 17 from the bottom, p. 567, left column, sections "Viruses", "Plasmids" fig. 4-7, S1 | 1-27 |
| A | entire text | 28-41 |
| Y | WO 2020/090871 A1 (THE UNIVERSITY OF TOKYO) 07 May 2020 (2020-05-07)<br>claims, paragraphs [0009], [0021] | 1-27 |
| A | entire text | 28-41 |
| A | 福原　武志　ほか, 独自の標的化抗体探索技術から見出したメラノーマの分子標的ＩＬ１３Ｒａ２, 第８６回日本生化学会大会　プログラム・講演要旨集, 2013, abstract no. 3T19a-11 (3LBA-057)<br>entire text, (FUKUHARA, Takeshi et al. IL13Ra2, therapeutic target of melanoma, identified by unique targeted antibody screening technology.), non-official translation (Programs and Lecture Abstracts of the 86th Annual Meeting of the Japanese Biochemical Society) | 1-41 |

Form PCT/ISA/210 (second sheet) (January 2015)

**INTERNATIONAL SEARCH REPORT**

International application No.
**PCT/JP2023/035689**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      - ☑ in the form of an Annex C/ST.25 text file.
      - ☐ on paper or in the form of an image file.
   
   b. ☐ furnished together with the international application under PCT Rule 13*ter*.1(a) for the purposes of international search only in the form of an Annex C/ST.25 text file.
   
   c. ☐ furnished subsequent to the international filing date for the purposes of international search only:
      - ☐ in the form of an Annex C/ST.25 text file (Rule 13*ter*.1(a)).
      - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

   "In the form of an Annex C/ST.25 text file" above should be understood as "in ST. 26 format".

Form PCT/ISA/210 (continuation of first sheet) (January 2015)

**INTERNATIONAL SEARCH REPORT**
**Information on patent family members**

International application No.
**PCT/JP2023/035689**

| Patent document cited in search report | Publication date (day/month/year) | Patent family member(s) | Publication date (day/month/year) |
|---|---|---|---|
| JP 2016-503295 A | 04 February 2016 | US 2015/0266962 A1 claims, paragraphs [0003], [0044], examples, fig. 4 | |
| WO 2021/041725 A1 | 04 March 2021 | US 2021/0128617 A1 claims, paragraph [0004], table 1, example 1, fig. 8 | |
| WO 2020/090871 A1 | 07 May 2020 | US 2021/0386807 A1 claims, paragraphs [0024], [0063]-[0064] | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020090871 A
- JP 2006528484 A
- US 7078029 B
- US 6261552 B
- US 5998174 B
- US 5879934 B
- US 5849572 B
- US 5849571 B
- US 5837532 B
- US 5804413 B
- US 5658724 B
- WO 9102788 A
- WO 9604394 A
- WO 9815637 A
- WO 9906583 A
- US 5543158 A
- US 5641515 A
- US 5399363 A
- US 5466468 A
- US 5756353 A
- US 5804212 A
- US 5725871 A
- US 5780045 A
- US 5545806 A
- US 5569825 A
- US 5714352 A
- US 20020197266 A1
- US 6265150 B
- US 5403484 A
- US 5571698 A
- US 5837500 A
- US 5702892 A
- US 5780279 A
- US 5821047 A
- US 5824520 A
- US 5855885 A
- US 5858657 A
- US 5871907 A
- US 5969108 A
- US 6057098 A
- US 6225447 B
- US 5225539 A
- US 5585089 A
- US 5693761 A
- EP 0239400 B1
- GB 2188638 A
- US 5639641 A
- US 4946778 A
- US 5693762 A


### Non-patent literature cited in the description


- **LIU et al.** *Mol Cancer Ther.*, August 2003, vol. 2 (8), 783-7
- **BEARD et al.** *Clin Cancer Res.*, 15 September 2013, vol. 19 (18), 4941-50
- **GESKIN et al.** *Blood*, 30 April 2015, vol. 125 (18), 2798-805
- **LUPARDUS et al.** *Structure.*, 10 March 2010, vol. 18 (3), 332-42
- **KUNWAR et al.** *J Clin Oncol*, 01 March 2007, vol. 25 (7), 837-44
- **AKAIWA et al.** *Cytokine*, 21 January 2001, vol. 13 (2), 75-84
- **ANDREWS et al.** *J Biol Chem*, 29 November 2002, vol. 277 (48), 46073-8
- **BALYASNIKOVA et al.** *J Biol Chem*, 31 August 2012, vol. 287 (36), 30215-27
- **UCHIDA et al.** *Curr Cancer Drug Targets*, 2018, vol. 18 (2), 162-170
- **MENOTTI** ; **AVITABILE**. *Int J Mol Sci*, 05 November 2020, vol. 21 (21), 8310
- **ZHOU** ; **ROIZMAN**. *Proc Natl Acad Sci USA*, 04 April 2006, vol. 103 (14), 5508-13
- **UCHIDA et al.** *Mol Ther.*, March 2013, vol. 21 (3), 561-9
- **SHIBATA et al.** *Gene Ther.*, 2016, vol. 23 (6), 479-88
- **IKEDA et al.** *J Virol*, 12 April 2021, vol. 95 (9), e01766-20
- **MOY, DIBLASIO et al.** *J Mol Biol*, 2001, vol. 310, 219-30
- **SIVAPRASAD et al.** *J Immunol.*, 2010, vol. 185 (11), 6802
- **KABAT et al.** Sequences of Proteins of Immunological Interest. US Department of Health and Human Services, NIH, 1987
- **KORTT et al.** *Biomol. Eng.*, 2001, vol. 18, 95-108
- **TODOROVSKA et al.** *J. Immunol Methods.*, 2001, vol. 248, 47-66

- **JANEWAY et al.** Immunobiology. Garland Publishing, 1996
- **REITER et al.** *Protein Engineering*, 1994, vol. 7, 697-704
- **MONTGOMERY et al.** *Cell*, 1996
- **GERAGHTY et al.** *Science*, 1998
- **SHUKLA et al.** *Cell*, 1999
- **CARFI et al.** *Mol Cell*, 2001
- **FUSCO et al.** *Proc Natl Acad Sci USA*, 2005
- **KRUMMENACHER et al.** *EMBO J*, 2005
- **ATANASIU et al.** *J Virol*, 2010
- **ATANASIU et al.** *Mbio*, 2013
- **GIANNI et al.** *J Biol Chem*, 2009
- **ATANASIU et al.** *Proc Natl Acad Sci USA*, 2007
- **CHOWDARY et al.** *Nat Struct Mol Biol*, 2010
- *PLoS Pathog*, 2017, vol. 13 (4), e1006352
- *Cancer Gene Ther*, 2010, vol. 17 (9), 655-663
- *PLoS Pathog*, 2015, vol. 11 (5), e1004907
- **YOON et al.** *J Virol.*, 2003, vol. 77, 9221-9231
- **SPEAR et al.** *Virology*, 2006, vol. 344, 17-24
- **CONNOLLY et al.** *J Virol.*, 2005, vol. 79, 1282-1295
- **UCHIDA et al.** *J Virol.*, 2009, vol. 83, 2951-2961
- **UCHIDA et al.** *J Virol.*, 2010, vol. 84, 12200-12209
- **SHIBATA et al.** *Gene Ther.*, 2016, vol. 23, 479-488
- **MENOTTI et al.** *J Virol.*, 2008, vol. 82, 10153-10161
- **MENOTTI et al.** *Proc Natl Acad Sci USA.*, 2009, vol. 106, 9039-9044
- **YOON** ; **SPEAR**. *Proc Natl Acad Sci USA.*, 2004, vol. 101, 17252-17257
- **DOLTER et al.** *J Virol.*, 1994, vol. 68, 8277-81
- **DEBROY et al.** *Virology*, 1985, vol. 145, 36-48
- **ISRAYELYAN et al.** *Hum Gene Ther.*, 2007, vol. 18, 457-73
- **TERRY-ALLISON et al.** *J Virol.*, 1998, vol. 72, 5802-10
- **UCHIDA et al.** *J Virol*, 2010, vol. 84, 12200-9
- **GAGE et al.** *J Virol.*, 1993, vol. 67, 2191-2201
- **CAI et al.** *J Virol.*, 1988, vol. 62, 2596-2604
- **ENGEL et al.** *Virology*, 1993, vol. 192, 112-120
- **DIAKIDI-KOSTA et al.** *Virus Res.*, vol. 93, 99-108
- **WALEV et al.** *Virus Genes*, 1994, vol. 8, 83-86
- **BZIK et al.** *Virology*, 1984, vol. 137, 185-190
- **FOSTER et al.** *Virology*, 2001, vol. 287, 18-29
- **BAGHIAN et al.** *J Virol.*, 1993, vol. 67, 2396-2401
- **MELANCON et al.** *J Virol.*, 2004, vol. 78, 7328-43
- **BAINES et al.** *J Virol.*, 1991, vol. 65, 6414-24
- **JACOBSON et al.** *Virology*, 1998, vol. 242, 161-69
- **SIMPSON et al.** *Cancer Res.*, 2006, vol. 66, 4835-4842
- **NAKAMORI et al.** *Clin Cancer Res.*, 2003, vol. 9, 2727-2733
- **CHOU et al.** *Science*, 1990, vol. 250, 1262-1266
- **MACLEAN et al.** *J. Gen. Virol.*, 1991, vol. 72, 631-639
- **PETERS et al.** *Mol Ther Oncolytics.*, 22 July 2015, vol. 2, 15010
- **PETERS et al.** *Mol Ther Oncolytics*, 22 July 2015, vol. 2, 15010
- **MINETA et al.** *Nat Med.*, 1995, vol. 1, 938-943
- **YAMAMOTO et al.** *Gene Ther.*, 2006, vol. 13, 1731-1736
- **ADUSUMILLI et al.** *FASEB J.*, 2006, vol. 20, 726-728
- **LI et al.** *Cancer Gene Ther.*, 2013, vol. 20, 478-485
- **NAKAMURA et al.** *Cancer Res.*, 2001, vol. 61, 5447-5452
- **TAMURA et al.** *Mol Ther.*, 2013, vol. 21, 68-77
- **LIU et al.** *Gene Ther.*, 2003, vol. 10, 292-303
- **ROTH et al.** *Ther Clin Dev.*, 2014, vol. 25, 16-27
- **ZHANG et al.** *Mol Ther.*, 2012, vol. 20, 37-45
- **LIU et al.** *Mol Ther.*, 2006, vol. 14, 789-797
- **DMITRIEVA et al.** *Clin Cancer Res.*, 2011, vol. 17, 1362-1372
- **EVE et al.** *Virus Res.*, 2006, vol. 119 (2), 195
- **TISCHER et al.** *Biotechniques*, 2006, vol. 40 (2), 191
- **MCGOECHETAL.** *J. Gen. Virol*, 1988, vol. 69 (PT7), 1531
- Remington: The Science and Practice of Pharmacy. Lippincott Williams & Wilkins, 2000
- **KORTT et al.** *Biomol. Eng*, 2001, vol. 18, 95-108
- **YAMAGUCHI, M.** ; **HAMADA, H. et al.** *Biochemical and Biophysical Research Communications*, 2014, vol. 454, 600-603
- Antibodies: A Laboratory Manual. CSH Press, 1988
- Immunobiology. Garland Publishing, 2001
- **KOEHLER** ; **MILSTEIN**. *Nature*, 1975, vol. 256, 495-497
- **KOSBOR et al.** *Immunol. Today*, 1983, vol. 4, 72
- **COTE et al.** *Proc. Natl. Acad. Sci.*, 1983, vol. 80, 2026-2030
- **COLE et al.** Monoclonal Antibodies and Cancer Therapy. Alan R Liss Inc, 1985, 77-96
- **HASKARD** ; **ARCHER**. *J. Immunol. Methods*, 1984, vol. 74 (2), 361-67
- **RODER et al.** *Methods Enzymol.*, 1986, vol. 121, 140-67

- **HUSE et al.** *Science*, 1989, vol. 246, 1275-81
- **ORLANDI et al.** *Proc. Natl. Acad. Sci.*, 1989, vol. 86, 3833-3837
- **WINTER** ; **MILSTEIN**. *Nature*, 1991, vol. 349, 293-299
- **SAMBROOK et al.** Molecular Cloning, A Laboratory Manual. Cold Spring Harbor Laboratory Press, 2001
- **PEDERSEN et al.** *J. Mol. Biol.*, 1994, vol. 235, 959-973
- **MORRISON et al.** *Proc. Natl. Acad. Sci.*, 1984, vol. 81, 6851-6855
- **NEUBERGER et al.** *Nature*, 1984, vol. 312, 604-608
- **TAKEDA et al.** *Nature*, 1985, vol. 314, 452-454
- **JONES et al.** *Nature*, 1986, vol. 321, 522-525
- **RIECHMANN et al.** *Nature*, 1988, vol. 332, 323-327
- **VERHOEYENET**. *Science*, 1988, vol. 239, 1534-1536
- **OWENS** ; **YOUNG**. *J. Immunol. Meth.*, 1994, vol. 168, 149-165
- **ANDREW C.R. MARTIN**. *KabatMan web page*, http://www.rubic.rdg.ac.uk/abs
- **LIGAS et al.** *J Virol.*, May 1988, vol. 62 (5), 1486-94
- **OKUBO et al.** *J Virol.*, 28 November 2016, vol. 90 (24), 11096-11105
- **YOSHIDA et al.** *Biochem Biophys Res Commun*, 1997, vol. 230, 426-430
- **YAMAGUCHI et al.** *Biochem Biophys Res Commun*, 28 November 2014, vol. 454 (4), 600-3
- **WANG et al.** *J Immunol Methods*, 2000, vol. 233, 167-177
- **GIERASCH et al.** *J Virol Methods.*, August 2006, vol. 135 (2), 197-206
- **TISCHER et al.** *Biotechniques.*, February 2006, vol. 40 (2), 191-7
- **SHIBATA et al.** *Gene Ther*, 2016, vol. 23 (6), 479-88
- **UCHIDA et al.** *J Virol.*, April 2009, vol. 83 (7), 2951-61
- **MIYAGAWA et al.** *Proc Natl Acad Sci USA.*, 31 March 2015, vol. 112 (13), E1632-41
- **UCHIDA et al.** *J Virol.*, February 2013, vol. 87 (3), 1430-42
- **TOMAYKO et al.** *Cancer Chemother Pharmacol*, 1989, vol. 24 (3), 148-54
- **YAMAGUCHI et al.** *Biochem Biophys Res Commun.*, 28 November 2014, vol. 454 (4), 600-3
- **DEBINSKI et al.** *Nat Biotechnol.*, May 1998, vol. 16 (5), 449-53
- **KAHLON et al.** *Cancer Res.*, 15 December 2004, vol. 64 (24), 9160-6
- **SUZUKI et al.** *Mol Ther Oncolytics.*, 2021, vol. 22, 265-276